# EUROPEAN PATENT APPLICATION

(11) **EP 4 803 504 A1**
(43) Date of publication of application: **09.09.2026**
(21) Application number: 24885599.1
(22) Date of filing: 24.10.2024
(51) Int. Cl.: C07C 211/63, C07C 211/08, C07C 215/40, C07C 219/08, C07C 229/12, C07C 233/36, C07C 271/44, C07D 487/08, C07F 7/08, C07F 7/10, C07F 7/12, C07F 9/54

(54) **HETEROPOLYOXOMETALATE HAVING MODIFIED LACUNARY SITE, OR MIXTURE THEREOF**

(30) Priority: 31.10.2023 JP 2023187314
(71) Applicant: TOKYO OHKA KOGYO CO., LTD., Kawasaki-shi, Kanagawa 211 0012 (JP)
(72) Inventor: NISHIZAWA, Akito, Kawasaki-shi, Kanagawa 211-0012 (JP); INARI, Takatoshi, Kawasaki-shi, Kanagawa 211-0012 (JP); UNO, Kakishi, Kawasaki-shi, Kanagawa 211-0012 (JP)
(74) Representative: Plasseraud IP
(86) International application number: PCT/JP2024/038004
(87) International publication number: WO 2025/094826

(57) **Abstract**

To provide a novel heteropolyacid salt having a modified lacunary site, or a mixture thereof, wherein a lacunary site of a heteropolyacid anion having the lacunary site is modified, and a metal ion, an ammonium cation, an ammonium dication, a phosphonium cation, or a phosphonium dication is used as a counter cation of the heteropolyacid anion.

## Description

### Technical Field

The present invention relates to a heteropolyacid salt having a modified lacunary site or a mixture thereof.

### Background Art

The polyacid is an anionic metal oxide cluster represented by the general formula [MₓO_{y}]ⁿ⁻ (wherein x, y, and n all denote natural numbers). The metal atom M constituting the polyacid is referred to as a polyatom and is, for example, Mo (hexavalent or pentavalent), W (hexavalent or pentavalent), V (pentavalent), Nb (pentavalent), Ta (pentavalent), or the like. The polyacid can be broadly divided into an isopolyacid composed of the polyatom M and an oxoacid, and a heteropolyacid ([X_{w}MₓO_{y}]ⁿ⁻ (wherein w, x, y, and n all denote natural numbers)) containing a different type of heteroatom X (for example, P⁵⁺, Si⁴⁺, Ge⁴⁺, B³⁺, S⁶⁺ or the like as a heteroatom X) in addition to the polyatom M and oxygen.

There are a plurality of lacunary species having a defect in part of the basic skeleton in the polyacid. A terminal oxygen atom at a lacunary site having a defect in part of the basic skeleton of the polyacid has a certain degree of negative charge density on the oxygen atom and has high reactivity and nucleophilicity. Thus, various functional polyacid complexes or organic/inorganic hybrid structures can be formed by reacting the terminal oxygen atom with an electrophilic atom or molecule.

Patent Literature 1 discloses, as a polyoxometalate compound having a metal-substituted polyoxometalate, a polyoxometalate compound that has a polyoxometalate having a lacunary site, a substituting metal atom (divalent platinum or palladium) introduced into the lacunary site, and an organic ligand.

### Citation List

### Patent Literature

PTL 1: U.S. Patent No. 11420871

### Summary of Invention

### Technical Problem

An object of the present invention is to provide a novel heteropolyacid salt having a modified lacunary site, or a mixture thereof.

### Solution to Problem

As a result of extensive studies to solve the above problems, the present inventors have completed the present invention by finding that a novel heteropolyacid salt having a modified lacunary site or a mixture thereof can be provided by modifying the lacunary site of a heteropolyacid anion having a lacunary site and introducing a predetermined metal ion, ammonium cation, ammonium dication, phosphonium cation, or phosphonium dication as a counter cation of the heteropolyacid anion.

More specifically, the present invention relates to the following inventions.
<1> A heteropolyacid salt having a modified lacunary site represented by the general formula (I) or a mixture thereof.

   (A^{m+})ₐ(Bⁿ⁺)_{b}(C(^{am+bn)-}) (I)

   [wherein
   A^{m+} each independently denotes H⁺ or a metal ion,
   Bⁿ⁺ each independently denotes an ammonium cation, an ammonium dication, a phosphonium cation, or a phosphonium dication,
   C^{(am+bn)-} denotes a heteropolyacid anion having a lacunary site, wherein the lacunary site is modified, and
   m denotes an integer in the range of 1 to 5, n denotes an integer of 1 or 2, and a and b denote real numbers]
<2> The heteropolyacid salt having the modified lacunary site or a mixture thereof according to <1>, wherein the heteropolyacid anion includes Mo, W, V, Nb, or Ta as a polyatom and P, Si, B, S, or Ge as a heteroatom.
<3> The heteropolyacid salt having the modified lacunary site or a mixture thereof according to <1>, wherein the heteropolyacid anion having the lacunary site is a lacunary Keggin-type heteropolyacid anion or a lacunary Dawson-type heteropolyacid anion.
<4> The heteropolyacid salt having the modified lacunary site or a mixture thereof according to <3>, wherein the lacunary Keggin-type heteropolyacid anion is represented by the general formula (II-1), (II-2), or (II-3).

   [XM₁₁O₃₉]^{c11-} (II-1)

   [XM₁₀O₃₆]^{c12-} (II-2)

   [XM₉O₃₄]^{c13-} (II-3)

   (wherein
   X denotes a heteroatom of P, Si, B, S, or Ge,
   M denotes a polyatom of Mo, W, V, Nb, or Ta, and
   c11- to c13- denote the number of negative charges, and c11 to c13 denote natural numbers)
<5> The heteropolyacid salt having the modified lacunary site or a mixture thereof according to <3>, wherein the lacunary Dawson-type heteropolyacid anion is represented by the general formula (III-1), (III-2), or (III-3).

   [X₂M₁₇O₆₁]^{c21-} (III-1)

   [X₂M₁₆O₅₈]^{c22-} (III-2)

   [X₂M₁₅O₅₆]^{c23-} (III-3)

   (wherein
   X denotes a heteroatom of P, Si, B, S, or Ge,
   M denotes a polyatom of Mo, W, V, Nb, or Ta, and
   c21- to c23- denote the number of negative charges, and c21 to c23 denote natural numbers)
<6> The heteropolyacid salt having the modified lacunary site or a mixture thereof according to <1>, wherein the modification is performed by bonding a group having one or more heteroatoms P, Si, Ge, or Sn, to which one or more hydro groups or organic groups are bonded, to the heteropolyacid anion having the lacunary site via a part or all of the heteroatoms.
<7> The heteropolyacid salt having the modified lacunary site or a mixture thereof according to <6>, wherein the organic group has one or more halogen atoms, haloalkyl groups, hydroxy groups, thiol groups, nitro groups, cyano groups, carboxy groups, amino groups, sulfo groups, epoxy groups, glycidyl groups, or amide groups.
<8> The heteropolyacid salt having the modified lacunary site or a mixture thereof according to <6>, wherein the organic group has one or more ethylenically unsaturated double bonds.
<9> The heteropolyacid salt having the modified lacunary site or a mixture thereof according to <8>, wherein the ethylenically unsaturated double bond is a vinyl group, a (meth)acryloyl group, a (meth)acryloyloxy group, a (meth)acrylamide group, or a styryl group.
<10> The heteropolyacid salt having the modified lacunary site or a mixture thereof according to <6>, wherein the organic group has one or more hydroxy groups or carboxy groups, each having a protective group.
<11> The heteropolyacid salt or a mixture thereof according to <1>, wherein the heteropolyacid anion having the modified lacunary site is represented by any one of the general formulae (VI-1) to (VI-12).

   [XM₁₁O₃₉(R^{1A}X')₂O]^{c31-} (VI-1)

   [XM₁₁O₃₉(R^{1B1}R^{1B2}X'₂]^{c31-} (VI-2)

   [XM₁₁O₃₉(R^{1D}P=O)₂]^{c31-} (VI-3)

   [XM₁₁O₃₉(R^{1E}X")]^{c31-} (VI-4)

   [XM₁₀O₃₆(R^{1A}X')₂O]^{c32-} (VI-5)

   [XM₁₀O₃₆(R^{1B1}R^{1B2}X')₂]^{c32-} (VI-6)

   [XM₁₀O₃₆(R^{1C}X'O)₄]^{c32-} (VI-7)

   [XM₁₀O₃₆(R^{1D}P=O)₂]^{c32-} (VI-8)

   [X₂M₁₇O₆₁(R^{1A}X')₂O]^{c33-} (VI-9)

   [X₂M₁₇O₆₁(R^{1B1}R^{1B2}X')₂]^{c33-} (VI-10)

   [X₂M₁₇O₆₁(R^{1D}P=O)₂]^{c33-} (VI-11)

   [X₂M₁₇O₆₁(R^{1E}X")]^{c33-} (VI-12)

   (wherein
   X denotes a heteroatom of P, Si, B, S, or Ge,
   M denotes a polyatom of Mo, W, V, Nb, or Ta, and
   X' denotes a heteroatom of Si or Ge,
   X" denotes a heteroatom of Si, Ge, or Sn,
   R^{1A} to R^{1E} each independently denote a hydro group, an optionally substituted C₁₋₁₈ hydrocarbyl group, an optionally substituted 3- to 18-membered non-aromatic heterocyclic group, or an optionally substituted 5- to 18-membered aromatic heterocyclic group,
   a divalent carbon atom at any position of R^{1A} to R^{1E} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
   a hydrogen atom in R^{1A} to R^{1E} may be substituted with, for example, (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group, and
   c11, c12, and c21 denote natural numbers)
<12> The heteropolyacid salt having the modified lacunary site or a mixture thereof according to <1>, wherein the ammonium cation is an organic quaternary ammonium cation represented by the general formula (VIII). 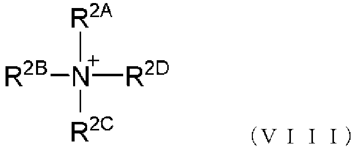 (In the formula (VIII),
   R^{2A} to R^{2D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
   a divalent carbon atom at any position of R^{2A} to R^{2D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
   a hydrogen atom in R^{2A} to R^{2D} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (s), and a divalent carbon atom at any position of these substituents except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
   any two of R^{2A} to R^{2D} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with the nitrogen atom in the formula (VIII))
<13> The heteropolyacid salt having the modified lacunary site or a mixture thereof according to <12>, wherein at least one hydrogen atom in R^{2A} to R^{2D} of the organic quaternary ammonium cation represented by the general formula (VIII) is substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group.
<14> The heteropolyacid salt having the modified lacunary site or a mixture thereof according to <12>, wherein at least one hydrogen atom in R^{2A} to R^{2D} of the organic quaternary ammonium cation represented by the general formula (VIII) is substituted with (j) a vinyl group, (k) an allyl group, (l) a (meth)acryloyl group, (m) a (meth)acryloyloxy group, (n) a (meth)acrylamide group, or (o) a styryl group.
<15> The heteropolyacid salt having the modified lacunary site or a mixture thereof according to <1>, wherein the ammonium dication is an organic quaternary ammonium dication represented by the general formula (IX). 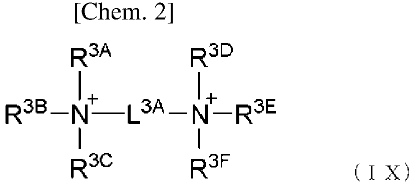 (In the formula (IX),
   R^{3A} to R^{3F} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
   a divalent carbon atom at any position of R^{3A} to R^{3F} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
   L^{3A} denotes an optionally substituted C₁₋₁₈ hydrocarbylene group,
   a divalent carbon atom at any position of L^{3A} may be substituted with -O-, -C(=O)-, -C(=O)O-, -OCO-, -CONH-, -NHCO-, -S-, or -SO₂- (provided that adjacent divalent carbon atoms are not substituted at the same time),
   a hydrogen atom in R^{3A} to R^{3F} and L^{3A} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (s), and a divalent carbon atom at any position of these substituents except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
   any two of R^{3A} to R^{3F} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with the nitrogen atom in the formula (IX))
<16> The heteropolyacid salt having the modified lacunary site or a mixture thereof according to <1>, wherein the phosphonium cation is an organic quaternary phosphonium cation represented by the general formula (X). 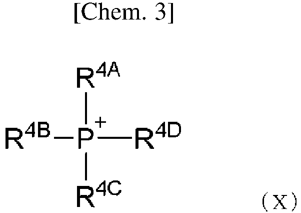 (In the formula (X),
   R^{4A} to R^{4D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
   a divalent carbon atom at any position of R^{4A} to R^{4D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
   a hydrogen atom in R^{4A} to R^{4D} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (s), and a divalent carbon atom at any position of these substituents except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
   any two of R^{4A} to R^{4D} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with the phosphorus atom in the formula (X))
<17> The heteropolyacid salt having the modified lacunary site or a mixture thereof according to <16>, wherein at least one hydrogen atom in R^{4A} to R^{4D} of the organic quaternary phosphonium cation represented by the general formula (X) is substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group.
<18> The heteropolyacid salt having the modified lacunary site or a mixture thereof according to <1>, wherein the phosphonium dication is an organic quaternary phosphonium dication represented by the general formula (XI). 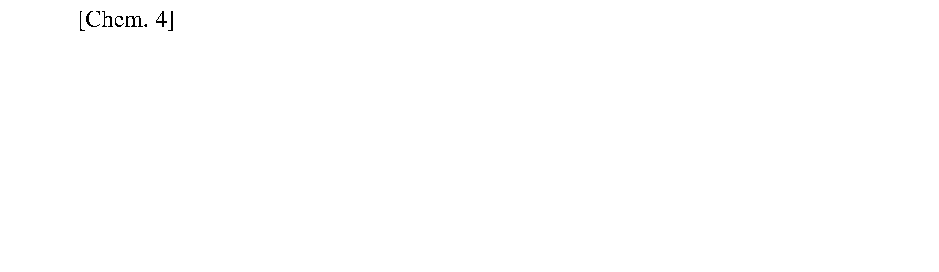 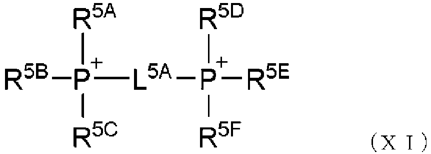 (In the formula (XI),
   R^{5A} to R^{5F} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
   a divalent carbon atom at any position of R^{5A} to R^{5F} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
   L^{5A} denotes an optionally substituted C₁₋₁₈ hydrocarbylene group,
   a divalent carbon atom at any position of L^{5A} may be substituted with -O-, -C(=O)-, -C(=O)O-, -OCO-, -CONH-, -NHCO-, -S-, or -SO₂- (provided that adjacent divalent carbon atoms are not substituted at the same time),
   a hydrogen atom in R^{5A} to R^{5F} and L^{5A} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (s), and a divalent carbon atom at any position of these substituents except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
   any two of R^{5A} to R^{5F} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with the phosphorus atom in the formula (XI))
<19> The heteropolyacid salt having the modified lacunary site or a mixture thereof according to <1>, wherein the metal ion is Na⁺, K⁺, Rb⁺, Cs⁺, Ca²⁺, Al³⁺, or Co³⁺.

### Advantageous Effects of Invention

The present invention can provide a novel heteropolyacid salt having a modified lacunary site or a mixture thereof. The heteropolyacid salt having the modified lacunary site or a mixture thereof can be suitably used as a novel building block having various functions, such as actinic radiation reactivity, by modifying the lacunary site and using, as a counter cation of the heteropolyacid anion, an ammonium cation, an ammonium dication, a phosphonium cation, or a phosphonium dication into which a predetermined metal ion, a predetermined substituent, an ethylenically unsaturated double bond having actinic radiation (including visible light, ultraviolet radiation, X-rays, electron beams, α radiations, β radiations, γ radiations, and the like) reactivity, or a hydroxy group or a carboxy group each having a protective group is introduced.

### Description of Embodiments

Preferred embodiments of the present invention will be described in detail below. However, the present invention is not limited to the following embodiments.

In the present description, the term "(meth)acryloyl group" is used to mean both an acryloyl group and a methacryloyl group.

The term "halogen atom", as used herein, refers to a fluorine atom, a chlorine atom, a bromine atom, or an iodine atom.

In the present description, for example, the term "C₁₋₆" or the like means the number of carbon atoms of a group serving as a mother nucleus.

The term "C₁₋₁₈ hydrocarbylene group", as used herein, refers to a divalent hydrocarbon group formed by removing two hydrogen atoms from a hydrocarbon with 1 to 18 carbon atoms. The hydrocarbylene group may be linear, branched, or partially or fully cyclic. Examples of the hydrocarbylene group include an alkylene group and an arylene group.

A divalent carbon atom at any position of the "C₁₋₁₈ hydrocarbylene group" may be replaced by -O-, -S-, -C(=O)-, -COO-, -OCO-, -CONH-, -NHCO-, -NH(C=O)O-, -S-, -SO-, or -SO₂- (provided that adjacent divalent carbon atoms are not replaced at the same time).

The "C₁₋₁₈ hydrocarbylene group" is, for example, but not limited to, a "C₁₋₁₈ alkylene group", such as a methylene group, an ethylene group, a n-propylene group, an i-propylene group, a cyclopentadiyl group, a cyclohexanediyl group, an oxyethane-1,1-diyl group, an oxyethane-1,2-diyl group, an oxypropane-1,3-diyl group, an oxypropane-1,2-diyl group, a 2-methylpropane-1,3-diyl group, or an oxyethyleneoxyethane-1,1-diyl group; a "C₆₋₁₈ arylene group", such as a 1,4-phenylene group, a 1,3-phenylene group, a 1,2-phenylene group, a 1,4-naphthylene group, a 1,5-naphthylene group, a 1,8-naphthylene group, a 4,4'-biphenylene group, an anthracenediyl group, a phenanthrenediyl group, a naphthacenediyl group, a pyrenediyl group, a perylenediyl group, or a chrysenediyl group; or the like.

The term "C₁₋₁₈ alkyl group", as used herein, refers to a linear or branched alkyl group with 1 to 18 carbon atoms.

Furthermore, a divalent carbon atom at any position excluding the terminals contained in the "C₁₋₁₈ alkyl group" may be replaced by -O-, -C(=O)-, -C(=O)O-, -OCO-, - CONH-, -NHCO-, -NH(C=O)O-, -S-, or -SO₂-. However, adjacent divalent carbon atoms are not replaced at the same time.

The "C₁₋₁₈ alkyl group" is, for example, but not limited to, a methyl group, an ethyl group, a n-propyl group, an i-propyl group, a n-butyl group, an i-butyl group, a sec-butyl group, a t-butyl group, a n-pentyl group, an i-pentyl group, a sec-pentyl group, a t-pentyl group, a neopentyl group, a 1-methylbutyl group, a 2-methylbutyl group, a 1,1-dimethylpropyl group, a 1,2-dimethylpropyl group, a n-hexyl group, an i-hexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group, a 1-ethyl-2-methylpropyl group, a n-heptyl group, a n-octyl group, a n-nonyl group, a n-decyl group, a n-undecyl group, a n-dodecyl group, or the like.

The "C₁₋₁₈ alkyl group" in which a divalent carbon atom at any position excluding the terminals thereof is replaced by -O-, -C(=O)-, -C(=O)O-, -OCO-, -CONH-, -NHCO-, - NH(C=O)O-, -S-, or -SO₂- is, for example, but not limited to, a 2-methoxyethoxymethyl group, an ethoxycarbonylmethyl group, or the like.

The term "C₁₋₁₈ haloalkyl group", as used herein, refers to a group in which one or more hydrogen atoms of the "C₁₋₁₈ alkyl group" are substituted by a halogen atom.

The "C₁₋₁₈ haloalkyl group" is, for example, but not limited to, a dichloromethyl group, a trifluoromethyl group, a 2,2-difluoroethyl group, a 2,2,2-trifluoroethyl group, a pentafluoroethyl group, a 3,3,3-trifluoropropyl group, or the like.

The term "C₂₋₁₈ alkenyl group", as used herein, refers to an alkenyl group with 2 or more carbon atoms having one or more double bonds in the "C₁₋₁₈ alkyl group" and includes an alkadienyl group, an alkatrienyl group, and the like.

The "C₂₋₁₈ alkenyl group" is, for example, but not limited to, a vinyl group (ethenyl group), an allyl group (2-propenyl group), a 1-propenyl group, an isopropenyl group (1-methylvinyl group), a 1-butenyl group, a 2-butenyl group, a 3-butenyl group, a pentenyl group, a hexenyl group, a heptenyl group, an octenyl group, a nonenyl group, a decenyl group, an undecenyl group, a dodecenyl group, or the like.

The term "C₂₋₁₈ alkynyl group", as used herein, refers to an alkynyl group with 2 or more carbon atoms having one or more triple bonds in the "C₁₋₁₈ alkyl group".

The "C₂₋₁₈ alkynyl group" is, for example, but not limited to, an ethynyl group, a 1-propynyl group, a 2-propynyl group, a pentynyl group, a hexynyl group, a heptynyl group, an octynyl group, a nonynyl group, a decynyl group, an undecynyl group, a dodecynyl group, or the like.

The term "C₃₋₁₈ alicyclic group", as used herein, refers to a hydrocarbon group with 3 to 18 carbon atoms and with a monocyclic or polycyclic structure in whole or in part. The alicyclic group includes a cycloalkyl group, a cycloalkenyl group, a cycloalkynyl group, a monocycloalkyl group, a polycycloalkyl group, and the like.

A divalent carbon atom at any position excluding the terminals contained in the "C₃₋₁₈ alicyclic group" may be replaced by -O-, -C(=O)-, -C(=O)O-, -OCO-, -CONH-, -NHCO-, -NH(C=O)O-, -S-, or SO₂- (provided that adjacent divalent carbon atoms are not replaced at the same time).

The "C₃₋₁₈ cycloalkyl group" is, for example, but not limited to, a cyclopropyl group, a cyclobutyl group, a cyclopentyl group, a 1-i-propylcyclopentane-1-yl group, a cyclohexyl group, a t-butylcyclohexyl group, a tricyclodecanyl group, a cycloheptyl group, a cyclooctyl group, a cyclodecyl group, a 2-methyladamantane-2-yl group, a 2-i-propyladamantane-2-yl group, a bornyl group, a norbornyl group, a fenchyl group, a pinanyl group, an adamantyl group, a tricyclodecyl group, a tetracyclododecyl group, a cyclopropylmethyl group, a cyclobutylmethyl group, a cyclopentylmethyl group, a cyclohexylmethyl group, a bornylmethyl group, a norbornylmethyl group, an adamantylmethyl group, a 1-methylcyclopentyloxycarbonylmethyl group, a cyclopentenyl group, a cyclohexenyl group, a cycloheptenyl group, or the like.

The term "3- to 18-membered non-aromatic heterocyclic group", as used herein, refers to a 3- to 18-membered non-aromatic heterocyclic group containing one or more heteroatoms selected from the group consisting of a nitrogen atom, an oxygen atom, and a sulfur atom, may be monocyclic, polycyclic, or condensed, and may be saturated or partially unsaturated.

The "3- to 18-membered non-aromatic heterocyclic group" is, for example, but not limited to, an aziridinyl group, an azetidil group, a pyrrolidinyl group, a pyrrolyl group, a piperidinyl group, a piperazinyl group, a morpholinyl group, a thiomorpholinyl group, a tetrahydrofuryl group, a tetrahydropyranyl group, an oxetanyl group, a tetrahydrofuryl group, a tetrahydropyranyl group, an imidazolinyl group, an oxazolinyl group, a 2,5-diazabicyclo[2.2.1]heptyl group, a 2,5-diazabicyclo[2.2.2]octyl group, a 3,8-diazabicyclo[3.2.1]octyl group, a 1,4-diazabicyclo[4.3.0]nonyl group, a 1-azaadamantyl group, a 2-azaadamantyl group, or the like.

The term "C₂₋₁₈ aryl group", as used herein, refers to an aromatic hydrocarbon ring group with 6 to 18 carbon atoms or an aromatic heterocyclic group with 2 to 10 carbon atoms; in the case of an aromatic heterocyclic group, 2 to 10 carbon atoms and one or more heteroatoms selected from the group consisting of a nitrogen atom, an oxygen atom, and a sulfur atom form a ring, and the ring may be monocyclic, polycyclic, or a fused ring.

The "C₂₋₁₈ aryl group" is, for example, but not limited to, a phenyl group, a 1-naphthyl group, a 2-naphthyl group, an azulenyl group, a pentalenyl group, a heptalenyl group, an indacenyl group, an acenaphthyl group, a phenanthrenyl group, an anthracenyl group, or the like.

The term "C₇₋₁₈ aralkyl group", as used herein, refers to a group in which a substitutable portion of a "C₁₋₁₂ alkyl group" is substituted by the "C₂₋₁₂ aryl group".

The "C₇₋₁₈ aralkyl group" is, for example, but not limited to, a benzyl group, a phenethyl group, a 3-phenylpropyl group, a 4-phenylbutyl group, a 1-naphthylmethyl group, or a 2-naphthylmethyl group, or the like.

The term "C₁₋₁₈ hydrocarbyl group", as used herein, refers to a monovalent group formed by removing one hydrogen atom from a hydrocarbon with 1 to 18 carbon atoms. Examples of the hydrocarbyl group include an alkyl group, an alkenyl group, an alkynyl group, an alicyclic group, an aryl group, an aralkyl group, and the like.

A divalent carbon atom at any position excluding the terminals contained in the "C₁₋₁₈ hydrocarbyl group" may be replaced by -O-, -C(=O)-, -C(=O)O-, -OCO-, -CONH-, - NHCO-, -NH(C=O)O-, -S-, or -SO₂- (provided that adjacent divalent carbon atoms are not replaced at the same time). The same applies to the "C₁₋₁₈ hydrocarbyl group" and the like used in the following description of the definition of the "C₁₋₁₈ hydrocarbyloxy group" and the like.

The "C₁₋₁₈ hydrocarbyl group" is, for example, but not limited to, a "C₁₋₁₈ alkyl group", a "C₂₋₁₈ alkenyl group", a "C₂₋₁₈ alkynyl group", a "C₃₋₁₈ alicyclic group", a "C₂₋₁₈ aryl group", a "C₇₋₁₈ aralkyl group", or the like.

The term "C₁₋₁₈ hydrocarbyloxy group", as used herein, refers to a group in which an oxygen atom (-O-) is bonded to the "C₁₋₁₈ hydrocarbyl group".

The "C₁₋₁₈ hydrocarbyloxy group" is, for example, but not limited to, a "C₁₋₁₈ alkoxy group", such as a methoxy group, an ethoxy group, a n-propoxy group, an i-propoxy group, a n-butoxy group, an i-butoxy group, a sec-butoxy group, a t-butoxy group, a n-pentoxy group, an i-pentoxy group, a sec-pentoxy group, a n-hexoxy group, an i-hexoxy group, a 1,1-dimethylpropyloxy group, a 1,2-dimethylpropyloxy group, a 2,2-dimethylpropyloxy group, a 1-methyl-2-ethylpropyloxy group, a 1-ethyl-2-methylpropyloxy group, a 1,1,2-trimethylpropyloxy group, a 1,2,2-trimethylpropyloxy group, a 1,1-dimethylbutyloxy group, a 1,2-dimethylbutyloxy group, a 2,2-dimethylbutyloxy group, a 2,3-dimethylbutyloxy group, a 1,3-dimethylbutyloxy group, a 2-ethylbutyloxy group, a 2-methylpentyloxy group, or a 3-methylpentyloxy group; a "C₃₋₁₈ alicyclic oxy group", such as a cyclopropyloxy group, a cyclobutyloxy group, a cyclopentyloxy group, a cyclohexyloxy group, a cycloheptyloxy group, a cyclooctyloxy group, a 1-methylcyclopentyloxycarbonylmethoxy group, a 1-ethylcyclohexyloxycarbonylmethoxy group, or a 1-methyladamantyloxycarbonylmethoxy group; a "C₆₋₁₈ aryloxy group", such as a phenyloxy group, a 1-naphthyloxy group, a 2-naphthyloxy group, an azulenyloxy group, a pentalenyloxy group, a heptalenyloxy group, an indacenyloxy group, an acenaphthyloxy group, a phenanthrenyloxy group, or an anthracenyloxy group; or the like.

In the "C₁₋₁₈ hydrocarbyloxy group", a divalent carbon atom at any position excluding the terminals in the "C₁₋₁₈ hydrocarbyl group" is preferably replaced by -O-, - C(=O)-, and/or -C(=O)O-, the "C₁₋₁₈ hydrocarbyloxy group" is more preferably a "C₁₋₁₈ hydrocarbyloxycarbonylalkyloxy group", and from the perspective of solubility, a carbon atom bonded to the oxygen atom of the hydrocarbyloxy is still more preferably a tertiary carbon. Specific examples of the hydrocarbyloxy include optionally substituted ethylcyclopentyloxy, methyladamantyloxy, ethyladamantyloxy, t-butyloxy, and the like.

The term "C₁₋₁₈ hydrocarbylcarbonyl group", as used herein, refers to a group in which a carbonyl group (-C(=O)-) is bonded to the "C₁₋₁₈ hydrocarbyl group".

The "C₁₋₁₈ hydrocarbylcarbonyl group" is, for example, but not limited to, a "C₁₋₁₈ alkyl carbonyl group", such as an acetyl group, a propionyl group, an isopropionyl group, a butyryl group, an isobutyryl group, a valeryl group, an isovaleryl group, a pentanoyl group, a 3-methylbutanoyl group, a pivaloyl group, a hexanoyl group, or a heptanoyl group; a "C₃₋₁₈ alicyclic carbonyl group", such as a cyclopropyl carbonyl group, a cyclobutyl carbonyl group, a cyclopentyl carbonyl group, a 2-methylcyclopentyl carbonyl group, a 3-methylcyclopentyl carbonyl group, a cyclohexyl carbonyl group, a 2-methylcyclohexyl carbonyl group, a 3-methylcyclohexyl carbonyl group, a 4-methylcyclohexyl carbonyl group, or an adamantyl carbonyl group; a "C₆₋₁₈ aryl carbonyl group", such as a benzoyl group, a 1-naphthoyl group, or a 2-naphthoyl group; or the like.

The term "C₁₋₁₈ hydrocarbylcarbonyloxy group", as used herein, refers to a group in which an oxygen atom (-O-) is bonded to the "C₁₋₁₈ hydrocarbylcarbonyl group".

The "C₁₋₁₈ hydrocarbylcarbonyloxy group" is, for example, but not limited to, a "C₁₋₁₈ alkyl carbonyloxy group", such as a methyl carbonyloxy group, an ethyl carbonyloxy group, a n-propyl carbonyloxy group, an isopropyl carbonyloxy group, a n-butyl carbonyloxy group, an isobutyl carbonyloxy group, a t-butyl carbonyloxy group, a n-pentyl carbonyloxy group, an isopentyl carbonyloxy group, or a hexyl carbonyloxy group; a "C₃₋₁₈ alicyclic carbonyloxy group", such as a cyclopropyl carbonyloxy group, a cyclobutyl carbonyloxy group, a cyclopentyl carbonyloxy group, or a cyclohexyl carbonyloxy group; a "C₆₋₁₈ aryl carbonyloxy group", such as a phenyl carbonyloxy group, a naphthyl carbonyloxy group, an acenaphthyl carbonyloxy group, a phenanthrenyl carbonyloxy group, or an anthracenyl carbonyloxy group; or the like.

The term "C₁₋₁₈ hydrocarbyloxycarbonyl group", as used herein, refers to a group in which a carbonyl group (-C(=O)-) is bonded to a "C₁₋₁₈ hydrocarbyloxy group". The "C₁₋₁₈ hydrocarbyloxycarbonyl group" is, for example, but not limited to, a "C₁₋₁₈ alkoxycarbonyl group", such as a methoxycarbonyl group, an ethoxycarbonyl group, a n-propoxycarbonyl group, an i-propoxycarbonyl group, a n-butoxycarbonyl group, an i-butoxycarbonyl group, a sec-butoxycarbonyl group, a t-butoxycarbonyl group, a n-pentoxycarbonyl group, or a neopentyloxycarbonyl group; a "C₃₋₁₈ alicyclic oxycarbonyl group", such as a cyclopropyloxycarbonyl group, a cyclobutyloxycarbonyl group, a cyclopentyloxycarbonyl group, a cyclohexyloxycarbonyl group, a 2-methylcyclopentyloxycarbonyl group, a 3-methylcyclopentyloxycarbonyl group, a 2-methylcyclohexyloxycarbonyl group, a 3-methylcyclohexyloxycarbonyl group, or a 4-methylcyclohexyloxycarbonyl group; a "C₆₋₁₈ aryloxycarbonyl group", such as a phenoxycarbonyl group, a naphthoxycarbonyl group, an acenaphthyloxycarbonyl group, a phenanthrenyloxycarbonyl group, or an anthracenyloxycarbonyl group; or the like.

The term "C₁₋₁₈ hydrocarbyloxycarbonyloxy group", as used herein, refers to a group in which an oxygen atom (-O-) is bonded to the "C₁₋₁₈ hydrocarbyloxycarbonyl group".

The "C₁₋₁₈ hydrocarbyloxycarbonyloxy group" is, for example, but not limited to, a "C₁₋₁₈ alkoxycarbonyloxy group", such as a methoxycarbonyloxy group, an ethoxycarbonyloxy group, a n-propyloxycarbonyloxy group, an i-propyloxycarbonyloxy group, a n-butoxycarbonyloxy group, an i-butoxycarbonyloxy group, a sec-butoxycarbonyloxy group, a t-butoxycarbonyloxy group, a n-pentyloxycarbonyloxy group, an i-pentyloxycarbonyloxy group, or a n-hexyloxycarbonyloxy group; a "C₃₋₁₈ alicyclic oxycarbonyloxy group", such as a cyclopropyloxycarbonyloxy group, a cyclobutyloxycarbonyloxy group, a cyclopentyloxycarbonyloxy group, or a cyclohexyloxycarbonyloxy group; a "C₆₋₁₈ aryloxycarbonyloxy group", such as a phenoxycarbonyloxy group, a naphthoxycarbonyloxy group, an acenaphthyloxycarbonyloxy group, a phenanthrenyloxycarbonyloxy group, or an anthracenyloxycarbonyloxy group; or the like.

The term "C₁₋₁₈ hydrocarbylamino group", as used herein, refers to a group in which one "C₁₋₁₈ hydrocarbyl group" is bonded to an amino group.

The "C₁₋₁₈ hydrocarbylamino group" is, for example, but not limited to, a "C₁₋₁₈ alkylamino group", such as a methylamino group, an ethylamino group, a n-propylamino group, an i-propylamino group, a n-butylamino group, an i-butylamino group, a sec-butylamino group, a t-butylamino group, a n-pentylamino group, an i-pentylamino group, a neopentylamino group, or a n-hexylamino group; a "C₃₋₁₈ alicyclic amino group", such as a cyclopropylamino group, a cyclobutylamino group, a cyclopentylamino group, a 2-methylcyclopentylamino group, a 3-methylcyclopentylamino group, a cyclohexylamino group, a 2-methylcyclohexylamino group, a 3-methylcyclohexylamino group, or a 4-methylcyclohexylamino group; a "C₆₋₁₈ arylamino group", such as a phenylamino group, a 1-naphthylamino group, or a 2-naphthylamino group; or the like.

The term "di-C₁₋₁₈ hydrocarbylamino group", as used herein, refers to a group in which two identical or different "C₁₋₁₈ hydrocarbyl groups" are bonded to an amino group.

The "di-C₁₋₁₈ hydrocarbylamino group" is, for example, but not limited to, a "di-C₁₋₁₈ alkylamino group", such as a dimethylamino group, a diethylamino group, a di-n-propylamino group, a diisopropylamino group, a di-n-butylamino group, a diisobutylamino group, a di-t-butylamino group, a di-n-pentylamino group, a di-n-hexylamino group, an N-ethyl-N-methylamino group, an N-methyl-N-n-propylamino group, an N-isopropyl-N-methylamino group, an N-n-butyl-N-methylamino group, an N-isobutyl-N-methylamino group, an N-t-butyl-N-methylamino group, an N-methyl-N-n-pentylamino group, an N-n-hexyl-N-methylamino group, an N-ethyl-N-n-propylamino group, an N-ethyl-N-isopropylamino group, an N-n-butyl-N-ethylamino group, an N-ethyl-N-isobutylamino group, an N-t-butyl-N-ethylamino group, an N-ethyl-N-n-pentylamino group, or an N-ethyl-N-n-hexylamino group; a "di-C₃₋₁₈ alicyclic amino group", such as a dicyclopropylamino group, a dicyclobutylamino group, a dicyclopentylamino group, or a dicyclohexylamino group; a "di-C₆₋₁₈ arylamino group", such as a diphenylamino group or a phenylnaphthylamino group; an "N-C₁₋₁₈ alkyl-N-C₃₋₁₈ cycloalkylamino group", such as an N-methylcyclopentaneamino group or an N-methylcyclohexylamino group; an "N-C₁₋₁₈ alkyl-N-C₆₋₁₈ arylamino group", such as an N-methyl-2-phenylethylamino group or an N-ethyl-N-(4-methylphenyl)amino group; or the like.

The term "C₁₋₁₈ hydrocarbylaminocarbonyl group", as used herein, refers to a group in which a carbonyl group (-C(=O)-) is bonded to the "C₁₋₁₈ hydrocarbylamino group".

The "C₁₋₁₈ hydrocarbylaminocarbonyl group" is, for example, but not limited to, a "C₁₋₁₈ alkylaminocarbonyl group", such as a methylaminocarbonyl group, an ethylaminocarbonyl group, a n-propylaminocarbonyl group, an i-propylaminocarbonyl group, a n-butylaminocarbonyl group, a sec-butylaminocarbonyl group, a t-butylaminocarbonyl group, a n-pentylaminocarbonyl group, a 2-pentylaminocarbonyl group, a neopentylaminocarbonyl group, a 4-methyl-2-pentylaminocarbonyl group, a n-hexylaminocarbonyl group, or a 3-methyl-n-pentylaminocarbonyl group; a "C₃₋₁₈ alicyclic aminocarbonyl group", such as a cyclopropylaminocarbonyl group, a cyclobutylaminocarbonyl group, a cyclopentylaminocarbonyl group, a cyclohexylaminocarbonyl group, a 2-methylcyclopentylaminocarbonyl group, a 3-methylcyclopentylaminocarbonyl group, a 2-methylcyclohexylaminocarbonyl group, a 3-methylcyclohexylaminocarbonyl group, or a 4-methylcyclohexylaminocarbonyl group; or a "C₆₋₁₈ arylaminocarbonyl group", such as a phenylaminocarbonyl group, a 1-naphthylaminocarbonyl group, or a 2-naphthylaminocarbonyl group.

The term "di-C₁₋₁₈ hydrocarbylaminocarbonyl group", as used herein, refers to a group in which a carbonyl group (-C(=O)-) is bonded to a "di-C₁₋₁₈ hydrocarbylamino group".

The "di-C₁₋₁₈ hydrocarbylaminocarbonyl group" is, for example, but not limited to, a "di-C₁₋₁₈ alkylamino group", such as a dimethylaminocarbonyl group, a diethylaminocarbonyl group, a di-n-propylaminocarbonyl group, a diisopropylaminocarbonyl group, a di-n-butylaminocarbonyl group, a diisobutylaminocarbonyl group, a di-t-butylaminocarbonyl group, a di-n-pentylaminocarbonyl group, a di-n-hexylaminocarbonyl group, an N-ethyl-N-methylaminocarbonyl group, an N-methyl-N-n-propylaminocarbonyl group, an N-isopropyl-N-methylaminocarbonyl group, an N-n-butyl-N-methylaminocarbonyl group, an N-isobutyl-N-methylaminocarbonyl group, an N-t-butyl-N-methylaminocarbonyl group, an N-methyl-N-n-pentylaminocarbonyl group, an N-n-hexyl-N-methylaminocarbonyl group, an N-ethyl-N-n-propylaminocarbonyl group, an N-ethyl-N-isopropylaminocarbonyl group, an N-n-butyl-N-ethylaminocarbonyl group, an N-ethyl-N-isobutylaminocarbonyl group, an N-t-butyl-N-ethylaminocarbonyl group, an N-ethyl-N-n-pentylaminocarbonyl group, or an N-ethyl-N-n-hexylaminocarbonyl group; a "di-C₃₋₁₈ alicyclic aminocarbonyl group", such as a dicyclopropylaminocarbonyl group, a dicyclobutylaminocarbonyl group, a dicyclopentylaminocarbonyl group, or a dicyclohexylaminocarbonyl group; a "di-C₆₋₁₈ arylaminocarbonyl group", such as a diphenylaminocarbonyl group or a phenylnaphthylaminocarbonyl group; or the like.

The term "C₁₋₁₈ hydrocarbylcarbonylamino group", as used herein, refers to a group in which an amino group is bonded to the "C₁₋₁₈ hydrocarbylcarbonyl group".

The "C₁₋₁₈ hydrocarbylcarbonylamino group" is, for example, but not limited to, a "C₁₋₁₈ alkyl carbonylamino group", such as a methyl carbonylamino group, an ethyl carbonylamino group, a n-propyl carbonylamino group, an i-propyl carbonylamino group, a n-butyl carbonylamino group, an i-butyl carbonylamino group, a sec-butyl carbonylamino group, a t-butyl carbonylamino group, a n-pentyl carbonylamino group, an i-pentyl carbonylamino group, or a n-hexyl carbonylamino group; a "C₃₋₁₈ alicyclic carbonylamino group", such as a cyclopropyl carbonylamino group, a cyclobutyl carbonylamino group, a cyclopentyl carbonylamino group, or a cyclohexyl carbonylamino group; a "C₆₋₁₈ aryl carbonylamino group", such as a phenyl carbonylamino group, a naphthyl carbonylamino group, an acenaphthyl carbonylamino group, a phenanthrenyl carbonylamino group, or an anthracenyl carbonyl amino group; or the like.

The term "C₁₋₁₈ hydrocarbylaminocarbonyloxy group", as used herein, refers to a group in which an oxygen atom (-O-) is bonded to the "C₁₋₁₈ hydrocarbylaminocarbonyl group".

The "C₁₋₁₈ hydrocarbylaminocarbonyloxy group" is, for example, but not limited to, a "C₁₋₁₈ alkylaminocarbonyloxy group", such as a methylaminocarbonyloxy group, an ethylaminocarbonyloxy group, or a n-propylaminocarbonyloxy group; a "C₃₋₁₈ alicyclic aminocarbonyloxy group", such as a cyclopropylaminocarbonyloxy group or a cyclohexylaminocarbonyloxy group; a "C₆₋₁₈ arylaminocarbonyloxy group", such as a phenylaminocarbonyloxy group or a 1-naphthylaminocarbonyloxy group; or the like.

The term "di-C₁₋₁₈ hydrocarbylaminocarbonyloxy group", as used herein, refers to a group in which an oxygen atom (-O-) is bonded to the "di-C₁₋₁₈ hydrocarbylaminocarbonyl group".

The "di-C₁₋₁₈ hydrocarbylaminocarbonyloxy group" is, for example, but not limited to, a "di-C₁₋₁₈ alkylaminocarbonyloxy group", such as a dimethylaminocarbonyloxy group, a diethylaminocarbonyloxy group, or a di-n-propylaminocarbonyloxy group; or the like.

The term "C₁₋₁₈ hydrocarbylaminocarbonylamino group", as used herein, refers to a group in which the "C₁₋₁₈ hydrocarbylaminocarbonyl group" is bonded to an amino group.

The "C₁₋₁₈ hydrocarbylaminocarbonylamino group" is, for example, but not limited to, a "C₁₋₁₈ alkylaminocarbonylamino group", such as a methylaminocarbonylamino group, an ethylaminocarbonyloxy group, or a n-propylaminocarbonylamino group; a "C₃₋₁₈ alicyclic aminocarbonylamino group", such as a cyclopropylaminocarbonylamino group or a cyclohexylaminocarbonylamino group; a "C₆₋₁₈ arylaminocarbonylamino group", such as a phenylaminocarbonylamino group or a 1-naphthylaminocarbonylamino group; or the like.

The term "di-C₁₋₁₈ hydrocarbylaminocarbonylamino group", as used herein, refers to a group in which a "di-C₁₋₁₈ hydrocarbylaminocarbonyl group" is bonded to an amino group.

The "di-C₁₋₁₈ hydrocarbylaminocarbonylamino group" is, for example, but not limited to, a "di-C₁₋₁₈ alkylaminocarbonylamino group", such as a dimethylaminocarbonylamino group, a diethylaminocarbonylamino group, or a di-n-propylaminocarbonylamino group; or the like.

The term "C₁₋₁₈ hydrocarbyloxycarbonylamino group", as used herein, refers to a group in which the "C₁₋₁₈ hydrocarbyloxycarbonyl group" is bonded to an amino group.

The "C₁₋₁₈ hydrocarbyloxycarbonylamino group" is, for example, but not limited to, a "C₁₋₁₈ alkoxycarbonylamino group", such as a methoxycarbonylamino group, an ethoxycarbonylamino group, a n-propoxycarbonylamino group, an i-propoxycarbonylamino group, a n-butoxycarbonylamino group, or a t-butoxycarbonylamino group; a "C₃₋₁₈ alicyclic oxycarbonylamino group", such as a cyclopropyloxycarbonylamino group or a cyclohexyloxycarbonylamino group; a "C₆₋₁₈ aryloxycarbonylamino group", such as a phenyloxycarbonylamino group or a 1-naphthyloxycarbonylamino group; or the like.

The term "C₁₋₁₈ hydrocarbylthio group", as used herein, refers to a group in which a sulfur atom (-S-) is bonded to the "C₁₋₁₈ hydrocarbyl group".

The "C₁₋₁₈ hydrocarbylthio group" is, for example, but not limited to, a "C₁₋₁₈ alkylthio group", such as a methylthio group, an ethylthio group, a n-propylthio group, an i-propylthio group, a n-butylthio group, an i-butylthio group, a t-butylthio group, a n-pentylthio group, or a n-hexylthio group; a "C₃₋₁₈ alicyclic thio group", such as a cyclopropylthio group, a cyclobutylthio group, a cyclopentylthio group, a cyclohexylthio group, a 2-methylcyclopentylthio group, a 3-methylcyclopentylthio group, a 2-methylcyclohexylthio group, a 3-methylcyclohexylthio group, or a 4-methylcyclohexylthio group; a "C₆₋₁₈ arylthio group", such as a phenylthio group, a 1-naphthylthio group, a 2-naphthylthio group, an acenaphthylthio group, a phenanthrenylthio group, or an anthracenylthio group; or the like.

The term "C₁₋₁₈ hydrocarbylsulfinyl group", as used herein, refers to a group in which a sulfinyl group (-S(=O)-) is bonded to the "C₁₋₁₈ hydrocarbyl group".

The "C₁₋₁₈ hydrocarbylsulfinyl group" is, for example, but not limited to, a "C₁₋₁₈ alkylsulfinyl group", such as a methylsulfinyl group, an ethylsulfinyl group, a n-propylsulfinyl group, an i-propylsulfinyl group, a n-butylsulfinyl group, a t-butylsulfinyl group, a pentylsulfinyl group, or a hexylsulfinyl group; a "C₃₋₁₈ alicyclic sulfinyl group", such as a cyclopropylsulfinyl group, a cyclobutylsulfinyl group, a cyclopentylsulfinyl group, a cyclohexylsulfinyl group, a 2-methylcyclopentylsulfinyl group, a 3-methylcyclopentylsulfinyl group, a 2-methylcyclohexylsulfinyl group, a 3-methylcyclohexylsulfinyl group, or a 4-methylcyclohexylsulfinyl group; a "C₆₋₁₈ arylsulfinyl group", such as a phenylsulfinyl group, a naphthylsulfinyl group, an acenaphthylsulfinyl group, a phenanthrenylsulfinyl group, or an anthracenylsulfinyl group; or the like.

The term "C₁₋₁₈ hydrocarbylsulfonyl group", as used herein, refers to a group in which a sulfonyl group (-SO₂-) is bonded to the "C₁₋₁₈ hydrocarbyl group".

The "C₁₋₁₈ hydrocarbylsulfonyl group" is, for example, but not limited to, a "C₁₋₁₈ alkylsulfonyl group", such as a methylsulfonyl group, an ethylsulfonyl group, a n-propylsulfonyl group, an i-propylsulfonyl group, a n-butylsulfonyl group, a t-butylsulfonyl group, or a pentylsulfonyl group; a "C₃₋₁₈ alicyclic sulfonyl group", such as a cyclopropylsulfonyl group, a cyclobutylsulfonyl group, a cyclopentylsulfonyl group, a cyclohexylsulfonyl group, a 2-methylcyclopentylsulfonyl group, a 3-methylcyclopentylsulfonyl group, a 2-methylcyclohexylsulfonyl group, a 3-methylcyclohexylsulfonyl group, or a 4-methylcyclohexyl group; a "C₆₋₁₈ arylsulfonyl group", such as a phenylsulfonyl group, a naphthylsulfonyl group, an acenaphthylsulfonyl group, a phenanthrenylsulfonyl group, or an anthracenylsulfonyl group; or the like.

The term "acid-dissociable group", as used herein, refers to a group that substitutes for a hydrogen atom of a hydroxy group (including a phenolic hydroxy group and the like) or a carboxy group and that is dissociated by the action of an acid.

The acid-dissociable group is, for example, an acid-dissociable group G represented by the following general formula (G-1) or (G-2).

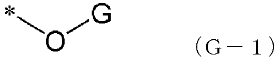

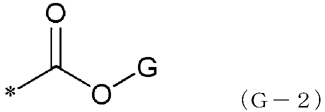

The acid-dissociable group G is not particularly limited, provided that it can be dissociated by the action of an acid, and a known and commonly used group can be used. The acid-dissociable group G is, for example, a "tertiary-carbon-type acid-dissociable group G_{A}" represented by the following general formula (g-1), or the like.

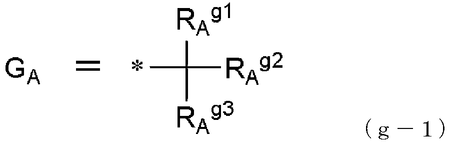

### <Tertiary-carbon-type Acid-Dissociable Group G_{A}>

As the acid-dissociable group G, an acid-dissociable group in which a carbon that is directly bonded to a polar group and to which R_{A}^{g1} to R_{A}^{g3} are bonded is a tertiary carbon atom, such as a "tertiary-carbon-type acid-dissociable group G_{A}" represented by the following general formula (g-1), can be used.

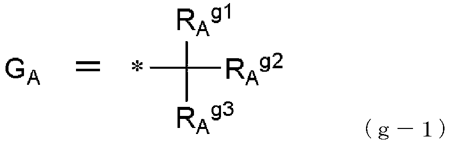

"R_{A}^{g1}" denotes an optionally substituted C₁₋₁₂ hydrocarbyl group in which any divalent carbon atom except the terminal positions may be replaced by -O-, -C(=O)-, - C(=O)O-, -OCO-, -CONH-, -NHCO-, -S-, or -SO₂- (provided that adjacent divalent carbon atoms are not replaced at the same time).

R_{A}^{g1} preferably denotes a C₁₋₁₂ alkyl group, a C₃₋₁₂ alicyclic group, a C₆₋₁₂ aryl group, or a C₇₋₁₂ aralkyl group each optionally having a substituent and any divalent carbon atom except the terminal positions may be replaced by -O-, -C(=O)-, -C(=O)O-, -OCO-, -CONH-, -NHCO-, -S-, or -SO₂- (provided that adjacent divalent carbon atoms are not replaced at the same time),
more preferably a C₁₋₁₂ alkyl group or a C₃₋₁₂ alicyclic group each optionally having a substituent and any divalent carbon atom except the terminal positions may be replaced by -O-, -C(=O)-, -C(=O)O-, -OCO-, -CONH-, -NHCO-, -S-, or -SO₂- (provided that adjacent divalent carbon atoms are not replaced at the same time).

"R_{A}^{g2}" and "R_{A}^{g3}" each independently denote an optionally substituted C₁₋₁₂ hydrocarbyl group, and a divalent carbon atom at any position except the terminal positions may be substituted with -O-, -C(=O)-, -C(=O)O-, -OCO-, -CONH-, -NHCO-, -S-, or -SO₂-(provided that adjacent divalent carbon atoms are not substituted at the same time), or R_{A}^{g2} and R_{A}^{g3} may be combined to be a C₃₋₁₈ alicyclic group or a 3- to 18-membered non-aromatic heterocyclic group, each optionally having a substituent, and a divalent carbon atom at any position except the terminal positions may be substituted with -O-, -C(=O)-, - C(=O)O-, -OCO-, -CONH-, -NHCO-, -S-, or -SO₂- (provided that adjacent divalent carbon atoms are not substituted at the same time).

In R_{A}^{g2} and R_{A}^{g3}, preferably, R_{A}^{g2} and R_{A}^{g3} may be combined to be a C₃₋₁₈ alicyclic group or a 3- to 18-membered non-aromatic heterocyclic group, each optionally having a substituent, and a divalent carbon atom at any position except the terminal positions may be substituted with -O-, -C(=O)-, -C(=O)O-, -OCO-, -CONH-, -NHCO-, -S-, or -SO₂-(provided that adjacent divalent carbon atoms are not substituted at the same time).

More preferably, the substituted C₃₋₁₈ alicyclic group or the 3- to 18-membered non-aromatic heterocyclic group, each optionally having a substituent, has a cyclopentane skeleton, a cyclohexane skeleton, a cycloheptane skeleton, a cyclooctane skeleton, a cyclononane skeleton, a cyclodecane skeleton, a cyclododecane skeleton, a cyclopentene skeleton, a cyclohexene skeleton, a cycloheptene skeleton, a cyclooctene skeleton, a cyclodecene skeleton, a norbornane skeleton, an adamantane skeleton, a tricyclodecane skeleton, a tetracyclododecane skeleton, a norbornene skeleton, or a tricyclodecene skeleton, and a divalent carbon atom at any position except the terminal positions may be substituted with -O-, -C(=O)-, -C(=O)O-, -OCO-, -CONH-, -NHCO-, -S-, or -SO₂- (provided that adjacent divalent carbon atoms are not substituted at the same time).

A tertiary-carbon-type acid-dissociable group G_{A} represented by the general formula (g-1) is, for example, but not limited to, a t-butyl group, a t-amyl group, a 1,1-dimethylpropyl group, a 1-methyl-1-cyclopentyl group, a 1-ethyl-1-cyclopentyl group, a 1-methyl-1-cyclohexyl group, a 1-ethyl-1-cyclohexyl group, a 2-methyl-2-adamantyl group, a 2-ethyl-2-adamantyl group, a 1-(1-methoxy-2-methylpropan-2-yl)cyclopentyl group, a 1-(1-ethoxy-2-methylpropan-2-yl)cyclopentyl group, or the like.

### <<Tertiary-Carbon-Type Acid-Dissociable Group G_{A1} and G_{A2}>>

In the "tertiary-carbon-type acid-dissociable group G_{A}" represented by the general formula (g-1), the case where R_{A}^{g2} and R_{A}^{g3} may be combined to form a C₃₋₁₈ alicyclic group or a 3- to 18-membered non-aromatic heterocyclic group, each optionally having a substituent, is, for example, a tertiary-carbon-type acid-dissociable group G_{A1} represented by the following general formula (g-1-1), a tertiary-carbon-type acid-dissociable group G_{A2} represented by the following general formula (g-1-2), or the like.

### <<Tertiary-Carbon-Type Acid-Dissociable Group G_{A1} Represented by General Formula (g-1-1)>>

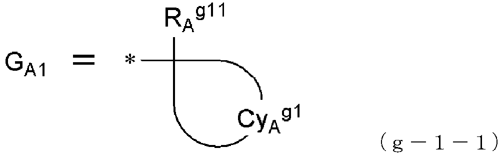

In the tertiary-carbon-type acid-dissociable group G_{A1} represented by the general formula (g-1-1), R_{A}^{g11} denotes a C₁₋₁₂ alkyl group, a C₃₋₁₂ alicyclic group, a C₆₋₁₂ aryl group, or a C₇₋₁₂ aralkyl group, each optionally having a substituent, and any divalent carbon atom except the terminal positions may be substituted with -O-, -C(=O)-, -C(=O)O-, -OCO-, - CONH-, -NHCO-, -S-, or -SO₂- (provided that adjacent divalent carbon atoms are not substituted at the same time).

Cy_{A}^{g1}, together with the tertiary carbon atom, forms a C₃₋₁₈ alicyclic group or a 3- to 18-membered non-aromatic heterocyclic group, each optionally having a substituent, and any divalent carbon atom may be substituted with -O-, -C(=O)-, -C(=O)O-, -OCO-, -CONH-, -NHCO-, -S-, or -SO₂- (provided that adjacent divalent carbon atoms are not substituted at the same time).

Preferably, Cy_{A}^{g1}, together with the tertiary carbon atom, forms a cyclopentane skeleton, a cyclohexane skeleton, a cycloheptane skeleton, a cyclooctane skeleton, a cyclononane skeleton, a cyclodecane skeleton, a cyclododecane skeleton, a cyclopentene skeleton, a cyclohexene skeleton, a cycloheptene skeleton, a cyclooctene skeleton, a cyclodecene skeleton, a norbornane skeleton, an adamantane skeleton, a tricyclodecane skeleton, a tetracyclododecane skeleton, a norbornene skeleton, or a tricyclodecene skeleton, each optionally having a substituent, and a divalent carbon atom at any position except the terminal positions may be substituted with -O-, -C(=O)-, -C(=O)O-, -OCO-, -CONH-, - NHCO-, -S-, or -SO₂- (provided that adjacent divalent carbon atoms are not substituted at the same time).

The tertiary-carbon-type acid-dissociable group G_{A1} represented by the general formula (g-1-1) is, for example, the following tertiary-carbon-type acid-dissociable group.

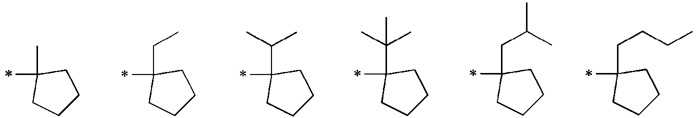

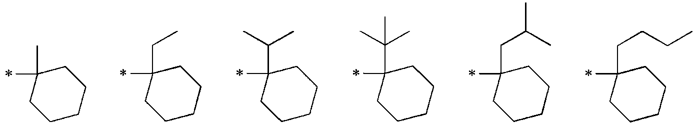

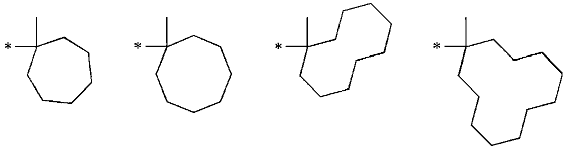

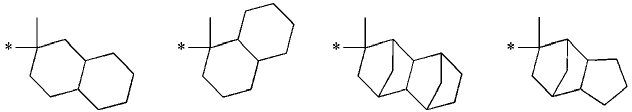

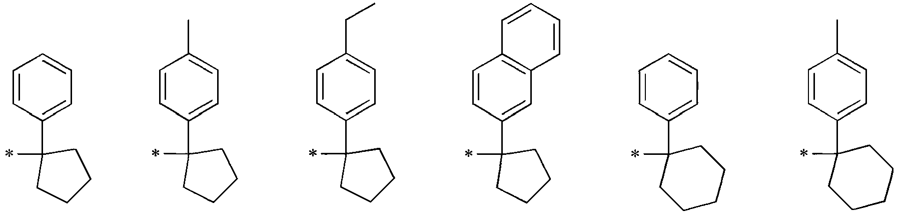

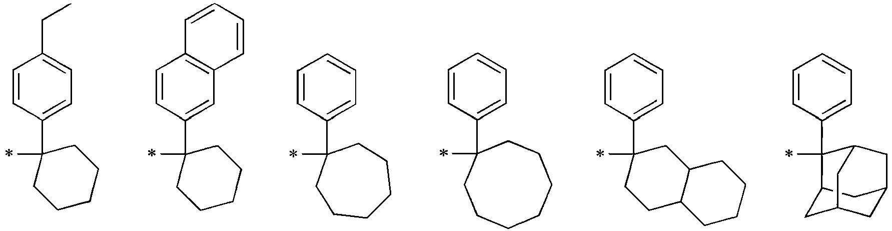

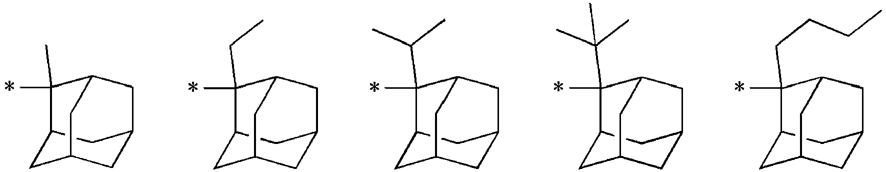

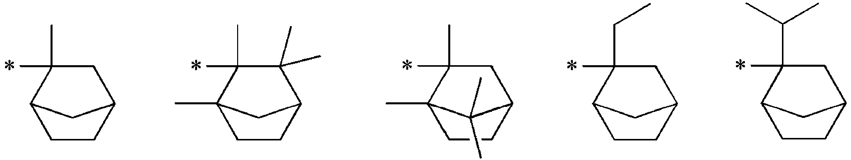

### <<Tertiary-Carbon-Type Acid-Dissociable Group G_{A2} Represented by General Formula (g-1-2)>>

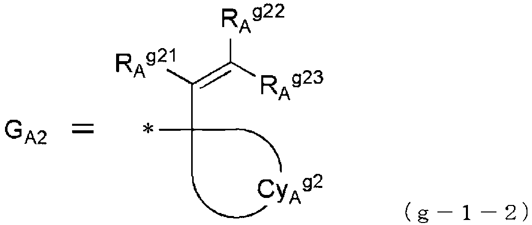

In the general formula (g-1-2), R_{A}^{g21} to R_{A}^{g23} each independently denote a hydro group or an optionally substituted C₁₋₁₂ hydrocarbyl group, any divalent carbon atom except the terminal positions may be substituted with -O-, -C(=O)-, -C(=O)O-, -OCO-, -CONH-, - NHCO-, -S-, or -SO₂- (provided that adjacent divalent carbon atoms are not substituted at the same time), and R_{A}^{g21} and R_{A}^{g22}, and/or R_{A}^{g22} and R_{A}^{g23} may be directly bonded to each other by a single bond or may be bonded to each other via a divalent linker such as, -O-, - S-, -C(=O)-, -S(=O)-, -S(=O)₂-, -C(=O)O-, or a C₁₋₃ alkylene group, to form a ring.

The case where R_{A}^{g21} and R_{A}^{g22}, and/or R_{A}^{g22} and R_{A}^{g23} form a ring together with a carbon atom contained in the ethylenically unsaturated double bond is, for example, a case where they form a cyclopentenyl group, a cyclohexenyl group, a cyclopentylidene ethenyl group, a cyclohexylidene ethenyl group, or the like, each optionally having a substituent.

Cy_{A}^{g2}, together with the tertiary carbon atom, forms a C₃₋₁₈ alicyclic group or a 3- to 18-membered non-aromatic heterocyclic group, each optionally having a substituent, and any divalent carbon atom may be substituted with -O-, -C(=O)-, -C(=O)O-, -OCO-, -CONH-, -NHCO-, -S-, or -SO₂- (provided that adjacent divalent carbon atoms are not substituted at the same time).

Preferably, Cy_{A}^{g2}, together with the tertiary carbon atom, forms a cyclopentane skeleton, a cyclohexane skeleton, a cycloheptane skeleton, a cyclooctane skeleton, a cyclononane skeleton, a cyclodecane skeleton, a cyclododecane skeleton, a cyclopentene skeleton, a cyclohexene skeleton, a cycloheptene skeleton, a cyclooctene skeleton, a cyclodecene skeleton, a norbornane skeleton, an adamantane skeleton, a tricyclodecane skeleton, a tetracyclododecane skeleton, a norbornene skeleton, or a tricyclodecene skeleton, each optionally having a substituent, and a divalent carbon atom at any position except the terminal positions may be substituted with -O-, -C(=O)-, -C(=O)O-, -OCO-, -CONH-, - NHCO-, -S-, or -SO₂- (provided that adjacent divalent carbon atoms are not substituted at the same time).

The tertiary-carbon-type acid-dissociable group G_{A2} represented by the general formula (g-1-2) is, for example, the following tertiary-carbon-type acid-dissociable group.

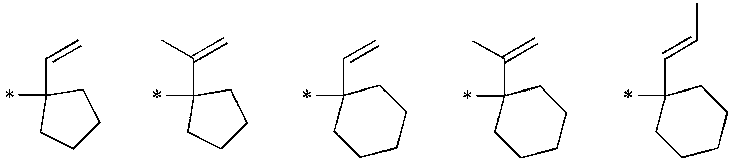

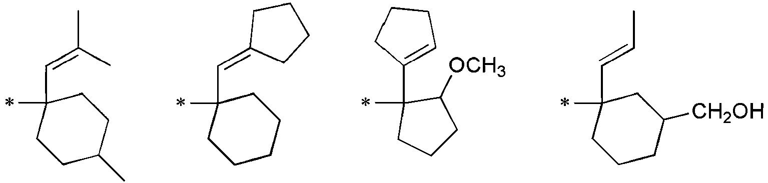

The phrase "a hydroxy group or a carboxy group each having a protective group", as used herein, refers to a hydroxy group (including a phenolic hydroxy group) or a carboxy group in which the hydroxy group or the carboxy group is protected by an ether protective group, a silyl ether protective group, an acetal protective group, an acyl protective group, an oxycarbonyl (alkyl) protective group, an aminocarbonyl protective group, or the like, or refers to a hydroxy group (including a phenolic hydroxy group) or a carboxy group each having an acid-dissociable group.

The ether protective group is, for example, but not limited to, a methyl group, a benzyl group, a p-methoxybenzyl group, a t-butyl group, a triphenylmethyl group, a p-methoxyphenyldiphenylmethyl group, a di(p-methoxyphenyl)phenylmethyl group, or the like.

The silyl ether protective group is, for example, but not limited to, a t-butyldimethylsilyl group (TBS), a triisopropylsilyl group (TIPS), a trimethylsilyl group (TMS), a triethylsilyl group (TES), a t-butyldiphenylsilyl group (TBDPS), or the like.

The acetal protective group is, for example, but not limited to, a methoxymethyl group, an ethoxyethyl group, a 2-tetrahydropyranyl group (THP), a methoxyethoxymethyl group, or the like.

The acyl protective group is, for example, but not limited to, an acetyl group, a pivaloyl group, a benzoyl group, or the like.

The oxycarbonyl (alkyl) protective group is, for example, but not limited to, a t-butoxycarbonyl group or the like.

The aminocarbonyl protective group is, for example, but not limited to, a dimethylaminocarbonyl group, a diethylaminocarbonyl group, a diisopropylaminocarbonyl group, an N-phenyl-N-methyl-aminocarbonyl group, or the like.

The protective group is, for example, a protective group P for a hydroxy group or a carboxy group represented by the following general formula (P-1) or (P-2).

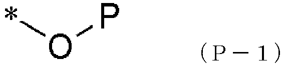

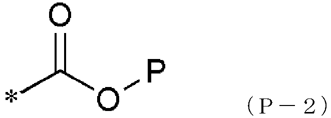

The protective group P is not particularly limited and may be a known and commonly used protective group. The protective group P other than the "acid-dissociable group" is, for example, an "acetal protective group P_{A}" represented by the following general formula (p-1), a "silyl ether protective group P_{B}" represented by the following general formula (p-2), an "aminocarbonyl protective group P_{C}" represented by the following general formula (p-3), or the like. Each of them will be sequentially described below.

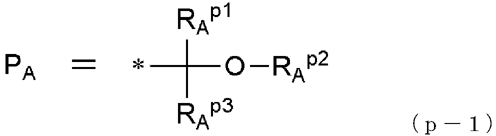

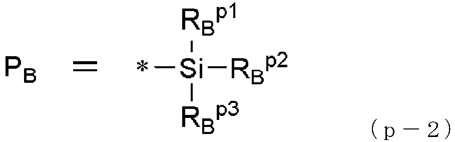

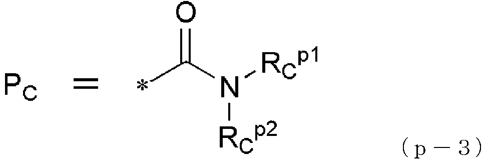

### <Acetal Protective Group P_{A}>

The protective group P may be a protective group in which an oxygen atom is bonded to a carbon atom directly bonded to an oxygen atom of a hydroxy group, a phenolic hydroxy group, or a carboxy group, such as the "acetal protective group P_{A}" represented by the following general formula (p-1).

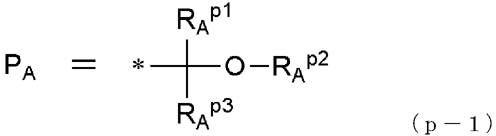

"R_{A}^{p1}" and "R_{A}^{p3}" each independently denote a hydro group or an optionally substituted C₁₋₁₂ hydrocarbyl group, and any divalent carbon atom except the terminal positions may be substituted with -O-, -C(=O)-, -C(=O)O-, -OCO-, -CONH-, -NHCO-, -S-, or -SO₂- (provided that adjacent divalent carbon atoms are not substituted at the same time).

"R_{A}^{p2}" denotes an optionally substituted C₁₋₁₂ hydrocarbyl group, and any divalent carbon atom except the terminal positions may be substituted with -O-, -C(=O)-, -C(=O)O-, -OCO-, -CONH-, -NHCO-, -S-, or -SO₂- (provided that adjacent divalent carbon atoms are not substituted at the same time).

Preferably, R_{A}^{p1} and R_{A}^{p3} denote a hydro group, or a C₁₋₁₂ alkyl group or a C₃₋₁₂ alicyclic group, the alkyl group and the alicyclic group each optionally having a substituent, and any divalent carbon atom except the terminal positions may be substituted with -O-, - C(=O)-, -C(=O)O-, -OCO-, -CONH-, -NHCO-, -S-, or -SO₂- (provided that adjacent divalent carbon atoms are not substituted at the same time),
more preferably a hydro group, or a C₁₋₆ alkyl group or a C₃₋₈ alicyclic group, the alkyl group and the alicyclic group each optionally having a substituent, and any divalent carbon atom except the terminal positions may be substituted with -O-, -C(=O)-, -C(=O)O-, -OCO-, -CONH-, -NHCO-, -S-, or -SO₂- (provided that adjacent divalent carbon atoms are not substituted at the same time).

Preferably, R_{A}^{p2} denotes a C₁₋₁₂ alkyl group or a C₃₋₁₂ alicyclic group, the alkyl group and the alicyclic group each optionally having a substituent, and any divalent carbon atom except the terminal positions may be substituted with -O-, -C(=O)-, -C(=O)O-, -OCO-, - CONH-, -NHCO-, -S-, or -SO₂- (provided that adjacent divalent carbon atoms are not substituted at the same time),
more preferably a C₁₋₆ alkyl group or a C₃₋₈ alicyclic group, the alkyl group and the alicyclic group each optionally having a substituent, and any divalent carbon atom except the terminal positions may be substituted with -O-, -C(=O)-, -C(=O)O-, -OCO-, -CONH-, - NHCO-, -S-, or -SO₂- (provided that adjacent divalent carbon atoms are not substituted at the same time).

The acetal protective group P_{A} represented by the general formula (p-1) is, for example, a methoxymethoxy group, an ethoxymethoxy group, a n-propoxymethoxy group, a n-butoxymethoxy group, a 2,2-dimethylpropoxymethoxy group, a 2,2-dimethylbutoxymethoxy group, a cyclohexyloxymethoxy group, a 1-ethoxyethoxy group, a 1-n-butoxyethoxy group, a 1-cyclohexyloxyethoxy group, or the like.

### <Silyl Ether Protective Group P_{B}>

The protective group P may be a protective group in which a silicon atom is directly bonded to an oxygen atom of a hydroxy group, a phenolic hydroxy group, or a carboxy group, such as a "silyl ether protective group P_{B}" represented by the following general formula (p-2).

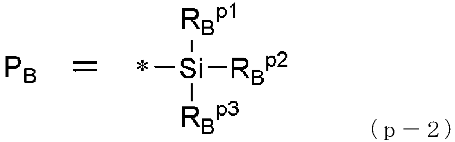

"R_{B}^{p1}" to "R_{B}^{p3}" each independently denote an optionally substituted C₁₋₁₂ hydrocarbyl group in which any divalent carbon atom except the terminal positions may be replaced by -O-, -C(=O)-, -C(=O)O-, -OCO-, -CONH-, -NHCO-, -S-, or -SO₂- (provided that adjacent divalent carbon atoms are not replaced at the same time).

R_{B}^{p1} to R_{B}^{p3} preferably denote a C₁₋₁₂ alkyl group or a C₃₋₁₂ alicyclic group each optionally having a substituent and any divalent carbon atom except the terminal positions may be replaced by -O-, -C(=O)-, -C(=O)O-, -OCO-, -CONH-, -NHCO-, -S-, or -SO₂-(provided that adjacent divalent carbon atoms are not replaced at the same time),
more preferably a C₁₋₆ alkyl group or a C₃₋₈ alicyclic group each optionally having a substituent and any divalent carbon atom except the terminal positions may be replaced by -O-, -C(=O)-, -C(=O)O-, -OCO-, -CONH-, -NHCO-, -S-, or -SO₂- (provided that adjacent divalent carbon atoms are not replaced at the same time).

The silyl ether protective group P_{B} represented by the general formula (p-2) is, for example, but not limited to, a t-butyldimethylsilyl group (TBS), a triisopropylsilyl group (TIPS), a trimethylsilyl group (TMS), a triethylsilyl group (TES), a t-butyldiphenylsilyl group (TBDPS), or the like.

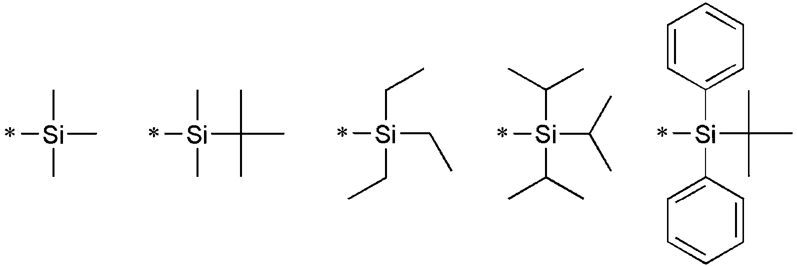

### <Aminocarbonyl Protective Group P_{C}>

The protective group P may be a protective group in which an aminocarbonyl group is bonded to an oxygen atom of a hydroxy group, a phenolic hydroxy group, or a carboxy group, such as an "aminocarbonyl protective group P_{C}" represented by the following general formula (p-3).

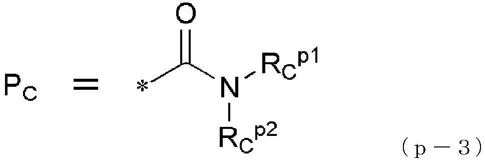

"R_{C}^{p1}" and "R_{C}^{p2}" each independently denote a hydro group, or an optionally substituted C₁₋₁₂ hydrocarbyl group in which any divalent carbon atom except the terminal positions may be replaced by -O-, -C(=O)-, -C(=O)O-, -OCO-, -CONH-, -NHCO-, -S-, or - SO₂- (provided that adjacent divalent carbon atoms are not replaced at the same time).

Preferably, R_{C}^{p1} and R_{C}^{p2} denote a hydro group, or a C₁₋₁₂ alkyl group or a C₃₋₁₂ alicyclic group, the alkyl group and the alicyclic group each optionally having a substituent, and any divalent carbon atom except the terminal positions may be substituted with -O-, - C(=O)-, -C(=O)O-, -OCO-, -CONH-, -NHCO-, -S-, or -SO₂- (provided that adjacent divalent carbon atoms are not substituted at the same time),
more preferably a hydro group, or a C₁₋₆ alkyl group or a C₃₋₈ alicyclic group, the alkyl group and the alicyclic group each optionally having a substituent, and any divalent carbon atom except the terminal positions may be substituted with -O-, -C(=O)-, -C(=O)O-, -OCO-, -CONH-, -NHCO-, -S-, or -SO₂- (provided that adjacent divalent carbon atoms are not substituted at the same time).

The aminocarbonyl protective group P_{C} represented by the general formula (p-3) is, for example, but not limited to, a dimethylaminocarbonyl group, a diethylaminocarbonyl group, a diisopropylaminocarbonyl group, an N-phenyl-N-methyl-aminocarbonyl group, or the like.

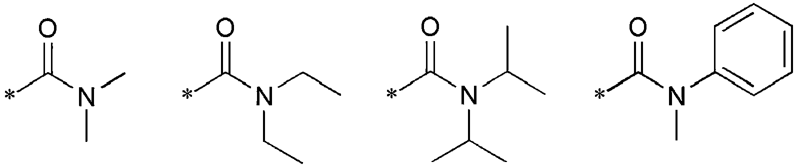

The phrase "optionally substituted" or "optionally having a substituent", as used herein, is not particularly limited provided that it is chemically acceptable and has the advantages of the present invention.

The "substituent" is, for example, (1) a halogen atom, (2) a haloalkyl group, (3) a hydroxy group, (4) a thiol group, (5) a nitro group, (6) a cyano group, (7) a carboxy group, (8) an amino group, (9) a sulfo group, (10) a vinyl group, (11) an allyl group, (12) a (meth)acryloyl group, (13) a (meth)acryloyloxy group, (14) a (meth)acrylamide group, (15) a styryl group, (16) an epoxy group, (17) a glycidyl group, (18) an amide group, (19) a hydroxy group or a carboxy group each having a protective group, or (20) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, a C₁₋₁₈ hydrocarbylthio group, a C₁₋₁₈ hydrocarbylsulfinyl group, or a C₁₋₁₈ hydrocarbylsulfonyl group, in which at least part of hydrogen atoms may be substituted with (1) to (19), and a divalent carbon atom at any position of these substituents except the terminal positions may be substituted with -O-, - C(=O)-, -C(=O)O-, -OCO-, -CONH-, -NHCO-, -NH(C=O)O-, -S-, or -SO₂- (provided that adjacent divalent carbon atoms are not substituted at the same time), or the like.

### [1. Heteropolyacid Salt Having Modified Lacunary Site or Mixture Thereof]

The heteropolyacid salt having the modified lacunary site or a mixture thereof according to the present embodiment is a heteropolyacid salt having a modified lacunary site represented by the general formula (I) or a mixture thereof.

(A^{m+})ₐ(Bⁿ⁺)_{b}(C^{(am+bn)-}) (I)

[wherein
A^{m+} each independently denotes H⁺ or a metal ion,
Bⁿ⁺ each independently denotes an ammonium cation, an ammonium dication, a phosphonium cation, or a phosphonium dication,
C^{(am+bn)-} denotes a heteropolyacid anion having a lacunary site, wherein the lacunary site is modified, and
m denotes an integer in the range of 1 to 5, n denotes an integer of 1 or 2, and a and b denote real numbers]

### [2. Heteropolyacid Anion]

The heteropolyacid anion according to the present embodiment is, for example, a heteropolyoxomolybdate anion, a heteropolyoxotungstate anion, a heteropolyoxovanadate anion, a heteropolyoxoniobate anion, a heteropolyoxotantalate anion, or the like.

The polyatom of the heteropolyacid anion is preferably Mo, W, V, Nb, or Ta, more preferably Mo or W.

The heteropolyoxomolybdate anion is, for example, a phosphomolybdate anion, a silicomolybdate anion, a boromolybdate anion, a phosphotungstomolybdate anion, a cobalt molybdate anion, an arsenic molybdate anion, a germanium molybdate anion, or the like. The heteropolyoxotungstate anion is, for example, a phosphotungstate anion, a silicotungstate anion, a borotungstate anion, a cobalt tungstate anion, an arsenic tungstate anion, a germanium tungstate anion, or the like. The heteropolyoxovanadate anion is, for example, a phosphomolybdovanadate anion, a phosphomolybdotungstomolybdate anion, a boromolybdovanadate anion, a boromolybdotungstovanadate anion, or the like.

The heteroatom of the heteropolyacid anion is preferably P, Si, Ge, B, S, Co, or As, more preferably P, Si, B, S, or Ge.

The heteropolyacid anion includes a Keggin type, a Dawson type, an Anderson type, and various isomers thereof.

### [3. Heteropolyacid Anion Having Lacunary Site]

A heteropolyacid anion having a lacunary site according to the present embodiment may be, but not limited to, a lacunary species having a defect in part of the basic skeleton of the heteropolyacid anion, and any of a mono-lacunary species, a di-lacunary species, a tri-lacunary species may be used. The heteropolyacid anion having the lacunary site is, for example, a lacunary Keggin-type heteropolyacid anion, a lacunary Dawson-type heteropolyacid anion, or the like.

### [3-1. Lacunary Keggin-Type Heteropolyacid Anion]

The lacunary Keggin-type heteropolyacid anion is, for example, a mono-lacunary Keggin-type heteropolyacid anion represented by the following general formula (II-1), a di-lacunary Keggin-type heteropolyacid anion represented by the following general formula (II-2), a tri-lacunary Keggin-type heteropolyacid anion represented by the following general formula (II-3), or the like.

[XM₁₁O₃₉]^{c11-} (II-1)

[XM₁₀O₃₆]^{c12-} (II-2)

[XM₉O₃₄]^{c13-} (II-3)

(wherein
X denotes a heteroatom of P, Si, B, S, or Ge,
M denotes a polyatom of Mo, W, V, Nb, or Ta, and
c11- to c13- denote the number of negative charges, and c11 to c13 denote natural numbers)

In the general formulae (II-1) to (II-3), the values of c11 to c13 vary with the types of X and M.

For example, in the general formula (II-1), when X denotes P, and M denotes Mo or W, c11 denotes 7 ([PMo₁₁O₃₉]⁷⁻, [PW₁₁O₃₉]⁷⁻), when X denotes Si, and M denotes Mo or W, c11 is 8 ([SiMo₁₁O₃₉]⁸⁻, [SiW₁₁O₃₉]⁸⁻), when X denotes B, and M denotes Mo or W, c11 is 9 ([BMo₁₁O₃₉]⁹⁻, [BW₁₁O₃₉]⁹⁻), when X denotes S, and M denotes Mo or W, c11 is 6 ([SMo₁₁O₃₉]⁶⁻, [SW₁₁O₃₉]⁶⁻), and when X denotes Ge, and M denotes Mo or W, c11 is 8 ([GeMo₁₁O₃₉]⁸⁻, [GeW₁₁O₃₉]⁸⁻).

In the general formula (II-2), when X denotes P, and M denotes Mo or W, c12 is 7 ([PMo₁₀O₃₆]⁷⁻, [PW₁₀O₃₆]⁷⁻), when X denotes Si, and M denotes Mo or W, c12 is 8 ([SiMo₁₀O₃₆]⁸⁻, [SiW₁₀O₃₆]⁸⁻), when X denotes B, and M denotes Mo or W, c12 is 9 ([BMo₁₀O₃₆]⁹⁻, [BW₁₀O₃₆]⁹⁻), and when X denotes Ge, and M denotes Mo or W, c12 is 8 ([GeMo₁₀O₃₆]⁸⁻, [GeW₁₀O₃₆]⁸⁻).

In the general formula (II-3), for example, when X denotes P, and M denotes Mo or W, c13 is 9 ([PMo₉O₃₄]⁹⁻, [PW₉O₃₄]⁹⁻), when X denotes Si, and M denotes Mo or W, c13 is 10 ([SiMo₉O₃₄]¹⁰⁻, [SiW₉O₃₄]¹⁰⁻), when X denotes S, and M denotes Mo or W, c13 is 8 ([SMo₉O₃₄]⁸⁻, [SW₉O₃₄]⁸⁻), and when X denotes Ge, and M denotes Mo or W, c13 is 10 ([GeMo₉O₃₄]¹⁰⁻, [GeW₉O₃₄]¹⁰⁻).

The isomer structure of the lacunary Keggin-type heteropolyacid anion is preferably an α-form, a β-form, or a γ-form.

The isomer structure of the mono-lacunary Keggin-type heteropolyacid anion represented by the general formula (II-1) is preferably [α-PW₁₁O₃₉]⁷⁻, [β-PW₁₁O₃₉]⁷⁻, [γ-PW₁₁O₃₉]⁷⁻, [α-PMo₁₁O₃₉]⁷⁻, [β-PMo₁₁O₃₉]⁷⁻, [γ-PW₁₁O₃₉]⁷⁻, [α-SiW₁₁O₃₉]⁸⁻, [β-SiW₁₁O₃₉]⁸⁻, [γ-SiW₁₁O₃₉]⁸⁻, [α-SiMo₁₁O₃₉]⁸⁻, [β-SiMo₁₁O₃₉]⁸⁻, or [γ-SiW₁₁O₃₉]⁸⁻, more preferably [α-PW₁₁O₃₉]⁷⁻, [α-PMo₁₁O₃₉]⁷⁻, [α-SiW₁₁O₃₉]⁸⁻, or [α-SiMo₁₁O₃₉]⁸⁻.

The isomer structure of the di-lacunary Keggin-type heteropolyacid anion represented by the general formula (II-2) is preferably [α-PW₁₀O₃₆]⁷⁻, [β-PW₁₀O₃₆]⁷⁻, [γ-PW₁₀O₃₆]⁷⁻, [α-PMo₁₀O₃₆]⁷⁻, [β-PMo₁₀O₃₆]⁷⁻, [γ-PW₁₀O₃₆]⁷⁻, [α-SiW₁₀O₃₆]⁸⁻, [β-SiW₁₀O₃₆]⁸⁻, [γ-SiW₁₀O₃₆]⁸⁻, [α-SiMo₁₀O₃₆]⁸⁻, [β-SiMo₁₀O₃₆]⁸⁻, or [γ-SiW₁₀O₃₆]⁸⁻, more preferably [γ-PW₁₀O₃₆]⁷⁻, [γ-PMo₁₀O₃₆]⁷⁻, [γ-SiW₁₀O₃₆]⁸⁻, or [γ-SiMo₁₀O₃₆]⁸⁻.

The isomer structure of the tri-lacunary Keggin-type heteropolyacid anion represented by the general formula (II-3) is preferably [α-PW₉O₃₄]⁹⁻, [β-PW₉O₃₄]⁹⁻, [γ-PW₉O₃₄]⁹⁻, [α-PMo₉O₃₄]⁹⁻, [β-PMo₉O₃₄]⁹⁻, [γ-PW₉O₃₄]⁹⁻, [α-SiW₉O₃₄]¹⁰⁻, [β-SiW₉O₃₄]¹⁰⁻, [γ-SiW₉O₃₄]¹⁰⁻, [α-SiMo₉O₃₄]¹⁰⁻, [β-SiMo₉O₃₄]¹⁰⁻, or [γ-SiW₉O₃₄]¹⁰⁻.

### [3-2. Lacunary Dawson-Type Heteropolyacid Anion]

The lacunary Dawson-type heteropolyacid anion is, for example, a mono-lacunary Dawson-type heteropolyacid anion represented by the following general formula (III-1), a di-lacunary Dawson-type heteropolyacid anion represented by the following general formula (III-2), a tri-lacunary Dawson-type heteropolyacid anion represented by the following general formula (III-3), or the like.

[X₂M₁₇O₆₁]^{c21-} (III-1)

[X₂M₁₆O₅₈]^{c22-} (III-2)

[X₂M₁₅O₅₆]^{c23-} (III-3)

(wherein
X denotes a heteroatom of P, Si, B, S, or Ge,
M denotes a polyatom of Mo, W, V, Nb, or Ta, and
c21- to c23- denote the number of negative charges, and c21 to c23 denote natural numbers)

In the general formulae (III-1) to (III-3), the values of c21 to c23 vary with the types of X and M.

For example, in the general formula (III-1), when X denotes P, and M denotes Mo or W, c21 denotes 10 ([P₂Mo₁₇O₆₁]¹⁰⁻, [P₂W₁₇O₆₁]¹⁰⁻), and when X denotes S, and M denotes W, c21 denotes 8 ([S₂W₁₇O₆₁]⁸⁻).

In the general formula (III-2), when X denotes Ge, and M denotes Mo, c22 denotes 12 ([Ge₂Mo₁₆O₅₈]¹²⁻).

In the general formula (III-3), when X denotes P, and M denotes Mo or W, c23 denotes 12 ([P₂Mo₁₅O₅₆]¹²⁻, [P₂W₁₅O₅₆]¹²⁻).

The isomer structure of the lacunary Dawson-type heteropolyacid anion is preferably an α-form, a β-form, or a γ-form.

The isomer structure of the lacunary Dawson-type heteropolyacid anion represented by the general formula (III-1), (III-2), or (III-3) is preferably [α-P₂W₁₇O₆₁]¹⁰⁻, [α-P₂W₁₅O₅₆]¹²⁻, [α-S₂W₁₇O₆₁]⁸⁻, or the like.

### [4. Heteropolyacid Anion Having Lacunary Site]

### [4-1. Modification of Lacunary Site and Notation Thereof]

In the heteropolyacid anion having the modified lacunary site according to the present embodiment, a terminal oxygen atom of the lacunary site of the heteropolyacid anion having the lacunary site is modified with a group having one or more heteroatoms P, Si, Ge, or Sn, to which one or more hydro groups or organic groups are bonded, and the modified portion and the lacunary site of the heteropolyacid anion are bonded via a part or all of the heteroatoms.

The group having one or more heteroatoms P, Si, Ge, or Sn, to which one or more hydro groups or organic groups are bonded, is not particularly limited, and can be represented by, for example, the following general formulae (IV-1) to (IV-5). 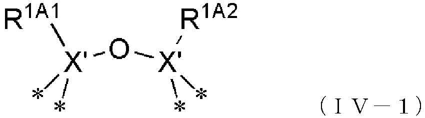 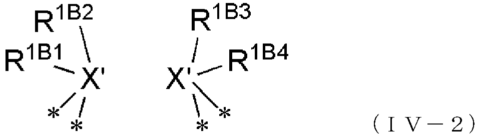 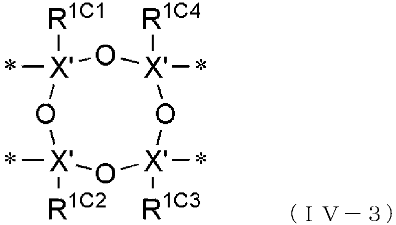 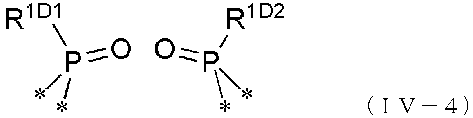 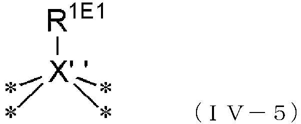 [In the formulae (IV-1) to (IV-5),
X' denotes Si or Ge,
X" denotes a heteroatom of Si, Ge, or Sn,
R^{1A1} to R^{1E1} each independently denote a hydro group, an optionally substituted C₁₋₁₈ hydrocarbyl group, an optionally substituted 3- to 18-membered non-aromatic heterocyclic group, or an optionally substituted 5- to 18-membered aromatic heterocyclic group,
a divalent carbon atom at any position of R^{1A1} to R^{1E1} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{1A1} to R^{1E1} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (1) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group, and
* denotes the binding portion to a terminal oxygen atom of the lacunary site.]

In the present description, for convenience, a heteropolyacid anion having a lacunary site modified with a group having one or more heteroatoms Si or Ge, which is represented by the general formula (IV-1) and to which one or more hydro groups or organic groups are bonded, can be represented by the following general formula (V-1).

[X_{w}MₓO_{y}(R^{1A1}X')(R^{1A2}X')O]^{c-} (V-1)

(wherein w, x, y, and c all denote natural numbers, and X, M, X', R^{1A1}, and R^{1A2} are the same as described above)

When R^{1A1} and R^{1A2} are identical, they can be denoted as R^{1A} in the following general formula (V-1').

[X_{w}MₓO_{y}(R^{1A}X')₂O]^{c-} (V-1')

The heteropolyacid anion having the modified lacunary site represented by the general formula (V-1) or (V-1') is not particularly limited and can be produced by, for example, reacting a heteropolyacid anion having a lacunary site with (R^{1A})X'Y₃ (Y denotes a leaving group, for example, a halogen atom, a hydroxy group, or an alkoxy group (preferably a C₁₋₄ alkoxy group, more preferably a C₁₋₂ alkoxy group), an alkylcarbonyloxy group (preferably a C₁₋₄ alkylcarbonyloxy group), a hydrocarbylsulfonyloxy group (preferably a C₁₋₆ alkyl group or a C₇₋₁₀ aralkylsulfonyloxy group, for example, a methanesulfonyloxy group, a p-toluenesulfonyloxy group, or the like)).

Similarly, in the present description, for convenience, a heteropolyacid anion having a lacunary site modified with a group having one or more heteroatoms Si or Ge, which is represented by the general formula (IV-2) and to which one or more hydro groups or organic groups are bonded, can be represented by the following general formula (V-2).

[X_{w}MₓO_{y}(R^{1B1}R^{1B2}X')(R^{1B3}R^{1B4}X^{'})]^{c-} (V-2)

(wherein w, x, y, and c all denote natural numbers, and X, M, X', R^{1B1}, R^{1B2}, R^{1B3}, and R^{1B4} are the same as described above)

When R^{1B1}R^{1B2} and R^{1B3}R^{1B4} are identical, it can be represented by the following general formula (V-2').

[X_{w}MₓO_{y}(R^{1B1}R^{1B2}Si)₂]^{c-} (V-2')

The heteropolyacid anion having the modified lacunary site represented by the general formula (V-2) or (V-2') is not particularly limited and can be produced by, for example, reacting a heteropolyacid anion having a lacunary site with (R^{1B1})(R^{1B2})X'Y₂ (Y denotes a leaving group in the same manner as described above).

In the present description, for convenience, a heteropolyacid anion having a lacunary site modified with a group having one or more heteroatoms Si or Ge, which is represented by the general formula (IV-3) and to which one or more hydro groups or organic groups are bonded, may be represented by the following general formula (V-3).

[X_{w}MₓO_{y}(R^{1C1}X'O)(R^{1C2}X'O)(R^{1C3}X'O)(R^{1C4}X'O)]^{c-} (V-3)

(wherein w, x, y, and c all denote natural numbers, and X, M, X', R^{1C1}, R^{1C2}, R^{1C3}, and R^{1C4} are the same as described above)

When R^{1C1}, R^{1C2}, R^{1C3}, and R^{1C4} are identical, they can be represented as R^{1C} in the following general formula (V-3').

[X_{w}MₓO_{y}(R^{1C}X'O)₄]^{c-} (V-3')

The heteropolyacid anion having the modified lacunary site represented by the general formula (V-3) or (V-3') is not particularly limited and can be produced by, for example, reacting a di-lacunary Keggin-type or Dawson-type heteropolyacid anion with R^{1C}X'Y₃ (Y denotes a leaving group in the same manner as described above).

In the present description, for convenience, a heteropolyacid anion having a lacunary site modified with a group having one or more heteroatoms P, which is represented by the general formula (IV-4) and to which one or more hydro groups or organic groups are bonded, may be represented by the following general formula (V-4).

[X_{w}MₓO_{y}(R^{1D1}P=O)(R^{1D2}P=O)]^{c-} (V-4)

(wherein w, x, y, and c all denote natural numbers, and X, M, R^{1D1} and R^{1D2} are the same as described above)

When R^{1D1} and R^{1D2} are identical, they can be denoted as R^{1D} in the following general formula (V-4').

[X_{w}MₓO_{y}(R^{1D}P=O)₂]^{c-} (V-4')

The heteropolyacid anion having the modified lacunary site represented by the general formula (V-4) or (V-4') is not particularly limited and can be produced by, for example, reacting a heteropolyacid anion having a lacunary site with R^{1D}P(=O)Y₂ (Y denotes a leaving group in the same manner as described above).

In the present description, for convenience, a heteropolyacid anion having a lacunary site modified with a group having one or more heteroatoms Si, Ge, or Sn, which is represented by the general formula (IV-5) and to which one or more hydro groups or organic groups are bonded, may be represented by the following general formula (V-5).

[X_{w}MₓO_{y}(R^{1E1}X")]^{c-} (V-5)

(wherein w, x, y, and c all denote natural numbers, and X, M, X", and R^{1E1} are the same as described above)

(V-5) may be represented by the following general formula (V-5') in some cases. Note that R^{1E} is defined in the same manner as R^{1E1}.

[X_{w}MₓO_{y}(R^{1E}X")]^{c-} (V-5')

The heteropolyacid anion having the modified lacunary site represented by the general formula (V-5) or (V-5') is not particularly limited and can be produced by, for example, reacting a heteropolyacid anion having a lacunary site with R^{1E}SnX"Y₃ (Y denotes a leaving group in the same manner as described above).

Among the heteropolyacid anions having the modified lacunary site represented by the general formulae (V-1) to (V-5) and (V-1') to (V-5'), heteropolyacid anions having a modified lacunary site represented by the following general formulae (VI-1) to (VI-12) are preferred from the perspective of being relatively stable and capable of controlling the reactivity.

[XM₁₁O₃₉(R^{1A}X')₂O]^{c31-} (VI-1)

[XM₁₁O₃₉(R^{1B1}R^{1B2}X')₂]^{c31-} (VI-2)

[XM₁₁O₃₉(R^{1D}P=O)₂]^{c31-} (VI-3)

[XM₁₁O₃₉(R^{1E}X")]^{c31-} (VI-4)

[XM₁₀O₃₆(R^{1A}X')₂O]^{c32-} (VI-5)

[XM₁₀O₃₆(R^{1B1}R^{1B2}X')₂]^{c32-} (VI-6)

[XM₁₀O₃₆(R^{1c}X'O)₄]^{c32-} (VI-7)

[XM₁₀O₃₆(R^{1D}P=O)₂]^{c32-} (VI-8)

[X₂M₁₇O₆₁(R^{1A}X')₂O]^{c33-} (VI-9)

[X₂M₁₇O₆₁(R^{1B1}R^{1B2}X')₂]^{c33-} (VI-10)

[X₂M₁₇O₆₁(R^{1D}P=O)₂]^{c33-} (VI-11)

[X₂M₁₇O₆₁(R^{1E}X")]^{c33-} (VI-12)

(wherein
X denotes a heteroatom of P, Si, B, S, or Ge,
M denotes a polyatom of Mo, W, V, Nb, or Ta,
X' denotes a heteroatom of Si or Ge,
X" denotes a heteroatom of Si, Ge, or Sn,
R^{1A} to R^{1E} each independently denote a hydro group, an optionally substituted C₁₋₁₈ hydrocarbyl group, an optionally substituted 3- to 18-membered non-aromatic heterocyclic group, or an optionally substituted 5- to 18-membered aromatic heterocyclic group,
a divalent carbon atom at any position of R^{1A} to R^{1E} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{1A} to R^{1E} may be, for example, substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (1) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group, and
c31, c32, and c33 are natural numbers)

In the heteropolyacid anion having the modified lacunary site, since four terminal oxygen atoms of the lacunary site are modified, in general, the value of c31 is c11 - 4, the value of c32 is c12 - 4, and the value of c33 is c21 - 4.

On the other hand, for example, when the organic group contains an amino group and the amino group is protonated to be an ammonium cation, or when the organic group contains a carboxy group and the carboxy group is converted into a carboxy anion, the values of c31, c32, and c33 are different from the above values.

### [4-2. Group Having One or More Heteroatoms P, Si, Ge, or Sn to Which One or More Hydro Groups or Organic Groups Are Bonded]

As described above, the group having one or more heteroatoms P, Si, Ge, or Sn to which one or more hydro groups or organic groups are bonded according to the present embodiment is not particularly limited and can be represented by the following general formulae (VII-1) to (VII-5).

(R^{1A1}X')(R^{1A2}X')O (VII-1)

(R^{1B1}R^{1B2}X')(R^{1B3}R^{1B4}X') (VII-2)

(R^{1C1}X'O)(R^{1C2}X'O)(R^{1C3}X'O)(R^{1C4}X'O) (VII-3)

(R^{1D1}P=O)(R^{1D2}P=O) (VII-4)

(R^{1E1}X") (VII-5)

[wherein
X' denotes a heteroatom of Si or Ge,
X" denotes a heteroatom of Si, Ge, or Sn,
R^{1A1} to R^{1E1} each independently denote a hydro group, an optionally substituted C₁₋₁₈ hydrocarbyl group, an optionally substituted 3- to 18-membered non-aromatic heterocyclic group, or an optionally substituted 5- to 18-membered aromatic heterocyclic group,
a divalent carbon atom at any position of R^{1A1} to R^{1E1} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
a hydrogen atom in R^{1A1} to R^{1E1} may be substituted with, for example, (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (1) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group]

### <Organic Group Having One or More Substituents, such as Hydroxy Group, Halogen Atom, or Epoxy Group>

From the perspective of providing a functional building block, R^{1A1} to R^{1E1} in the general formulae (VII-1) to (VII-5) are
preferably optionally substituted C₁₋₁₈ hydrocarbyl groups; any divalent carbon atom at a non-terminal position of R^{1A1} to R^{1E1} may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and one or more hydrogen atoms in R^{1A1} to R^{1E1} is substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (p) an epoxy group, (q) a glycidyl group, or (r) an amide group,
more preferably C₁₋₁₈ alkyl groups, C₃₋₁₈ alicyclic groups, or C₇₋₁₈ aralkyl groups, each optionally having a substituent; any divalent carbon atom at a non-terminal position of R^{1A1} to R^{1E1} may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and one or more hydrogen atoms in R^{1A1} to R^{1E1} are substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (p) an epoxy group, (q) a glycidyl group, or (r) an amide group.

Specific examples of R^{1A1} to R^{1E1} are not particularly limited and include a 1-glycidyloxypropan-3-yl group, a 1-glycidyloxy-n-octan-8-yl group, a 1-aminopropan-3-yl group, a 1-(2-aminoethylamino)-propan-3-yl group, a 3,3,3-trifluoropropan-1-yl group, a chloromethyl group, a 1-chloropropan-3-yl group, a 1-bromopropan-3-yl group, a 1-iodopropan-3-yl group, and the like.

### <Organic Group Having One or More Ethylenically Unsaturated Double Bonds>

Furthermore, from the perspective of providing a building block having actinic radiation reactivity, the organic group preferably includes at least one ethylenically unsaturated double bond.

That is, R^{1A1} to R^{1E1} in the general formulae (IV-1) to (IV-5) are
preferably optionally substituted C₁₋₁₈ hydrocarbyl groups; any divalent carbon atom at a non-terminal position of R^{1A1} to R^{1E1} may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and one or more hydrogen atoms in R^{1A1} to R^{1E1} are substituted with (j) a vinyl group, (k) an allyl group, (1) a (meth)acryloyl group, (m) a (meth)acryloyloxy group, (n) a (meth)acrylamide group, or (o) a styryl group,
more preferably C₁₋₁₈ alkyl groups, C₃₋₁₈ alicyclic groups, or C₇₋₁₈ aralkyl groups, each optionally having a substituent; any divalent carbon atom at a non-terminal position of R^{1A1} to R^{1E1} may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and one or more hydrogen atoms in R^{1A1} to R^{1E1} are substituted with (j) a vinyl group, (k) an allyl group, (1) a (meth)acryloyl group, (m) a (meth)acryloyloxy group, (n) a (meth)acrylamide group, or (o) a styryl group.

Specific examples of the organic groups R^{1A1} to R^{1E1} having the ethylenically unsaturated double bond are not particularly limited and include a vinyl group, an allyl group, a 5-hexen-1-yl group, a 6-hepten-1-yl group, a 7-octen-1-yl group, a 1-acryloyloxypropan-3-yl group, a 1-methacryloyloxypropan-3-yl group, a 1-vinylbenzene-4-yl group, and the like.

### <Organic Group Having One or More Hydroxy Groups or Carboxy Groups Having Protective Group>

Furthermore, from the perspective of providing a building block whose functionality, such as solubility, is changed by deprotection due to the action of an acid or the like, the organic group preferably has one or more hydroxy groups or carboxy groups, each having a protective group.

That is, R^{1A1} to R^{1E1} in the general formulae (IV-1) to (IV-5) are
preferably optionally substituted C₁₋₁₈ hydrocarbyl groups; any divalent carbon atom at a non-terminal position of R^{1A1} to R^{1E1} may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and one or more hydrogen atoms in R^{1A1} to R^{1E1} are substituted with (s) a hydroxy group or a carboxy group each having a protective group,
more preferably C₁₋₁₈ alkyl groups, C₃₋₁₈ alicyclic groups, or C₇₋₁₈ aralkyl groups, each optionally having a substituent; any divalent carbon atom at a non-terminal position of R^{1A1} to R^{1E1} may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and one or more hydrogen atoms in R^{1A1} to R^{1E1} are substituted with (s) a hydroxy group or a carboxy group each having a protective group.

The protective group for the hydroxy group or the carboxy group may be, but not limited to, an ether protective group, a silyl ether protective group, an acetal protective group, an acyl protective group, an oxycarbonyl (alkyl) protective group, an aminocarbonyl protective group, or the like.

The protective group for the hydroxy group or the carboxy group is preferably an ether protective group, such as a t-butyl group or a benzyl group; a silyl ether protective group, such as a t-butyldimethylsilyl group, a triisopropylsilyl group, a trimethylsilyl group, a triethylsilyl group, or a t-butyldiphenylsilyl group; an acetal protective group, such as a methoxymethyl group, an ethoxyethyl group, a 2-tetrahydropyranyl group, or a methoxyethoxymethyl group; an acyl protective group, such as an acetyl group, a pivaloyl group, or a benzoyl group; an alkoxycarbonyl protective group, such as a t-butoxycarbonyl group; an aminocarbonyl protective group, such as a dimethylaminocarbonyl group, a diethylaminocarbonyl group, a diisopropylaminocarbonyl group, or an N-phenyl-N-methyl-aminocarbonyl group; or an acid-dissociable group in which carbon bonded to a hydroxy group or a carboxy group is tertiary carbon,
more preferably an acetal protective group, such as a methoxymethyl group, an ethoxyethyl group, a 2-tetrahydropyranyl group, or a methoxyethoxymethyl group; an oxycarbonyl (alkyl) protective group, such as a t-butoxycarbonyl group; an aminocarbonyl protective group, such as a dimethylaminocarbonyl group, a diethylaminocarbonyl group, a diisopropylaminocarbonyl group, or an N-phenyl-N-methyl-aminocarbonyl group; or an acid-dissociable group in which carbon bonded to a hydroxy group or a carboxy group is tertiary carbon and that has a cyclopentane skeleton, a cyclohexane skeleton, a cycloheptane skeleton, a norbornane skeleton, or an adamantane skeleton.

The protective group is, for example, but not limited to, a methoxymethyl group, an ethoxyethyl group, a methoxyethoxymethyl group, a t-butoxycarbonyl group, a 1-methylcyclopentyl group, a 1-ethylcyclopentyl group, a 1-phenylcyclopentyl group, a 1-tolylcyclopentyl group, a 1-methylcyclohexyl group, a 1-ethylcyclohexyl group, a 1-phenylcyclohexyl group, a 1-tolylcyclohexyl group, a 1-(naphthalen-2-yl)cyclohexyl group, a 1-(1-methoxy-2-methylpropan-2-yl)cyclopentyl group, a 1-(1-ethoxy-2-methylpropan-2-yl)cyclopentyl group, a 2-methyladamantan-2-yl group, a 2-ethyladamantan-2-yl group, a 2-phenyladamantan-2-yl group, a diethylaminocarbonyl group, a diisopropylaminocarbonyl group, or the like.

### [5. Cation Moiety of Heteropolyacid Salt Having Modified Lacunary Site]

The heteropolyacid salt having the modified lacunary site or a mixture thereof according to the present embodiment is represented by the general formula (I). Here, A^{m+} and Bⁿ⁺ correspond to the cation moieties of the heteropolyacid salt having the modified lacunary site.

(A^{m+})ₐ(Bⁿ⁺)_{b}(C(^{am+bn)-}) (I)

[wherein
A^{m+} each independently denotes H⁺ or a metal ion,
Bⁿ⁺ each independently denotes an ammonium cation, an ammonium dication, a phosphonium cation, or a phosphonium dication,
C^{(am+bn)-} denotes a heteropolyacid anion having a lacunary site, wherein the lacunary site is modified, and
m denotes an integer in the range of 1 to 5, n denotes an integer of 1 or 2, and a and b denote real numbers]
First, "Bⁿ⁺" will be described below, and then "A^{m+}" will be described as another counter cation.

### [5-1. Ammonium Cation (Bⁿ⁺)]

The ammonium cation is not particularly limited and may be a known and commonly used ammonium cation. The ammonium cation is, for example, but not limited to, an ammonium ion (NH₄⁺), a primary ammonium cation (NH₃(R^{2A})⁺), a secondary ammonium cation (NH₂(R^{2A})(R^{2B})⁺), a tertiary ammonium cation (NH(R^{2A})(R^{2B})(R^{2C})⁺), or a quaternary ammonium cation (N(R^{2A})(R^{2B})(R^{2C})(R^{2D})⁺). R^{2A} to R^{2D} denote the same as defined below.

The ammonium cation is preferably a quaternary ammonium cation, particularly preferably an organic quaternary ammonium cation represented by the following general formula (VIII).

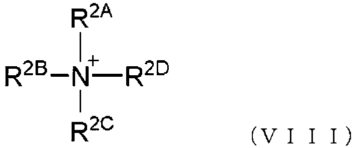

In the general formula (VIII), R^{2A} to R^{2D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{2A} to R^{2D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{2A} to R^{2D} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (1) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (s), and a divalent carbon atom at any position of these substituents except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
any two of R^{2A} to R^{2D} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with the nitrogen atom in the formula (VIII).

### <Organic Quaternary Ammonium Cation Having One or More Substituents>

In the organic quaternary ammonium cation represented by the general formula (VIII), from the perspective of providing a functional building block,
R^{2A} to R^{2D} each independently denote
a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{2A} to R^{2D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
at least one hydrogen atom in R^{2A} to R^{2D} is preferably substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (1) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group,
R^{2A} to R^{2D} each independently denote a C₁₋₁₈ alkyl group, a C₃₋₁₈ alicyclic group, a C₆₋₁₈ aryl group, or a C₇₋₁₈ aralkyl group, each optionally having a substituent,
a divalent carbon atom at any position of R^{2A} to R^{2D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
at least one hydrogen atom in R^{2A} to R^{2D} is more preferably substituted with (a) a halogen atom, (b) a C₁₋₃ haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (1) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group.

R^{2A} to R^{2D} each having one or more substituents is, for example, but not limited to, a 2-trifluoromethylbenzyl group, a 2-hydroxyethyl group, a 3-hydroxypropyl group, a vinyl group, an allyl group, a propenyl group, a butenyl group, a pentenyl group, a hexenyl group, a heptenyl group, an acryloylethyl group, an acryloylpropyl group, a methacryloylethyl group, a methacryloylpropyl group, an acryloyloxyethyl group, an acryloyloxypropyl group, a methacryloyloxyethyl group, a methacryloyloxypropyl group, an acryloylaminoethyl group, an acryloylaminopropyl group, a methacryloylaminoethyl group, a methacryloylaminopropyl group, a styryl group, a 1-glycidyloxypropan-3-yl group, a vinylbenzyl group, a 4-methoxymethoxyphenyl group, or the like.

### <Organic Quaternary Ammonium Cation Having One or More Ethylenically Unsaturated Double Bonds>

Here, from the perspective of improving the actinic radiation reactivity, in the organic quaternary ammonium cation represented by the general formula (VIII), R^{2A} to R^{2D} preferably contain at least one ethylenically unsaturated double bond.

In this case, R^{2A} to R^{2D} in the general formula (VIII) each independently preferably denote
an optionally substituted C₁₋₁₈ hydrocarbyl group; a divalent carbon atom at any position of R^{2A} to R^{2D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{2A} to R^{2D} is substituted with (j) a vinyl group, (k) an allyl group, (1) a (meth)acryloyl group, (m) a (meth)acryloyloxy group, (n) a (meth)acrylamide group, or (o) a styryl group,
more preferably a C₁₋₁₈ alkyl group, a C₃₋₁₈ alicyclic group, a C₆₋₁₈ aryl group, or a C₇₋₁₈ aralkyl group, each optionally having a substituent; a divalent carbon atom at any position of R^{2A} to R^{2D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{2A} to R^{2D} is substituted with (j) a vinyl group, (k) an allyl group, (1) a (meth)acryloyl group, (m) a (meth)acryloyloxy group, (n) a (meth)acrylamide group, or (o) a styryl group.

R^{2A} to R^{2D} each having at least one ethylenically unsaturated double bond is, for example, but not limited to, a vinyl group, an allyl group, a propenyl group, a butenyl group, a pentenyl group, a hexenyl group, a heptenyl group, an acryloylethyl group, an acryloylpropyl group, a methacryloylethyl group, a methacryloylpropyl group, an acryloyloxyethyl group, an acryloyloxypropyl group, a methacryloyloxyethyl group, a methacryloyloxypropyl group, an acryloylaminoethyl group, an acryloylaminopropyl group, a methacryloylaminoethyl group, a methacryloylaminopropyl group, a styryl group, or the like.

### <Organic Quaternary Ammonium Cation Having One or More Hydroxy Groups or Carboxy Groups Having Protective Group>

Furthermore, from the perspective of providing a building block whose functionality, such as solubility, is changed by deprotection due to the action of an acid or the like, at least one of R^{2A} to R^{2D} preferably contains one or more hydroxy groups or carboxy groups each having a protective group.

In this case, R^{2A} to R^{2D} in the general formula (VIII) each independently preferably denote
an optionally substituted C₁₋₁₈ hydrocarbyl group; a divalent carbon atom at any position of R^{2A} to R^{2D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{2A} to R^{2D} is substituted with (s) a hydroxy group or a carboxy group each having a protective group,
more preferably a C₁₋₁₈ alkyl group, a C₃₋₁₈ alicyclic group, a C₆₋₁₈ aryl group, or a C₇₋₁₈ aralkyl group, each optionally having a substituent; a divalent carbon atom at any position of R^{2A} to R^{2D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{2A} to R^{2D} is substituted with (s) a hydroxy group or a carboxy group each having a protective group.

The protective group for the hydroxy group or the carboxy group may be, but not limited to, an ether protective group, a silyl ether protective group, an acetal protective group, an acyl protective group, an oxycarbonyl (alkyl) protective group, an aminocarbonyl protective group, or the like.

The protective group for the hydroxy group or the carboxy group is preferably an ether protective group, such as a t-butyl group or a benzyl group; a silyl ether protective group, such as a t-butyldimethylsilyl group, a triisopropylsilyl group, a trimethylsilyl group, a triethylsilyl group, or a t-butyldiphenylsilyl group; an acetal protective group, such as a methoxymethyl group, an ethoxyethyl group, a 2-tetrahydropyranyl group, or a methoxyethoxymethyl group; an acyl protective group, such as an acetyl group, a pivaloyl group, or a benzoyl group; an oxycarbonyl (alkyl) protective group, such as a t-butoxycarbonyl group; an aminocarbonyl protective group, such as a dimethylaminocarbonyl group, a diethylaminocarbonyl group, a diisopropylaminocarbonyl group, or an N-phenyl-N-methyl-aminocarbonyl group; or an acid-dissociable group in which carbon bonded to a hydroxy group or a carboxy group is tertiary carbon,
more preferably an acetal protective group, such as a methoxymethyl group, an ethoxyethyl group, a 2-tetrahydropyranyl group, or a methoxyethoxymethyl group; an oxycarbonyl (alkyl) protective group, such as a t-butoxycarbonyl group; an aminocarbonyl protective group, such as a dimethylaminocarbonyl group, a diethylaminocarbonyl group, a diisopropylaminocarbonyl group, or an N-phenyl-N-methyl-aminocarbonyl group; or an acid-dissociable group in which carbon bonded to a hydroxy group or a carboxy group is tertiary carbon and that has a cyclopentane skeleton, a cyclohexane skeleton, a cycloheptane skeleton, a norbornane skeleton, or an adamantane skeleton.

The protective group is, for example, but not limited to, a methoxymethyl group, an ethoxyethyl group, a methoxyethoxymethyl group, a t-butoxycarbonyl group, a 1-methylcyclopentyl group, a 1-ethylcyclopentyl group, a 1-phenylcyclopentyl group, a 1-tolylcyclopentyl group, a 1-methylcyclohexyl group, a 1-ethylcyclohexyl group, a 1-phenylcyclohexyl group, a 1-tolylcyclohexyl group, a 1-(naphthalen-2-yl)cyclohexyl group, a 1-(1-methoxy-2-methylpropan-2-yl)cyclopentyl group, a 1-(1-ethoxy-2-methylpropan-2-yl)cyclopentyl group, a 2-methyladamantan-2-yl group, a 2-ethyladamantan-2-yl group, a 2-phenyladamantan-2-yl group, a diethylaminocarbonyl group, a diisopropylaminocarbonyl group, or the like.

### <Examples of Organic Quaternary Ammonium Cation>

Specific examples of the organic quaternary ammonium cation include a diallyldimethylammonium cation, a (3-acrylamidopropyl)trimethylammonium cation, a trimethyl-2-methacroyloxyethylammonium cation, an N-(2-acryloyloxyethyl)-N-benzyl-N,N-dimethylammonium cation, a trimethylvinylammonium cation, an N-4-vinylbenzyltriallylammonium cation, a 3-hydroxypropyltriallylammonium cation, a 2-trifluoromethylbenzyltriallylammonium cation, a di-2-(N-methylacrylamido)ethyldimethylammonium cation, an allyltrimethylammonium cation, a butyl(2-methacryloyloxyethyl)dimethylammonium cation, an ethoxycarbonylmethyltriethylammonium cation, a 2-hydroxyethyltrimethylammonium cation, a 2-acetylethyltrimethylammonium cation, a (4-((diisopropylcarbamoyl)oxy)phenyl)trimethylammonium cation, a (4-(methacryloyloxy)phenyl)trimethylammonium cation, a trimethyl(4-(nonanoyloxy)phenyl)ammonium cation, and the like.

### [5-2. Ammonium Dication (Bⁿ⁺)]

The ammonium dication is not particularly limited and may be a known and commonly used ammonium dication. The ammonium dication is, for example, an organic quaternary ammonium dication represented by the following general formula (IX).

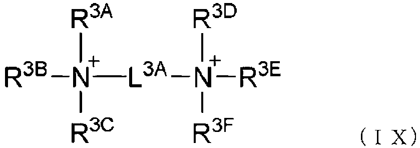

In the general formula (IX), R^{3A} to R^{3F} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{3A} to R^{3F} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
L^{3A} denotes an optionally substituted C₁₋₁₈ hydrocarbylene group,
a divalent carbon atom at any position of L^{3A} may be substituted with -O-, -C(=O)-, -C(=O)O-, -OCO-, -CONH-, -NHCO-, -S-, or -SO₂- (provided that adjacent divalent carbon atoms are not substituted at the same time), and
a hydrogen atom in R^{3A} to R^{3F} and L^{3A} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (1) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (s), and a divalent carbon atom at any position of these substituents except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
any two of R^{3A} to R^{3F} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with the nitrogen atom in the formula (IX).

The organic quaternary ammonium dication represented by the general formula (IX) is, for example, but not limited to, a 1,4-diallyl-1,4-diazabicyclo[2.2.2]octane-1,4-diium, 1,4-di(2-(meth)acryloyloxyethyl)-1,4-diazabicyclo[2.2.2]octane-1,4-diium, 1,4-bis(2-(meth)acrylamidoethyl)-1,4-diazabicyclo[2.2.2]octane-1,4-diium, or the like.

### [5-3. Phosphonium Cation (Bⁿ⁺)]

The phosphonium cation is not particularly limited and may be a known and commonly used phosphonium cation. The phosphonium cation is, for example, an organic quaternary phosphonium cation represented by the following general formula (X).

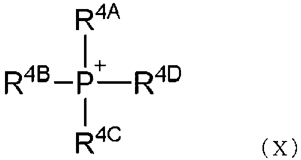

In the general formula (X), R^{4A} to R^{4D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{4A} to R^{4D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{4A} to R^{4D} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (1) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (s), and a divalent carbon atom at any position of these substituents except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
any two of R^{4A} to R^{4D} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with the phosphorus atom in the formula (X).

### <Organic Phosphonium Cation Having One or More Substituents>

In the organic quaternary phosphonium cation represented by the general formula (X), from the perspective of providing a functional building block,
R^{4A} to R^{4D} each independently preferably denote
a C₁₋₁₈ hydrocarbyl group, a 3- to 18-membered non-aromatic heterocyclic group, or a 5- to 18-membered aromatic heterocyclic group, each optionally having a substituent; a divalent carbon atom at any position of R^{4A} to R^{4D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{4A} to R^{4D} is substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (1) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group,
more preferably a C₁₋₁₈ alkyl group, a C₃₋₁₈ alicyclic group, a C₆₋₁₈ aryl group, or a C₇₋₁₈ aralkyl group, each optionally having a substituent; a divalent carbon atom at any position of R^{4A} to R^{4D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{4A} to R^{4D} is substituted with (a) a halogen atom, (b) a C₁₋₃ haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (1) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group.

R^{4A} to R^{4D} each having one or more substituents is, for example, but not limited to, a 2-trifluoromethylbenzyl group, a 2-hydroxyethyl group, a 3-hydroxypropyl group, a vinyl group, an allyl group, a propenyl group, a butenyl group, a pentenyl group, a hexenyl group, a heptenyl group, an acryloylethyl group, an acryloylpropyl group, a methacryloylethyl group, a methacryloylpropyl group, an acryloyloxyethyl group, an acryloyloxypropyl group, a methacryloyloxyethyl group, a methacryloyloxypropyl group, an acryloylaminoethyl group, an acryloylaminopropyl group, a methacryloylaminoethyl group, a methacryloylaminopropyl group, a styryl group, a 1-glycidyloxypropan-3-yl group, a vinylbenzyl group, a 4-methoxymethoxyphenyl group, or the like.

### <Organic Phosphonium Cation Having One or More Ethylenically Unsaturated Double Bonds>

Here, from the perspective of improving the actinic radiation reactivity, at least one of R^{4A} to R^{4D} preferably contains at least one ethylenically unsaturated double bond.

In this case, R^{4A} to R^{4D} in the general formula (X) each independently preferably denote
an optionally substituted C₁₋₁₈ hydrocarbyl group; a divalent carbon atom at any position of R^{4A} to R^{4D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{4A} to R^{4D} is substituted with (j) a vinyl group, (k) an allyl group, (1) a (meth)acryloyl group, (m) a (meth)acryloyloxy group, (n) a (meth)acrylamide group, or (o) a styryl group,
more preferably a C₁₋₁₈ alkyl group, a C₃₋₁₈ alicyclic group, a C₆₋₁₈ aryl group, or a C₇₋₁₈ aralkyl group, each optionally having a substituent; a divalent carbon atom at any position of R^{4A} to R^{4D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{4A} to R^{4D} is substituted with (j) a vinyl group, (k) an allyl group, (1) a (meth)acryloyl group, (m) a (meth)acryloyloxy group, (n) a (meth)acrylamide group, or (o) a styryl group.

R^{4A} to R^{4D} each having at least one ethylenically unsaturated double bond is, for example, but not limited to, a vinyl group, an allyl group, a propenyl group, a butenyl group, a pentenyl group, a hexenyl group, a heptenyl group, an acryloylethyl group, an acryloylpropyl group, a methacryloylethyl group, a methacryloylpropyl group, an acryloyloxyethyl group, an acryloyloxypropyl group, a methacryloyloxyethyl group, a methacryloyloxypropyl group, an acryloylaminoethyl group, an acryloylaminopropyl group, a methacryloylaminoethyl group, a methacryloylaminopropyl group, a styryl group, or the like.

### <Organic Phosphonium Cation Having One or More Hydroxy Groups or Carboxy Groups Having Protective Group>

Furthermore, from the perspective of providing a building block whose functionality, such as solubility, is changed by deprotection due to the action of an acid or the like, at least one of R^{4A} to R^{4D} preferably contains one or more hydroxy groups or carboxy groups each having a protective group.

In this case, R^{4A} to R^{4D} in the general formula (X) each independently preferably denote
an optionally substituted C₁₋₁₈ hydrocarbyl group; a divalent carbon atom at any position of R^{4A} to R^{4D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{4A} to R^{4D} is substituted with (s) a hydroxy group or a carboxy group each having a protective group,
more preferably a C₁₋₁₈ alkyl group, a C₃₋₁₈ alicyclic group, a C₆₋₁₈ aryl group, or a C₇₋₁₈ aralkyl group, each optionally having a substituent; a divalent carbon atom at any position of R^{4A} to R^{4D} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and at least one hydrogen atom of R^{4A} to R^{4D} is substituted with (s) a hydroxy group or a carboxy group each having a protective group.

The protective group for the hydroxy group or the carboxy group may be, but not limited to, an ether protective group, a silyl ether protective group, an acetal protective group, an acyl protective group, an oxycarbonyl (alkyl) protective group, an aminocarbonyl protective group, or the like.

The protective group for the hydroxy group or the carboxy group is preferably an ether protective group, such as a t-butyl group or a benzyl group; a silyl ether protective group, such as a t-butyldimethylsilyl group, a triisopropylsilyl group, a trimethylsilyl group, a triethylsilyl group, or a t-butyldiphenylsilyl group; an acetal protective group, such as a methoxymethyl group, an ethoxyethyl group, a 2-tetrahydropyranyl group, or a methoxyethoxymethyl group; an acyl protective group, such as an acetyl group, a pivaloyl group, or a benzoyl group; an oxycarbonyl (alkyl) protective group, such as a t-butoxycarbonyl group; an aminocarbonyl protective group, such as a dimethylaminocarbonyl group, a diethylaminocarbonyl group, a diisopropylaminocarbonyl group, or an N-phenyl-N-methyl-aminocarbonyl group; or an acid-dissociable group in which carbon bonded to a hydroxy group or a carboxy group is tertiary carbon,
more preferably an acetal protective group, such as a methoxymethyl group, an ethoxyethyl group, a 2-tetrahydropyranyl group, or a methoxyethoxymethyl group; an oxycarbonyl (alkyl) protective group, such as a t-butoxycarbonyl group; an aminocarbonyl protective group, such as a dimethylaminocarbonyl group, a diethylaminocarbonyl group, a diisopropylaminocarbonyl group, or an N-phenyl-N-methyl-aminocarbonyl group; or an acid-dissociable group in which carbon bonded to a hydroxy group or a carboxy group is tertiary carbon and that has a cyclopentane skeleton, a cyclohexane skeleton, a cycloheptane skeleton, a norbornane skeleton, or an adamantane skeleton.

The protective group is, for example, but not limited to, a methoxymethyl group, an ethoxyethyl group, a methoxyethoxymethyl group, a t-butoxycarbonyl group, a 1-methylcyclopentyl group, a 1-ethylcyclopentyl group, a 1-phenylcyclopentyl group, a 1-tolylcyclopentyl group, a 1-methylcyclohexyl group, a 1-ethylcyclohexyl group, a 1-phenylcyclohexyl group, a 1-tolylcyclohexyl group, a 1-(naphthalen-2-yl)cyclohexyl group, a 1-(1-methoxy-2-methylpropan-2-yl)cyclopentyl group, a 1-(1-ethoxy-2-methylpropan-2-yl)cyclopentyl group, a 2-methyladamantan-2-yl group, a 2-ethyladamantan-2-yl group, a 2-phenyladamantan-2-yl group, a diethylaminocarbonyl group, a diisopropylaminocarbonyl group, or the like.

### <Examples of Organic Quaternary Phosphonium Cation>

The organic quaternary phosphonium cation represented by the general formula (X) is, for example, but not limited to, a tributylcyanomethylphosphonium cation, a methyltriphenylphosphonium cation, an ethyltriphenylphosphonium cation, a cyanomethyltriphenylphosphonium cation, a formylmethyltriphenylphosphonium cation, a methoxymethyltriphenylphosphonium cation, a chloromethyltriphenylphosphonium cation, an acetonyltriphenylphosphonium cation, a tributyl-1,3-dioxan-2-ylmethylphosphonium cation, a triphenylpropargylphosphonium cation, an allyltriphenylphosphonium cation, a cyclopropyltriphenylphosphonium cation, a benzyltriphenylphosphonium cation, a tetrakis(hydroxymethyl)phosphonium cation, a 2-carboxyethyltriphenylphosphonium cation, a methoxycarbonylmethyltriphenylphosphonium cation, a t-butoxycarbonylmethyltriphenylphosphonium cation, a (4-((diisopropylcarbamoyl)oxy)phenyl)triphenylphosphonium cation, a (4-(methacryloyloxy)phenyl)triphenylphosphonium cation, a triphenyl(4-(nonanoyloxy)phenyl)phosphonium cation, or the like.

### [5-4. Phosphonium Dication (Bⁿ⁺)]

The phosphonium dication is not particularly limited and may be a known and commonly used phosphonium dication. The phosphonium dication is, for example, an organic quaternary phosphonium dication represented by the following general formula (XI).

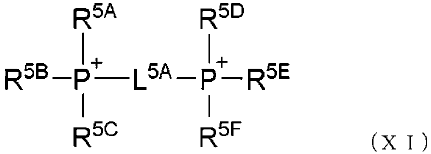

In the general formula (XI), R^{5A} to R^{5F} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{5A} to R^{5F} except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
L^{5A} denotes an optionally substituted C₁₋₁₈ hydrocarbylene group,
a divalent carbon atom at any position of L^{5A} may be substituted with -O-, -C(=O)-, -C(=O)O-, -OCO-, -CONH-, -NHCO-, -S-, or SO₂- (provided that adjacent divalent carbon atoms are not substituted at the same time), and
a hydrogen atom in R^{5A} to R^{5F} and L^{5A} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (1) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (s), and a divalent carbon atom at any position of these substituents except the terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
any two of R^{5A} to R^{5F} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with the phosphorus atom in the formula (XI).

The organic quaternary phosphonium dication represented by the general formula (XI) is, for example, but not limited to, a trans-2-butene-1,4-bis(triphenylphosphonium) dication, an ethylenebis(triphenylphosphonium) dication, a pentamethylenebis(triphenylphosphonium) dication, or the like.

### [5-5. Another Counter Cation (A^{m+})]

Another counter cation (A^{m+}) according to the present embodiment is H⁺ or a metal ion.

A metal ion that can be used as "A^{m+}" is, for example, but not limited to, Li⁺, Na⁺, K⁺, Rb⁺, Cs⁺, Be²⁺, Mg²⁺, Ca²⁺, Sr²⁺, Ba²⁺, Al³⁺, Co³⁺, Bi³⁺, Zr⁴⁺, Hf⁴⁺, Bi⁵⁺, or the like. These metal ions may be used alone or in combination of two or more types thereof.

"A^{m+}" is preferably Li⁺, Na⁺, K⁺, Rb⁺, Cs⁺, Mg²⁺, Ca²⁺, Sr²⁺, Ba²⁺, Al³⁺, or Co³⁺, more preferably Na⁺, K⁺, Rb⁺, Cs⁺, Ca²⁺, Al³⁺, or Co³⁺.

### [5-6. Ratio of A^{m+} to Bⁿ⁺]

In the general formula (I), m denotes an integer in the range of 1 to 5, n denotes an integer of 1 or 2, a and b denote real numbers.

(A^{m+})ₐ(Bⁿ⁺)_{b}(C^{(am+bn)-}) (I)

Furthermore, a mixture of heteropolyacid salts having a modified lacunary site may contain a plurality of heteropolyacid salts having different ratios of A^{m+} to Bⁿ⁺. In this case, the values of a and b can be determined by, for example, NMR analysis, XRF analysis, XPS analysis, or the like.

The ratio of a to b depends on the types of the heteropolyacid anion having the modified lacunary site, A^{m+}, and Bⁿ⁺, and is preferably a:b = 0 to 3:0 to 12, more preferably 0 to 2:0 to 8, still more preferably 0 to 1:1 to 4.

In particular, when the value of a + b is a real number in the range of 3 to 7, and m is 1, it is preferable that a is a real number in the range of 0 to 3, and b is a real number in the range of 0 to 7, and it is more preferable that a is a real number in the range of 0 to 2, and b is a real number in the range of 2 to 7.

### EXAMPLES

The present invention will be more specifically described below with reference to examples, but the present invention is not limited to these examples.

### [1. Synthesis of Heteropolyacid Salt Having Modified Lacunary Site (Hereinafter also Referred to as "Polyacid Salt")]

### <Production Example 1: Potassium Undecatungstosilicate (α-K₈[SiW₁₁O₃₉])>

380 g of 1 M acetic acid was added to 63 g of silicotungstic acid hydrate (manufactured by Nippon Inorganic Colour & Chemical Co., Ltd.: H₄[SiW₁₂O₄₀]·xH₂O), and the reaction liquid was heated to 45°C. The pH was adjusted to 6.0 by addition of potassium hydrogen carbonate (52 g). The reaction liquid was then cooled to room temperature and was suction-filtered to prepare 56 g of a crude product. 216 g of pure water was added to the crude product, followed by heating to 73°C to redissolve the crude product. The solution was cooled at 4°C for 16 hours to recrystallize, and the resulting crystals were suction-filtered and dried under vacuum to obtain 34 g of the target compound. ²⁹Si-NMR (119.22 MHz, D₂O): δ (ppm) = -84.6 (s, 1Si).

¹⁸³W-NMR (20.84 MHz, D₂O): δ (ppm) = -101.85 (s, 2W), -116.26 (s, 2W), -119.29 (s, 1W), -125.62 (s, 2W), -140.85 (s, 2W), -174.53 (s, 2W).

### <Synthesis Example 1: tetrakis((2-trifluoromethylbenzyl)triallylammonium)(1,3-dimethyldisiloxane-1,1,3,3-tetrayl)undecatungstosilicate>

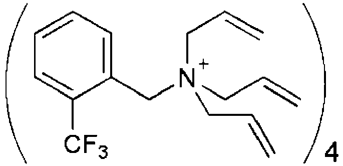

[SiW₁₁O₃₉(CH₃-Si)₂O]⁴⁻

3.8 g (1.3 mmol) of the mono-lacunary Keggin-type potassium undecatungstosilicate (α-K₈[SiW₁₁O₃₉]) synthesized above was added to 95 g of pure water. After stirring, 0.9 mL (6.3 mmol) of methyltrimethoxysilane (CH₃Si(OCH₃)₃) was added thereto, and 10.5 g of 1 M hydrochloric acid was added thereto and was stirred for 20 hours. After the unreacted powder was suction-filtered, an aqueous solution of 2.9 g (7.6 mmol) of ((2-trifluoromethylbenzyl)triallylammonium)bromide dissolved in 27 g of pure water was added thereto to precipitate a powder. The precipitated powder was poured into a suction funnel together with a small amount of pure water, was suction-filtered, and was washed with 151 g of isopropanol. The powder was sufficiently dried under vacuum to produce 4.5 g of the target compound as a white powder.

¹H-NMR (400 MHz, DMSO-d6): δ (ppm) = 0.04 (s, 6H), 4.04 (d, 24H), 4.73 (s, 8H), 5.65 (d, 12H), 5.73 (d, 12H), 6.02-6.12 (m, 12H), 7.80-7.88 (m, 12H), 7.94 (d, 4H).

²⁹Si-NMR (119.22 MHz, DMSO-d6): δ (ppm) = -51.15 (s, 2Si), -85.08 (s, 1Si).

ESI-MS: POSITIVE m/z 296.2 ([C₁₇H₂₁F₃N]⁺)
NEGATIVE m/z 694.1 (median) ([C₂H₆O₄₀Si₃W₁₁]⁴⁻)

### <Synthesis Example 2: tetrakis((2-trifluoromethylbenzyl)triallylammonium)(1,3-di(n-hexyl)disiloxane-1,1,3,3-tetrayl)undecatungstosilicate>

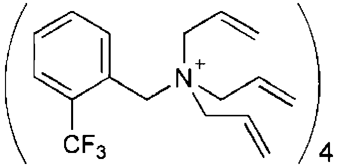

[SiW₁₁O₃₉((n-C₆H₁₃)-Si)₂O] ⁴⁻

The target compound was produced in the same manner as in Synthesis Example 1 except that n-hexyltrimethoxysilane was used as a raw material compound instead of methyltrimethoxysilane.

¹H-NMR (400 MHz, DMSO-d6): δ (ppm) = 0.54 (t, 4H), 0.83 (t, 6H), 1.21-1.51 (m, 16H), 4.04 (d, 24H), 4.73 (s, 8H), 5.65 (d, 12H), 5.73 (d, 12H), 6.02-6.12 (m, 12H), 7.80-7.88 (m, 12H), 7.94 (d, 4H).

²⁹Si-NMR (119.22 MHz, DMSO-d6): δ (ppm) = -52.82 (s, 2Si), -85.18 (s, 1Si).

ESI-MS: POSITIVE m/z 296.2 ([C₁₇H₂₁F₃N]⁺)
NEGATIVE m/z 729.1 (median) ([C₁₂H₂₆O₄₀Si₃W₁₁]⁴⁻)

### <Synthesis Example 3: tetrakis((2-trifluoromethylbenzyl)triallylammonium)(1,3-diphenyldisiloxane-1,1,3,3-tetrayl)undecatungstosilicate>

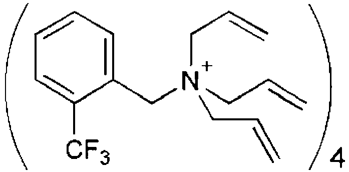

[SiW₁₁O₃₉((C₆H₅)-Si)₂O] ⁴⁻

The target compound was produced in the same manner as in Synthesis Example 1 except that phenyltrimethoxysilane was used as a raw material compound instead of methyltrimethoxysilane.

¹H-NMR (400 MHz, DMSO-d6): δ (ppm) = 4.04 (d, 24H), 4.73 (s, 8H), 5.65 (d, 12H), 5.73 (d, 12H), 6.02-6.12 (m, 12H), 7.38-7.45 (m, 6H), 7.74-7.88 (m, 16H), 7.94 (d, 4H). ²⁹Si-NMR (119.22 MHz, DMSO-d6): δ (ppm) = -65.42 (s, 2Si), -85.29 (s, 1Si).

ESI-MS: POSITIVE m/z 296.2 ([C₁₇H₂₁F₃N]⁺)
NEGATIVE m/z 725.1 (median) ([C₁₂H₁₀O₄₀Si₃W₁₁]⁴⁻)

### <Synthesis Example 4: bis((2-trifluoromethylbenzyl)triallylammonium)(1,3-di(3-ammoniumpropyl)disiloxane-1,1,3,3-tetrayl)undecatungstosilicate>

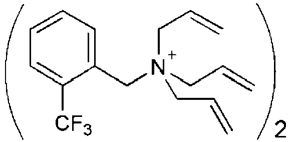

[SiW₁₁O₃₉(H₃N⁺-(CH₂)₃-Si)₂O] ⁴⁻

The target compound was produced in the same manner as in Synthesis Example 1 except that 3-aminopropyltrimethoxysilane was used as a raw material compound instead of methyltrimethoxysilane.

¹H-NMR (400 MHz, DMSO-d6): δ (ppm) = 0.63-0.67 (m, 4H), 1.79 (quint, 4H), 2.89 (t, 4H), 4.04 (d, 24H), 4.73 (s, 8H), 5.65 (d, 12H), 5.73 (d, 12H), 6.02-6.12 (m, 12H), 7.68 (br, 6H), 7.80-7.88 (m, 12H), 7.94 (d, 4H).

²⁹Si-NMR (119.22 MHz, DMSO-d6): δ (ppm) = -54.43 (s, 2Si), -85.03 (s, 1Si).

ESI-MS: POSITIVE m/z 296.2 ([C₁₇H₂₁F₃N]⁺)
NEGATIVE m/z 1432.2 (median) ([C₆H₁₈N₂O₄₀Si₃W₁₁]²⁻)

### <Synthesis Example 5: tetrakis((2-trifluoromethylbenzyl)triallylammonium)(1,3-di(3,3,3-trifluoropropyl)disiloxane-1,1,3,3-tetrayl)undecatungstosilicate>

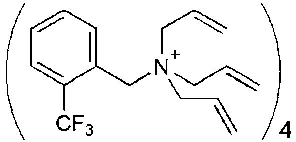

[SiW₁₁O₃₉(F₃C-(CH₂)₂-Si)₂O] ⁴⁻

The target compound was produced in the same manner as in Synthesis Example 1 except that 3,3,3-trifluoropropyltrimethoxysilane was used as a raw material compound instead of methyltrimethoxysilane.

¹H-NMR (400 MHz, DMSO-d6): δ (ppm) = 0.75 (t, 4H), 2.20-2.41 (m, 4H), 4.04 (d, 24H), 4.73 (s, 8H), 5.65 (d, 12H), 5.73 (d, 12H), 6.02-6.12 (m, 12H), 7.80-7.88 (m, 12H), 7.94 (d, 4H).

²⁹Si-NMR (119.22 MHz, DMSO-d6): δ (ppm) = -54.43 (s, 2Si), -85.03 (s, 1Si).

ESI-MS: POSITIVE m/z 296.2 ([C₁₇H₂₁F₃N]⁺)
NEGATIVE m/z 735.1 (median) ([C₆H₈F₆O₄₀Si₃W₁₁]⁴⁻)

### <Synthesis Example 6: tetrakis((2-trifluoromethylbenzyl)triallylammonium)(1,3-di(3-glycidyloxypropyl)disiloxane-1,1,3,3-tetrayl)undecatungstosilicate>

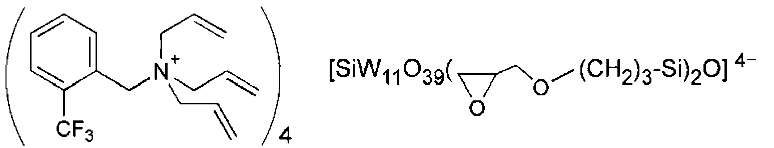

The target compound was produced in the same manner as in Synthesis Example 1 except that 3-glycidyloxypropyltrimethoxysilane was used as a raw material compound instead of methyltrimethoxysilane.

¹H-NMR (400 MHz, DMSO-d6): δ (ppm) = 0.57 (t, 4H), 1.71 (quint, 4H), 2.68 (t, 2H), 3.06-3.10 (m, 2H), 3.26-3.48 (m, 8H), 3.63 (dd, 2H), 4.04 (d, 24H), 4.73 (s, 8H), 5.65 (d, 12H), 5.73 (d, 12H), 6.02-6.12 (m, 12H), 7.80-7.88 (m, 12H), 7.94 (d, 4H).

²⁹Si-NMR (119.22 MHz, DMSO-d6): δ (ppm) = -52.68 (s, 2Si), -85.11 (s, 1Si).

ESI-MS: POSITIVE m/z 296.2 ([C₁₇H₂₁F₃N]⁺)
NEGATIVE m/z 744.1 (median) ([C₁₂H₂₂O₄₄Si₃W₁₁]⁴⁻)

### <Synthesis Example 7: tetrakis((2-trifluoromethylbenzyl)triallylammonium)(1,3-di(2-cyanoethyl)disiloxane-1,1,3,3-tetrayl)undecatungstosilicate>

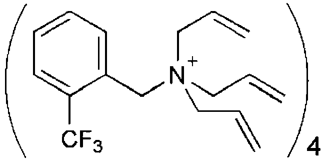

[SiW₁₁O₃₉(NC-(CH₂)₂-Si)₂O] ⁴⁻

The target compound was produced in the same manner as in Synthesis Example 1 except that 2-cyanoethyltriethoxysilane was used as a raw material compound instead of methyltrimethoxysilane.

¹H-NMR (400 MHz, DMSO-d6): δ (ppm) = 0.93 (t, 4H), 2.46-2.62 (m, 4H), 4.04 (d, 24H), 4.73 (s, 8H), 5.65 (d, 12H), 5.73 (d, 12H), 6.02-6.12 (m, 12H), 7.80-7.88 (m, 12H), 7.94 (d, 4H).

²⁹Si-NMR (119.22 MHz, DMSO-d6): δ (ppm) = -55.55 (s, 2Si), -85.02 (s, 1Si).

ESI-MS: POSITIVE m/z 296.2 ([C₁₇H₂₁F₃N]⁺)
NEGATIVE m/z 713.6 (median) ([C₆H₈N₂O₄₀Si₃W₁₁]⁴⁻)

### <Synthesis Example 8: tetrakis(tetraallylammonium)(1,3-dimethyldisiloxane-1,1,3,3-tetrayl)undecatungstosilicate>

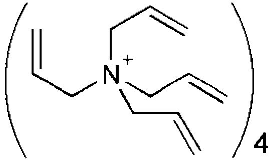

[SiW₁₁O₃₉(CH₃-Si)₂O] ⁴⁻

The target compound was produced in the same manner as in Synthesis Example 1 except that an aqueous solution of tetraallylammonium bromide was used as a raw material compound instead of the aqueous solution of (2-trifluoromethylbenzyl)triallylammonium bromide.

¹H-NMR (400 MHz, DMSO-d6): δ (ppm) = 0.04 (s, 6H), 3.90 (d, 32H), 5.66-5.72 (m, 32H), 6.05-6.14 (s, 16H).

²⁹Si-NMR (119.22 MHz, DMSO-d6): δ (ppm) = -51.15 (s, 2Si), -85.08 (s, 1Si).

ESI-MS: POSITIVE m/z 178.2 ([C₁₂H₂₀N]⁺)
NEGATIVE m/z 694.1 (median) ([C₂H₆O₄₀Si₃W₁₁]⁴⁻)

### <Synthesis Example 9: tetrakis(diallyldimethylammonium)(1,3-dimethyldisiloxane-1,1,3,3-tetrayl)undecatungstosilicate>

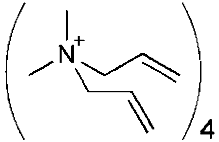

[SiW₁₁O₃₉(CH₃-Si)₂O] ⁴⁻

The target compound was produced in the same manner as in Synthesis Example 1 except that an aqueous solution of diallyldimethylammonium chloride was used as a raw material compound instead of the aqueous solution of (2-trifluoromethylbenzyl)triallylammonium bromide.

¹H-NMR (400 MHz, DMSO-d6): δ (ppm) = 0.04 (s, 6H), 3.01 (s, 24H), 4.02 (d, 16H), 5.62-5.68 (m, 16H), 6.04-6.14 (m, 8H).

²⁹Si-NMR (119.22 MHz, DMSO-d6): δ (ppm) = -51.15 (s, 2Si), -85.08 (s, 1Si).

ESI-MS: POSITIVE m/z 126.1 ([C₈H₁₆N]⁺)
NEGATIVE m/z 694.1 (median) ([C₂H₆O₄₀Si₃W₁₁]⁴⁻)

### <Synthesis Example 10: tetrakis(3-acryloylaminopropyltrimethylammonium)(1,3-dimethyldisiloxane-1,1,3,3-tetrayl)undecatungstosilicate>

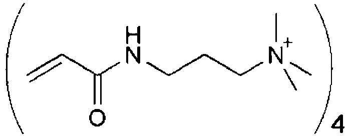

[SiW₁₁O₃₉(CH₃-Si)₂O]⁴⁻

The target compound was produced in the same manner as in Synthesis Example 1 except that an aqueous solution of 3-acryloylaminopropyltrimethylammonium chloride was used as a raw material compound instead of the aqueous solution of (2-trifluoromethylbenzyl)triallylammonium bromide.

¹H-NMR (400 MHz, DMSO-d6): δ (ppm) = 0.04 (s, 6H), 1.86-1.93 (m, 8H), 3.09 (s, 36H), 3.20 (q, 8H), 3.38-3.42 (m, 8H), 5.60 (dd, 4H), 6.10 (dd, 4H), 6.32 (dd, 4H), 8.68-8.71 (m, 4H).

²⁹Si-NMR (119.22 MHz, DMSO-d6): δ (ppm) = -51.15 (s, 2Si), -85.08 (s, 1Si).

ESI-MS: POSITIVE m/z 171.1 ([C₉H₁₉N₂O]⁺)
NEGATIVE m/z 694.1 (median) ([C₂H₆O₄₀Si₃W₁₁]⁴⁻)

### <Synthesis Example 11: tetrakis(methacryloylcholine)(1,3-dimethyldisiloxane-1,1,3,3-tetrayl)undecatungstosilicate>

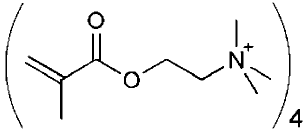

[SiW₁₁O₃₉(CH₃-Si)₂O] ⁴⁻

The target compound was produced in the same manner as in Synthesis Example 1 except that an aqueous solution of methacryloylcholine chloride was used as a raw material compound instead of the aqueous solution of (2-trifluoromethylbenzyl)triallylammonium bromide.

¹H-NMR (400 MHz, DMSO-d6): δ (ppm) = 0.04 (s, 6H), 1.90 (t, 12H), 3.20 (s, 36H), 3.79-3.81 (m, 8H), 4.54 (t, 8H), 5.75 (t, 4H), 6.09 (t, 4H).

²⁹Si-NMR (119.22 MHz, DMSO-d6): δ (ppm) = -51.15 (s, 2Si), -85.08 (s, 1Si).

ESI-MS: POSITIVE m/z 172.1 ([C₉H₁₈NO₂]⁺)
NEGATIVE m/z 694.1 (median) ([C₂H₆O₄₀Si₃W₁₁]⁴⁻)

### <Synthesis Example 12: tetrakis((2-acryloyloxyethyl)benzyldimethylammonium)(1,3-dimethyldisiloxane-1,1,3,3-tetrayl)undecatungstosilicate>

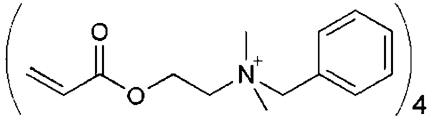

[SiW₁₁O₃₉(CH₃-Si)₂O]⁴⁻

The target compound was produced in the same manner as in Synthesis Example 1 except that an aqueous solution of (2-acryloyloxyethyl)benzyldimethylammonium chloride was used as a raw material compound instead of the aqueous solution of (2-trifluoromethylbenzyl)triallylammonium bromide.

¹H-NMR (400 MHz, DMSO-d6): δ (ppm) = 0.04 (s, 6H), 3.01 (s, 24H), 3.78-3.81 (m, 8H), 4.65 (br, 8H), 4.75 (s, 8H), 6.04 (dd, 4H), 6.24 (dd, 4H), 6.41 (dd, 4H), 7.52-7.5 (m, 12H), 7.63 (d, 8H).

²⁹Si-NMR (119.22 MHz, DMSO-d6): δ (ppm) = -51.15 (s, 2Si), -85.08 (s, 1Si).

ESI-MS: POSITIVE m/z 234.1 ([C₁₄H₂₀NO₂]⁺)
NEGATIVE m/z 694.1 (median) ([C₂H₆O₄₀Si₃W₁₁]⁴⁻)

### <Synthesis Example 13: tetrakis(vinyltrimethylammonium)(1,3-dimethyldisiloxane-1,1,3,3-tetrayl)undecatungstosilicate>

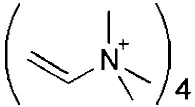

[SiW₁₁O₃₉(CH₃-Si)₂O] ⁴⁻

The target compound was produced in the same manner as in Synthesis Example 1 except that an aqueous solution of vinyltrimethylammonium bromide was used as a raw material compound instead of the aqueous solution of (2-trifluoromethylbenzyl)triallylammonium bromide.

¹H-NMR (400 MHz, DMSO-d6): δ (ppm) = 0.04 (s, 6H), 3.33 (s, 36H), 5.54-5.58 (m, 4H), 5.80 (d, 4H), 6.67-6.73 (m, 4H).

²⁹Si-NMR (119.22 MHz, DMSO-d6): δ (ppm) = -51.15 (s, 2Si), -85.08 (s, 1Si).

ESI-MS: POSITIVE m/z 86.1 ([C₅H₁₂N]⁺)
NEGATIVE m/z 694.1 (median) ([C₂H₆O₄₀Si₃W₁₁]⁴⁻)

### <Synthesis Example 14: tetrakis((4-vinylbenzyl)trimethylammonium)(1,3-dimethyldisiloxane-1,1,3,3-tetrayl)undecatungstosilicate>

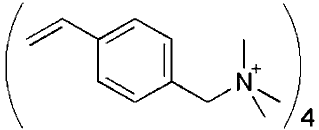

[SiW₁₁O₃₉(CH₃-Si)₂O]⁴⁻

The target compound was produced in the same manner as in Synthesis Example 1 except that an aqueous solution of (4-vinylbenzyl)trimethylammonium chloride was used as a raw material compound instead of the aqueous solution of (2-trifluoromethylbenzyl)triallylammonium bromide.

¹H-NMR (400 MHz, DMSO-d6): δ (ppm) = 0.04 (s, 6H), 3.13 (s, 36H), 4.74 (s, 8H), 5.38 (dd, 4H), 5.96 (dd, 4H), 6.81 (dd, 4H), 7.42-7.80 (m, 16H).

²⁹Si-NMR (119.22 MHz, DMSO-d6): δ (ppm) = -51.15 (s, 2Si), -85.08 (s, 1Si).

ESI-MS: POSITIVE m/z 176.1 ([C₁₂H₁₈N]⁺)
NEGATIVE m/z 694.1 (median) ([C₂H₆O₄₀Si₃W₁₁]⁴⁻)

### <Synthesis Example 15: tetrakis((2-trifluoromethylbenzyl)triallylammonium)(1,3-divinyldisiloxane-1,1,3,3-tetrayl)undecatungstosilicate>

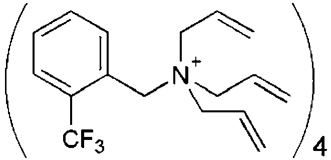

[SiW₁₁O₃₉(CH₂=CH-Si)₂O] ⁴⁻

0.64 mL (4.2 mmol) of vinyltrimethoxysilane (CH₂=CH-Si(OCH₃)₃) was added to a mixed solvent of 165 g of acetonitrile and 71 g of pure water, and 4.2 g (1.4 mmol) of potassium silicotungstate (α-K₈[SiW₁₁O₃₉]) synthesized in the production example was added thereto. The pH was adjusted to 1.8 using 1 M hydrochloric acid. After stirring for 2 hours, the unreacted powder was suction-filtered, and the filtrate was concentrated to approximately 72 g using a rotary evaporator. 17 g (7.0 mmol) of a 20% aqueous solution of (2-trifluoromethylbenzyl)triallylammonium bromide was added to the concentrate to precipitate a powder. The precipitated powder was suction-filtered and washed three times each with 70 mL of pure water, 70 mL of methanol, and 70 mL of methyl-t-butyl ether. The powder was then dispersed and washed with 60 g of methanol for 3 hours, was suction-filtered again, and was washed with 60 g of methanol three times. The powder was sufficiently dried under vacuum to produce 4.5 g of the target compound as a white powder. ¹H-NMR (400 MHz, DMSO-d6): δ (ppm) = 4.04 (d, 24H), 4.73 (s, 8H), 5.65 (d, 12H), 5.73 (d, 12H), 5.94 (dd, 2H), 6.02-6.13 (m, 16H), 7.80-7.88 (m, 12H), 7.94 (d, 4H).

²⁹Si-NMR (119.22 MHz, DMSO-d6): δ (ppm) = -66.56 (s, 2Si), -85.29 (s, 1Si).

¹⁸³W-NMR (20.84 MHz, DMSO-d6): δ (ppm) = -105.52 (s, 2W), -107.54 (s, 2W), -112.04 (s, 1W), -126.08 (s, 2W), -170.95 (s, 2W), -247.66 (s, 2W).

ESI-MS: POSITIVE m/z 296.2 ([C₁₇H₂₁F₃N]⁺)
NEGATIVE m/z 700.3 (median) ([C₄H₆O₄₀Si₃W₁₁]⁴⁻)

### <Synthesis Example 16: tetrakis((2-trifluoromethylbenzyl)triallylammonium)(1,3-di(p-styryl)disiloxane-1,1,3,3-tetrayl)undecatungstosilicate>

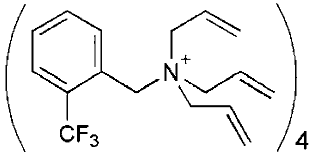

[SiW₁₁O₃₉(p-CH₂=CH-(C₆H₄)-Si)₂O] ⁴⁻

The target compound was produced in the same manner as in Synthesis Example 1 except that p-styryltrimethoxysilane was used as a raw material compound instead of methyltrimethoxysilane.

¹H-NMR (400 MHz, DMSO-d6): δ (ppm) = 4.04 (d, 24H), 4.73 (s, 8H), 5.29 (dd, 2H), 5.65 (d, 12H), 5.73 (d, 12H), 5.90 (dd, 2H), 6.02-6.12 (m, 12H), 6.75 (dd, 2H), 7.51 (d, 4H), 7.72 (d, 4H), 7.80-7.88 (m, 12H), 7.94 (d, 4H).

²⁹Si-NMR (119.22 MHz, DMSO-d6): δ (ppm) = -65.44 (s, 2Si), -84.98 (s, 1Si).

ESI-MS: POSITIVE m/z 296.2 ([C₁₇H₂₁F₃N]⁺)
NEGATIVE m/z 738.1 (median) ([C₁₆H₁₄O₄₀Si₃W₁₁]⁴⁻)

### <Synthesis Example 17: tetrakis((2-trifluoromethylbenzyl)triallylammonium)(1,3-di(3-acryloyloxypropyl)disiloxane-1,1,3,3-tetrayl)undecatungstosilicate>

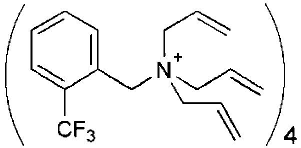

[SiW₁₁O₃₉(CH₂=CH-C(=O)-O-(CH₂)₃-Si)₂O] ⁴⁻

The target compound was produced in the same manner as in Synthesis Example 1 except that 3-acryloyloxypropyltrimethoxysilane was used as a raw material compound instead of methyltrimethoxysilane.

¹H-NMR (400 MHz, DMSO-d6): δ (ppm) = 0.57-0.61 (m, 4H), 1.77-1.85 (m, 4H), 4.04-4.10 (m, 28H), 4.73 (s, 8H), 5.65 (d, 12H), 5.73 (d, 12H), 5.89 (dd, 2H), 6.02-6.12 (m, 12H), 6.17 (dd, 2H), 6.31 (dd, 2H), 7.80-7.88 (m, 12H), 7.94 (d, 4H).

²⁹Si-NMR (119.22 MHz, DMSO-d6): δ (ppm) = -53.11 (s, 2Si), -85.07 (s, 1Si).

ESI-MS: POSITIVE m/z 296.2 ([C₁₇H₂₁F₃N]⁺)
NEGATIVE m/z 743.1 (median) ([C₁₂H₁₈O₄₄Si₃W₁₁]⁴⁻)

### <Synthesis Example 18: tetrakis((2-trifluoromethylbenzyl)triallylammonium)(1,3-di(3-methacryloyloxypropyl)disiloxane-1,1,3,3-tetrayl)undecatungstosilicate>

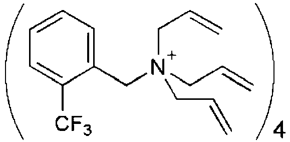

[SiW₁₁O₃₉(CH₂=C(CH₃)-C(=O)-O-(CH₂)₃-Si)₂O] ⁴⁻

The target compound was produced in the same manner as in Synthesis Example 1 except that 3-methacryloyloxypropyltrimethoxysilane was used as a raw material compound instead of methyltrimethoxysilane.

¹H-NMR (400 MHz, DMSO-d6): δ (ppm) = 0.57-0.61 (m, 4H), 1.78-1.83 (m, 4H), 1.86 (s, 6H), 4.04-4.09 (m, 28H), 4.73 (s, 8H), 5.62-5.65 (m, 14H), 5.73 (d, 12H)6.02-6.12 (m, 14H), 7.80-7.88 (m, 12H), 7.94 (d, 4H).

²⁹Si-NMR (119.22 MHz, DMSO-d6): δ (ppm) = -53.06 (s, 2Si), -85.08 (s, 1Si).

ESI-MS: POSITIVE m/z 296.2 ([C₁₇H₂₁F₃N]⁺)
NEGATIVE m/z 750.1 (median) ([C₁₄H₂₂O₄₄Si₃W₁₁]⁴⁻)

### <Synthesis Example 19: tetrakis((2-trifluoromethylbenzyl)triallylammonium)(1,3-di(3-chloropropyl)disiloxane-1,1,3,3-tetrayl)undecatungstosilicate>

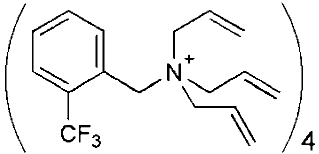

[SiW₁₁O₃₉(Cl-(CH₂)₃-Si)₂O] ⁴⁻

The target compound was produced in the same manner as in Synthesis Example 1 except that 3-chloropropyltrimethoxysilane was used as a raw material compound instead of methyltrimethoxysilane.

¹H-NMR (400 MHz, DMSO-d6): δ (ppm) = 0.68 (t, 4H), 1.87-1.96 (m, 4H), 3.67 (t, 4H), 4.04 (d, 24H), 4.73 (s, 8H), 5.65 (d, 12H), 5.73 (d, 12H)6.02-6.12 (m, 12H), 7.80-7.88 (m, 12H), 7.94 (d, 4H).

²⁹Si-NMR (119.22 MHz, DMSO-d6): δ (ppm) = -53.30 (s, 2Si), -85.07 (s, 1Si).

ESI-MS: POSITIVE m/z 296.2 ([C₁₇H₂₁F₃N]⁺)
NEGATIVE m/z 725.1 (median) ([C₆H₁₂Cl₂O₄₀Si₃W₁₁]⁴⁻)

### <Synthesis Example 20: tetrakis((2-trifluoromethylbenzyl)triallylammonium)(1,3-di(3-bromopropyl)disiloxane-1,1,3,3-tetrayl)undecatungstosilicate>

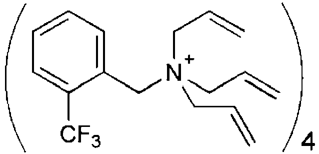

[SiW₁₁O₃₉(Br-(CH₂)₃-Si)₂O] ⁴⁻

The target compound was produced in the same manner as in Synthesis Example 1 except that 3-bromopropyltrimethoxysilane was used as a raw material compound instead of methyltrimethoxysilane.

¹H-NMR (400 MHz, DMSO-d6): δ (ppm) = 0.69 (t, 4H), 1.9502.04 (m, 4H), 3.58 (t, 4H), 4.04 (d, 24H), 4.73 (s, 8H), 5.65 (d, 12H), 5.73 (d, 12H)6.02-6.12 (m, 12H), 7.80-7.88 (m, 12H), 7.94 (d, 4H).

²⁹Si-NMR (119.22 MHz, DMSO-d6): δ (ppm) = -53.53 (s, 2Si), -85.07 (s, 1Si).

ESI-MS: POSITIVE m/z 296.2 ([C₁₇H₂₁F₃N]⁺)
NEGATIVE m/z 747.5 (median) ([C₆H₁₂Br₂O₄₀Si₃W₁₁]⁴⁻)

### <Synthesis Example 21: tetrakis((2-trifluoromethylbenzyl)triallylammonium)(1,3-di(3-iodopropyl)disiloxane-1,1,3,3-tetrayl)undecatungstosilicate>

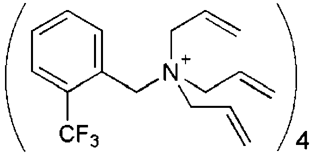

[SiW₁₁O₃₉(I-(CH₂)₃-Si)₂O] ⁴⁻

The target compound was produced in the same manner as in Synthesis Example 1 except that 3-iodopropyltrimethoxysilane was used as a raw material compound instead of methyltrimethoxysilane.

¹H-NMR (400 MHz, DMSO-d6): δ (ppm) = 0.66 (t, 4H), 1.90-2.02 (m, 4H), 3.56 (t, 4H), 4.04 (d, 24H), 4.73 (s, 8H), 5.65 (d, 12H), 5.73 (d, 12H)6.02-6.12 (m, 12H), 7.80-7.88 (m, 12H), 7.94 (d, 4H).

²⁹Si-NMR (119.22 MHz, DMSO-d6): δ (ppm) = -54.09 (s, 2Si), -85.09 (s, 1Si).

ESI-MS: POSITIVE m/z 296.2 ([C₁₇H₂₁F₃N]⁺)
NEGATIVE m/z 771.0 (median) ([C₆H₁₂I₂O₄₀Si₃W₁₁]⁴⁻)

### <Synthesis Example 22: tetrakis((2-trifluoromethylbenzyl)triallylammonium)(1,3-di(3-mercaptopropyl)disiloxane-1,1,3,3-tetrayl)undecatungstosilicate>

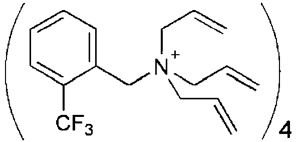

[SiW₁₁O₃₉(HS-(CH₂)₃-Si)₂O] ⁴⁻

The target compound was produced in the same manner as in Synthesis Example 1 except that 3-mercaptopropyltrimethoxysilane was used as a raw material compound instead of methyltrimethoxysilane.

¹H-NMR (400 MHz, DMSO-d6): δ (ppm) = 0.67 (t, 4H), 1.70-1.83 (m, 4H), 2.14 (t, 2H), 2.53-2.61 (m, 4H), 4.04 (d, 24H), 4.73 (s, 8H), 5.65 (d, 12H), 5.73 (d, 12H)6.02-6.12 (m, 12H), 7.80-7.88 (m, 12H), 7.94 (d, 4H).

²⁹Si-NMR (119.22 MHz, DMSO-d6): δ (ppm) = -53.06 (s, 2Si), -85.20 (s, 1Si).

ESI-MS: POSITIVE m/z 296.2 ([C₁₇H₂₁F₃N]⁺)
NEGATIVE m/z 724.1 (median) ([C₆H₁₄O₄₀S₂Si₃W₁₁]⁴⁻)

### <Synthesis Example 23: tetrakis((2-trifluoromethylbenzyl)triallylammonium)(1,3-di(chloromethyl)disiloxane-1,1,3,3-tetrayl)undecatungstosilicate>

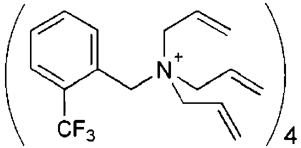

[SiW₁₁O₃₉(Cl-CH₂-Si)₂O] ⁴⁻

The target compound was produced in the same manner as in Synthesis Example 1 except that chloromethyltrimethoxysilane was used as a raw material compound instead of methyltrimethoxysilane.

¹H-NMR (400 MHz, DMSO-d6): δ (ppm) = 2.79 (s, 4H), 4.04 (d, 24H), 4.73 (s, 8H), 5.65 (d, 12H), 5.73 (d, 12H)6.02-6.12 (m, 12H), 7.80-7.88 (m, 12H), 7.94 (d, 4H).

²⁹Si-NMR (119.22 MHz, DMSO-d6): δ (ppm) = -63.05 (s, 2Si), -84.88 (s, 1Si).

ESI-MS: POSITIVE m/z 296.2 ([C₁₇H₂₁F₃N]⁺)
NEGATIVE m/z 711.0 (median) ([C₂H₄Cl₂O₄₀Si₃W₁₁]⁴⁻)

### <Synthesis Example 24: tetrakis((2-trifluoromethylbenzyl)triallylammonium)(1,3-di(3-(2-bromo-2-methylpropanoyloxy)propyl)disiloxane-1,1,3,3-tetrayl)undecatungstosilicate>

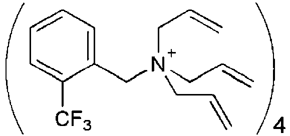

[SiW₁₁O₃₉(CH₃-C(Br)(CH₃)-C(=O)-O-(CH₂)₃-Si)₂O] ⁴⁻

The target compound was produced in the same manner as in Synthesis Example 1 except that 3-(2-bromo-2-methylpropanoyloxy)propyltrimethoxysilane was used as a raw material compound instead of methyltrimethoxysilane.

¹H-NMR (400 MHz, DMSO-d6): δ (ppm) = 0.60-0.64 (m, 4H), 1.79-1.86 (m, 4H), 1.89 (s, 12H), 4.04 (d, 24H), 4.15 (t, 4H), 4.73 (s, 8H), 5.65 (d, 12H), 5.73 (d, 12H)6.02-6.12 (m, 12H), 7.80-7.88 (m, 12H), 7.94 (d, 4H).

²⁹Si-NMR (119.22 MHz, DMSO-d6): δ (ppm) = -53.13 (s, 2Si), -85.17 (s, 1Si).

ESI-MS: POSITIVE m/z 296.2 ([C₁₇H₂₁F₃N]⁺)
NEGATIVE m/z 790.5 (median) ([C₁₄H₂₄Br₂O₄₀Si₃W₁₁]⁴⁻)

### <Synthesis Example 25: tetrakis((2-trifluoromethylbenzyl)triallylammonium)(1,3-di(4-methoxymethoxyphenyl)disiloxane-1,1,3,3-tetrayl)undecatungstosilicate>

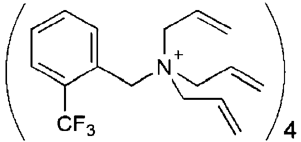

[SiW₁₁O₃₉(p-CH₃-O-CH₂-O-(C₆H₄)-Si)₂O]⁴⁻

The target compound was produced in the same manner as in Synthesis Example 1 except that 4-methoxymethoxyphenyltrimethoxysilane was used as a raw material compound instead of methyltrimethoxysilane.

¹H-NMR (400 MHz, DMSO-d6): δ (ppm) = 3.37 (s, 6H), 4.04 (d, 24H), 4.73 (s, 8H), 5.22 (s, 4H), 5.65 (d, 12H), 5.73 (d, 12H)6.02-6.12 (m, 12H), 7.05 (d, 4H), 7.66 (d, 4H), 7.80-7.88 (m, 12H), 7.94 (d, 4H).

²⁹Si-NMR (119.22 MHz, DMSO-d6): δ (ppm) = -64.72 (s, 2Si), -85.02 (s, 1Si).

ESI-MS: POSITIVE m/z 296.2 ([C₁₇H₂₁F₃N]⁺)
NEGATIVE m/z 755.1 (median) ([C₁₆H₁₈O₄₄Si₃W₁₁]⁴⁻)

### <Synthesis Example 26: tetrakis((2-trifluoromethylbenzyl)triallylammonium)(1,3-di(4-methoxybenzyl)disiloxane-1,1,3,3-tetrayl)undecatungstosilicate>

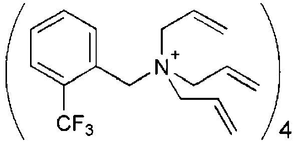

[SiW₁₁O₃₉(p-CH₃-O-(C₆H₄)-CH₂-Si)₂O]⁴⁻

The target compound was produced in the same manner as in Synthesis Example 1 except that 4-methoxybenzyltrimethoxysilane was used as a raw material compound instead of methyltrimethoxysilane.

¹H-NMR (400 MHz, DMSO-d6): δ (ppm) = 1.99 (q, 4H), 3.71 (s, 6H), 4.04 (d, 24H), 4.73 (s, 8H), 5.65 (d, 12H), 5.73 (d, 12H)6.02-6.12 (m, 12H), 6.73 (d, 4H), 7.05 (d, 4H), 7.80-7.88 (m, 12H), 7.94 (d, 4H).

²⁹Si-NMR (119.22 MHz, DMSO-d6): δ (ppm) = -57.96 (s, 2Si), -85.02 (s, 1Si).

ESI-MS: POSITIVE m/z 296.2 ([C₁₇H₂₁F₃N]⁺)
NEGATIVE m/z 747.1 (median) ([C₁₆H₁₈O₄₄Si₃W₁₁]⁴⁻)

### <Synthesis Example 27: tetrakis((2-trifluoromethylbenzyl)triallylammonium)(1,3-di(4-hydroxyphenyl)disiloxane-1,1,3,3-tetrayl)undecatungstosilicate>

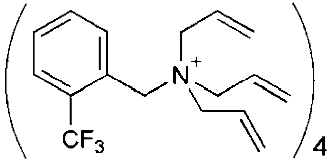

[SiW₁₁O₃₉(p-HO-(C₆H₄)-Si)₂O]⁴⁻

The target compound was produced in the same manner as in Synthesis Example 1 except that 4-hydroxyphenyltrimethoxysilane was used as a raw material compound instead of methyltrimethoxysilane.

¹H-NMR (400 MHz, DMSO-d6): δ (ppm) = 4.04 (d, 24H), 4.73 (s, 8H), 5.65 (d, 12H), 5.73 (d, 12H)6.02-6.12 (m, 12H), 6.76 (d, 4H), 7.54 (d, 4H), 7.80-7.88 (m, 12H), 7.94 (d, 4H), 9.56 (s, 2H).

²⁹Si-NMR (119.22 MHz, DMSO-d6): δ (ppm) = -64.28 (s, 2Si), -85.00 (s, 1Si).

ESI-MS: POSITIVE m/z 296.2 ([C₁₇H₂₁F₃N]⁺)
NEGATIVE m/z 733.1 (median) ([C₁₂H₁₀O₄₂Si₃W₁₁]⁴⁻)

### <Synthesis Example 28: tetrakis((2-trifluoromethylbenzyl)triallylammonium)(1,3-di(2-carboxyethyl)disiloxane-1,1,3,3-tetrayl)undecatungstosilicate>

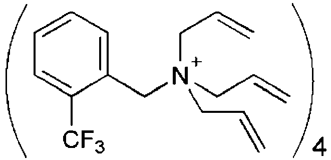

[SiW₁₁O₃₉(HOOC-(CH₂)₂-Si)₂O]⁴⁻

The target compound was produced in the same manner as in Synthesis Example 1 except that 2-carboxyethyltrihydroxysilane was used as a raw material compound instead of methyltrimethoxysilane.

¹H-NMR (400 MHz, DMSO-d6): δ (ppm) = 0.77-0.81 (m, 4H), 2.32-2.37 (m, 4H), 4.04 (d, 24H), 4.73 (s, 8H), 5.65 (d, 12H), 5.73 (d, 12H)6.02-6.12 (m, 12H), 7.80-7.88 (m, 12H), 7.94 (d, 4H), 11.97 (br, 2H).

²⁹Si-NMR (119.22 MHz, DMSO-d6): δ (ppm) = -53.31 (s, 2Si), -85.07 (s, 1Si).

ESI-MS: POSITIVE m/z 296.2 ([C₁₇H₂₁F₃N]⁺)
NEGATIVE m/z 723.1 (median) ([C₆H₁₀O₄₄Si₃W₁₁]⁴⁻)

### <Production Example 2: 4-(t-butoxycarbonyloxy)phenyltrimethoxysilane>

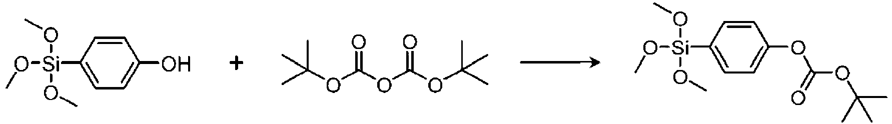

1.1 g of 4-hydroxyphenyltrimethoxysilane was dissolved in 10 mL of THF, and 0.033 g of DMAP was added thereto. 1.27 g of Boc anhydride was then added thereto and was stirred under reflux conditions. Volatile components were evaporated using a rotary evaporator, and the residue was extracted into an organic layer by liquid-liquid extraction with ethyl acetate and water. The organic layer was washed with an aqueous solution of sodium chloride and was subjected to column chromatography using hexane and ethyl acetate to produce 1.42 g of the target compound as a colorless liquid.

¹H-NMR (400 MHz, DMSO-d6): δ (ppm) = 1.42 (s, 9H), 3.55 (s, 9H), 7.15 (s, 4H).

### <Synthesis Example 29: tetrakis((2-trifluoromethylbenzyl)triallylammonium)(1,3-di(4-(t-butoxycarbonyloxy)phenyl)disiloxane-1,1,3,3-tetrayl)undecatungstosilicate>

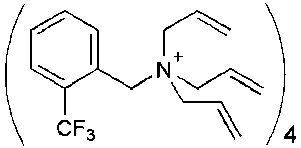

[SiW₁₁O₃₉(p-t-Bu-O-C(=O)-O-(C₆H₄)-Si)₂O]⁴⁻

The target compound was produced in the same manner as in Synthesis Example 1 except that 4-(t-butoxycarbonyloxy)phenyltrimethoxysilane was used as a raw material compound instead of methyltrimethoxysilane.

¹H-NMR (400 MHz, DMSO-d6): δ (ppm) = 1.55 (s, 18H), 4.04 (d, 24H), 4.73 (s, 8H), 5.65 (d, 12H), 5.73 (d, 12H), 6.02-6.12 (m, 12H), 7.01 (d, 4H), 7.76 (d, 4H), 7.80-7.88 (m, 12H), 7.94 (d, 4H).

²⁹Si-NMR (119.22 MHz, DMSO-d6): δ (ppm) = -65.64 (s, 2Si), -85.03 (s, 1Si).

ESI-MS: POSITIVE m/z 296.2 ([C₁₇H₂₁F₃N]⁺)
NEGATIVE m/z 783.1 (median) ([C₂₂H₂₆O₄₆Si₃W₁₁]⁴⁻)

### <Production Example 3: 2-((1-methylcyclopentyl)oxycarbonyl)ethyltrimethoxysilane>

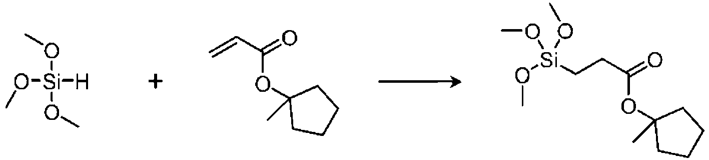

6.1 g of trimethoxysilane and 6.4 g of 1-methylcyclopentyl acrylate were added thereto, the liquid mixture was stirred at room temperature, and 100 µL of 1 M hexachloroplatinic acid in isopropyl alcohol was added thereto. After the beginning of the reaction, the reaction liquid was heated to 60°C and was then allowed to cool to room temperature. The reaction liquid was stirred at room temperature for 72 hours and was filtered through Celite, and the resulting filtrate was sufficiently dried under vacuum to produce 13.1 g of the target compound as a colorless oily liquid.

¹H-NMR (400 MHz, DMSO-d6): δ (ppm) = 0.97 (t, 2H), 1.39 (s, 3H), 1.56-1.81 (m, 8H), 2.21 (t, 2H), 3.55 (s, 9H).

### <Synthesis Example 30: tetrakis((2-trifluoromethylbenzyl)triallylammonium)(1,3-di(2-((1-methylcyclopentyl)oxycarbonyl)ethyl)disiloxane-1,1,3,3-tetrayl)undecatungstosilicate>

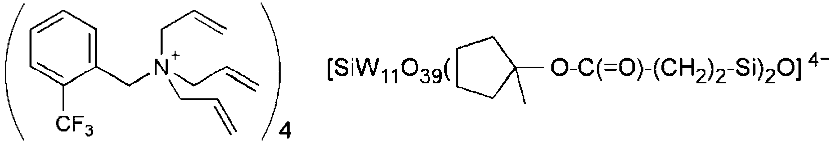

The target compound was produced in the same manner as in Synthesis Example 1 except that 2-((1-methylcyclopentyl)oxycarbonyl)ethyltrimethoxysilane was used as a raw material compound instead of methyltrimethoxysilane.

¹H-NMR (400 MHz, DMSO-d6): δ (ppm) = 0.77-0.81 (m, 4H), 1.49-1.84 (m, 22H), 2.37-2.42 (m, 4H), 4.04 (d, 24H), 4.73 (s, 8H), 5.65 (d, 12H), 5.73 (d, 12H), 6.02-6.12 (m, 12H), 7.80-7.88 (m, 12H), 7.94 (d, 4H).

²⁹Si-NMR (119.22 MHz, DMSO-d6): δ (ppm) = -53.12 (s, 2Si), -85.01 (s, 1Si).

ESI-MS: POSITIVE m/z 296.2 ([C₁₇H₂₁F₃N]⁺)
NEGATIVE m/z 764.4 (median) ([C₁₈H₃₀O₄₄Si₃W₁₁]⁴⁻)

### <Production Example 4: 2-((2-methyl-2-adamantyl)oxycarbonyl)ethyltrimethoxysilane>

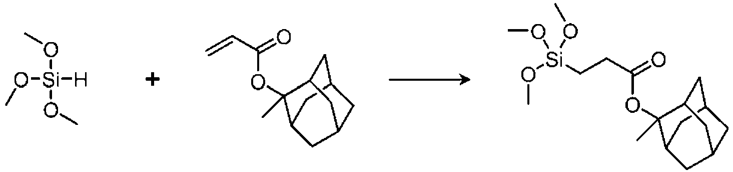

The target compound was produced in the same manner as in Production Example 3 except that 2-methyl-2-adamantyl acrylate was used as a raw material compound instead of 1-methylcyclopentyl acrylate.

¹H-NMR (400 MHz, DMSO-d6): δ (ppm) = 0.97 (t, 2H), 1.45-2.00 (m, 17H), 2.21 (t, 2H), 3.55 (s, 9H).

### <Synthesis Example 31: tetrakis((2-trifluoromethylbenzyl)triallylammonium)(1,3-di(2-((2-methyl-2-adamantyl)oxycarbonyl)ethyl)disiloxane-1,1,3,3-tetrayl)undecatungstosilicate>

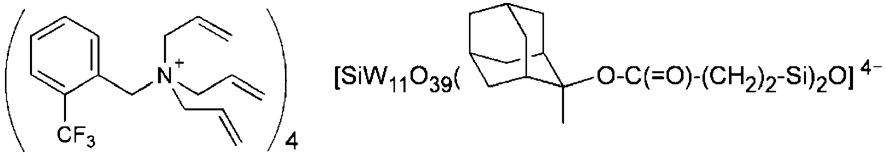

The target compound was produced in the same manner as in Synthesis Example 1 except that 2-((2-methyl-2-adamantyl)oxycarbonyl)ethyltrimethoxysilane was used as a raw material compound instead of methyltrimethoxysilane.

¹H-NMR (400 MHz, DMSO-d6): δ (ppm) = 0.76-0.81 (m, 4H), 1.45-2.00 (m, 34H), 2.38-2.44 (m, 4H), 4.04 (d, 24H), 4.73 (s, 8H), 5.65 (d, 12H), 5.73 (d, 12H), 6.02-6.12 (m, 12H), 7.80-7.88 (m, 12H), 7.94 (d, 4H).

²⁹Si-NMR (119.22 MHz, DMSO-d6): δ (ppm) = -53.07 (s, 2Si), -85.03 (s, 1Si).

ESI-MS: POSITIVE m/z 296.2 ([C₁₇H₂₁F₃N]⁺)
NEGATIVE m/z 797.1 (median) ([C₂₈H₄₂O₄₄Si₃W₁₁]⁴⁻)

### <Synthesis Example 32: tetrakis(benzyltrimethylammonium)(1,3-di(4-methoxymethoxyphenyl)disiloxane-1,1,3,3-tetrayl)undecatungstosilicate>

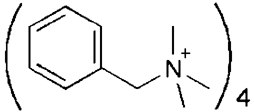

[SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of benzyltrimethylammonium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 3.04 (s, 36H), 3.37 (s, 6H), 4.53 (s, 8H), 5.22 (s, 4H), 7.05 (d, 4H), 7.50-7.57 (m, 20H), 7.66 (d, 4H).

²⁹Si-NMR (119.22 MHz, DMSO-d6): δ (ppm) = -64.72 (s, 2Si), -85.02 (s, 1Si).

ESI-MS: POSITIVE m/z 150.1 ([C₁₀H₁₆N]⁺)
NEGATIVE m/z 755.1 (median) ([C₁₆H₁₈O₄₄Si₃W₁₁]⁴⁻)

### <Synthesis Example 33: tetrakis(dimethyloctadecylammonium)(1,3-di(4-methoxymethoxyphenyl)disiloxane-1,1,3,3-tetrayl)undecatungstosilicate>

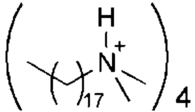

[SiW₁₁O₃₉(p-CH₃-O-CH₂-O-(C₆H₄)-Si)₂O]⁴⁻

The target compound was produced in the same manner as in Synthesis Example 1 except that, as raw material compounds, dimethyloctadecylamine was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 0.88 (t, 12H), 1.02-1.55 (m, 108H), 1.58-1.98 (m, 8H), 2.76-3.24 (m, 24H), 3.37 (s, 6H), 5.22 (s, 4H), 7.05 (d, 4H), 7.66 (d, 4H), 8.10 (br, 4H).

²⁹Si-NMR (119.22 MHz, DMSO-d6): δ (ppm) = -64.72 (s, 2Si), -85.02 (s, 1Si).

ESI-MS: POSITIVE m/z 298.3 ([C₂₀H₄₄N]⁺)
NEGATIVE m/z 755.1 (median) ([C₁₆H₁₈O₄₄Si₃W₁₁]⁴⁻)

### <Synthesis Example 34: tetrakis(hexyltriethylammonium)(1,3-di(4-methoxymethoxyphenyl)disiloxane-1,1,3,3-tetrayl)undecatungstosilicate>

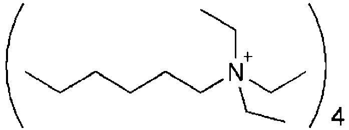

[SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of hexyltriethylammonium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 0.88 (t, 12H), 1.17-1.21 (m, 36H), 1.31 (br, 24H), 1.58 (br, 8H), 3.10-3.14 (m, 8H), 3.26 (q, 24H), 3.37 (s, 6H), 5.22 (s, 4H), 7.05 (d, 4H), 7.66 (d, 4H).

²⁹Si-NMR (119.22 MHz, DMSO-d6): δ (ppm) = -64.72 (s, 2Si), -85.02 (s, 1Si).

ESI-MS: POSITIVE m/z 186.2 ([C₁₂H₂₈N]⁺)
NEGATIVE m/z 755.1 (median) ([C₁₆H₁₈O₄₄Si₃W₁₁]⁴⁻)

### <Synthesis Example 35: tetrakis(2-acetoxyethyltrimethylammonium)(1,3-di(4-methoxymethoxyphenyl)disiloxane-1,1,3,3-tetrayl)undecatungstosilicate>

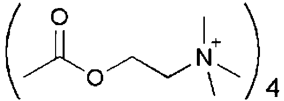

[SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of 2-acetoxyethyltrimethylammonium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 2.07 (s, 12H), 3.12 (s, 36H), 3.37 (s, 6H), 3.63-3.66 (m, 8H), 4.42-4.44 (m, 8H), 5.22 (s, 4H), 7.05 (d, 4H), 7.66 (d, 4H).

²⁹Si-NMR (119.22 MHz, DMSO-d6): δ (ppm) = -64.72 (s, 2Si), -85.02 (s, 1Si).

ESI-MS: POSITIVE m/z 146.1 ([C₇H₁₆NO₂]⁺)
NEGATIVE m/z 755.1 (median) ([C₁₆H₁₈O₄₄Si₃W₁₁]⁴⁻)

### <Synthesis Example 36: tetrakis((3-(dimethylcarbamoyloxy)phenyl)trimethylammonium)(1,3-di(4-methoxymethoxyphenyl)disiloxane-1,1,3,3-tetrayl)undecatungstosilicate>

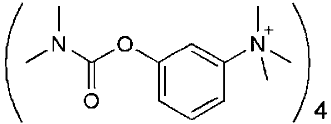

[SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of (3-(dimethylcarbamoyloxy)phenyl)trimethylammonium bromide was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 2.94 (s, 12H), 3.08 (s, 12H), 3.37 (s, 6H), 3.60 (s, 36H), 5.22 (s, 4H), 7.05 (d, 4H), 7.38 (d, 4H), 7.65-7.66 (m, 8H), 7.79-7.85 (s, 8H). ²⁹Si-NMR (119.22 MHz, DMSO-d6): δ (ppm) = -64.72 (s, 2Si), -85.02 (s, 1Si).

ESI-MS: POSITIVE m/z 223.1 ([C₁₂H₁₉N₂O₂]⁺)
NEGATIVE m/z 755.1 (median) ([C₁₆H₁₈O₄₄Si₃W₁₁]⁴⁻)

### <Synthesis Example 37: tetrakis(2-hydroxyethyltrimethylammonium)(1,3-di(4-methoxymethoxyphenyl)disiloxane-1,1,3,3-tetrayl)undecatungstosilicate>

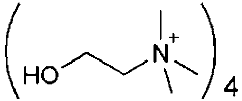

[SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of 2-hydroxyethyltrimethylammonium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 3.22 (s, 36H), 3.37 (s, 6H), 3.53 (t, 8H), 4.07 (m, 8H), 5.22 (s, 4H), 5.42 (t, 4H), 7.05 (d, 4H), 7.66 (d, 4H).

²⁹Si-NMR (119.22 MHz, DMSO-d6): δ (ppm) = -64.72 (s, 2Si), -85.02 (s, 1Si).

ESI-MS: POSITIVE m/z 104.1 ([C₅H₁₄NO]⁺)
NEGATIVE m/z 755.1 (median) ([C₁₆H₁₈O₄₄Si₃W₁₁]⁴⁻)

### <Synthesis Example 38: tetrakis((ethoxycarbonylmethyl)triethylammonium)(1,3-di(4-methoxymethoxyphenyl)disiloxane-1,1,3,3-tetrayl)undecatungstosilicate>

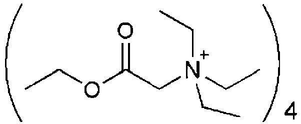

[SiW₁₁O₃₉(p-CH₃-O-CH₂-O-(C₆H₄)-Si)₂O]**⁴⁻**

The target compound was produced in the same manner as in Synthesis Example 1 except that, as raw material compounds, an aqueous solution of (ethoxycarbonylmethyl)triethylammonium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 1.23-1.28 (m, 48H), 3.37 (s, 6H), 3.51 (q, 24H), 4.25 (q, 8H), 4.33 (s, 8H), 5.22 (s, 4H), 7.05 (d, 4H), 7.66 (d, 4H).

²⁹Si-NMR (119.22 MHz, DMSO-d6): δ (ppm) = -64.72 (s, 2Si), -85.02 (s, 1Si).

ESI-MS: POSITIVE m/z 188.2 ([C₁₀H₂₂NO₂]⁺)
NEGATIVE m/z 755.1 (median) ([C₁₆H₁₈O₄₄Si₃W₁₁]⁴⁻)

### <Synthesis Example 39: tetrakis(dodecyltriethylammonium)(1,3-di(4-methoxymethoxyphenyl)disiloxane-1,1,3,3-tetrayl)undecatungstosilicate>

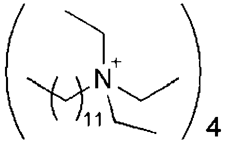

[SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of dodecyltriethylammonium bromide was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 0.86 (t, 12H), 1.18-1.31 (m, 108H), 1.57 (br, 8H), 3.10-3.14 (m, 8H), 3.26 (q, 24H), 3.37 (s, 6H), 5.22 (s, 4H), 7.05 (d, 4H), 7.66 (d, 4H). ²⁹Si-NMR (119.22 MHz, DMSO-d6): δ (ppm) = -64.72 (s, 2Si), -85.02 (s, 1Si).

ESI-MS: POSITIVE m/z 270.3 ([C₁₈H₄₀N]⁺)
NEGATIVE m/z 755.1 (median) ([C₁₆H₁₈O₄₄Si₃W₁₁]⁴⁻)

### <Synthesis Example 40: tetrakis(4,4,5,5,6,6,7,7,8,8,9,9,10,10,11,11,11-heptadecafluoroundecyltriethylammonium)(1,3-di(4-methoxymethoxyphenyl)disiloxane-1,1,3,3-tetrayl)undecatungstosilicate>

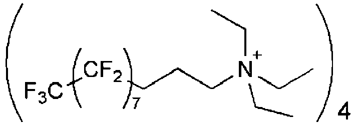

[SiW₁₁O₃₉(p-CH₃-O-CH₂-O-(C₆H₄)-Si)₂O]⁴⁻

The target compound was produced in the same manner as in Synthesis Example 1 except that, as raw material compounds, a 4,4,5,5,6,6,7,7,8,8,9,9,10,10,11,11,11-heptadecafluoroundecyltriethylammonium iodide solution was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 1.22 (t, 36H), 1.85-1.93 (m, 8H), 2.34-2.48 (m, 8H), 3.25-3.31 (m, 32H), 3.37 (s, 6H), 5.22 (s, 4H), 7.05 (d, 4H), 7.66 (d, 4H).

²⁹Si-NMR (119.22 MHz, DMSO-d6): δ (ppm) = -64.72 (s, 2Si), -85.02 (s, 1Si).

ESI-MS: POSITIVE m/z 562.1 ([C₁₇H₂₁F₁₇N]⁺)
NEGATIVE m/z 755.1 (median) ([C₁₆H₁₈O₄₄Si₃W₁₁]⁴⁻)

### <Synthesis Example 41: tetrakis(cyanomethyltriphenylphosphonium)(1,3-di(4-methoxymethoxyphenyl)disiloxane-1,1,3,3-tetrayl)undecatungstosilicate>

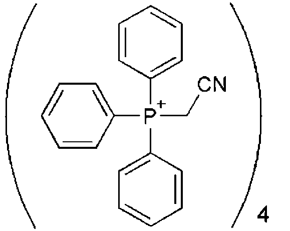

[SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of cyanomethyltriphenylphosphonium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 3.37 (s, 6H), 5.22 (s, 4H), 6.32 (d, 8H), 7.05 (d, 4H), 7.66 (d, 4H), 7.81-8.01 (m, 60H).

²⁹Si-NMR (119.22 MHz, DMSO-d6): δ (ppm) = -64.72 (s, 2Si), -85.02 (s, 1Si).

ESI-MS: POSITIVE m/z 302.1 ([C₂₀H₁₇NP]⁺)
NEGATIVE m/z 755.1 (median) ([C₁₆H₁₈O₄₄Si₃W₁₁]⁴⁻)

### <Synthesis Example 42: tetrakis(carbamoylmethyltriphenylphosphonium)(1,3-di(4-methoxymethoxyphenyl)disiloxane-1,1,3,3-tetrayl)undecatungstosilicate>

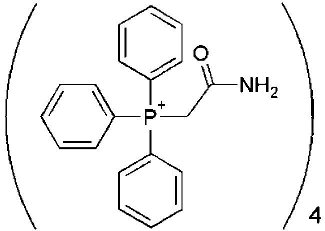

[SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of carbamoylmethyltriphenylphosphonium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 3.37 (s, 6H), 5.12 (d, 8H), 5.22 (s, 4H), 7.05 (d, 4H), 7.62-7.66 (m, 12H), 7.74-7.89 (m, 60H).

²⁹Si-NMR (119.22 MHz, DMSO-d6): δ (ppm) = -64.72 (s, 2Si), -85.02 (s, 1Si).

ESI-MS: POSITIVE m/z 320.1 ([C₂₀H₁₉NOP]⁺)
NEGATIVE m/z 755.1 (median) ([C₁₆H₁₈O₄₄Si₃W₁₁]⁴⁻)

### <Synthesis Example 43: tetrakis(propargyltriphenylphosphonium)(1,3-di(4-methoxymethoxyphenyl)disiloxane-1,1,3,3-tetrayl)undecatungstosilicate>

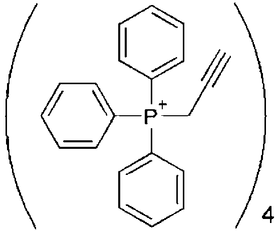

[SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of propargyltriphenylphosphonium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 3.37 (s, 6H), 3.48 (d, 4H), 5.21-5.22 (m, 12H), 7.05 (d, 4H), 7.66 (d, 4H), 7.76-7.99 (m, 60H).

²⁹Si-NMR (119.22 MHz, DMSO-d6): δ (ppm) = -64.72 (s, 2Si), -85.02 (s, 1Si).

ESI-MS: POSITIVE m/z 301.1 ([C₂₁H₁₈P]⁺)
NEGATIVE m/z 755.1 (median) ([C₁₆H₁₈O₄₄Si₃W₁₁]⁴⁻)

### <Synthesis Example 44: tetrakis(allyltriphenylphosphonium)(1,3-di(4-methoxymethoxyphenyl)disiloxane-1,1,3,3-tetrayl)undecatungstosilicate>

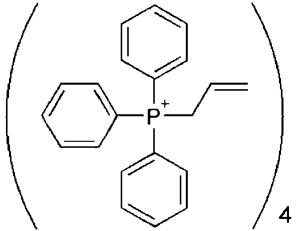

[SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of allyltriphenylphosphonium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 3.37 (s, 6H), 4.89 (dd, 8H), 5.22 (s, 4H), 5.38-5.42 (m, 4H), 5.64-5.74 (m, 8H), 7.05 (d, 4H), 7.66 (d, 4H), 7.69-7.88 (m, 60H).

²⁹Si-NMR (119.22 MHz, DMSO-d6): δ (ppm) = -64.72 (s, 2Si), -85.02 (s, 1Si).

ESI-MS: POSITIVE m/z 303.1 ([C₂₁H₂₀P]⁺)
NEGATIVE m/z 755.1 (median) ([C₁₆H₁₈O₄₄Si₃W₁₁]⁴⁻)

### <Synthesis Example 45: bis(trans-2-butene-1,4-bis(triphenylphosphonium))(1,3-di(4-methoxymethoxyphenyl)disiloxane-1,1,3,3-tetrayl)undecatungstosilicate>

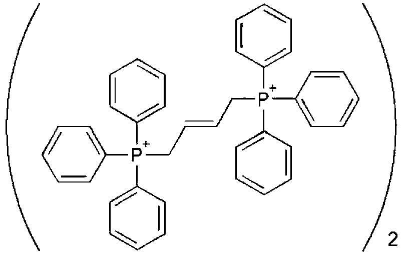

[SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of trans-2-butene-1,4-bis(triphenylphosphonium)chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 3.37 (s, 6H), 5.22 (s, 4H), 5.73 (dd, 16H), 6.34 (m, 8H), 7.05 (d, 4H), 7.76-7.99 (m, 124H).

²⁹Si-NMR (119.22 MHz, DMSO-d6): δ (ppm) = -64.72 (s, 2Si), -85.02 (s, 1Si).

ESI-MS: POSITIVE m/z 289.1 ([C₄₀H₃₆P₂]²⁺)
NEGATIVE m/z 755.1 (median) ([C₁₆H₁₈O₄₄Si₃W₁₁]⁴⁻)

### <Synthesis Example 46: tetrakis((4-vinylbenzyl)triallylammonium)(1,3-dimethyldisiloxane-1,1,3,3-tetrayl)undecatungstosilicate>

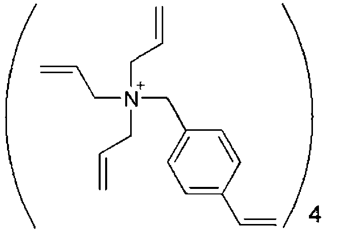

[SiW₄₄O₃₉(CH₃-Si)₂O]⁴⁻

The target compound was produced in the same manner as in Synthesis Example 1 except that an aqueous solution of (4-vinylbenzyl)triallylammonium chloride was used as a raw material compound instead of the aqueous solution of (2-trifluoromethylbenzyl)triallylammonium bromide.

¹H-NMR (400 MHz, DMSO-d6): δ (ppm) = 0.04 (s, 6H), 3.93 (d, 24H), 4.58 (s, 8H), 5.38 (d, 4H), 5.64-5.73 (m, 24H), 5.96 (d, 4H), 6.12-6.22 (m, 12H), 6.80 (dd, 4H), 7.62 (s, 16H). ²⁹Si-NMR (119.22 MHz, DMSO-d6): δ (ppm) = -51.15 (s, 2Si), -85.08 (s, 1Si).

ESI-MS: POSITIVE m/z 254.2 ([C₁₈H₂₄N]⁺)
NEGATIVE m/z 694.1 (median) ([C₂H₆O₄₀Si₃W₁₁]⁴⁻)

### <Synthesis Example 47: tetrakis((3-hydroxypropyl)triallylammonium)(1,3-dimethyldisiloxane-1,1,3,3-tetrayl)undecatungstosilicate>

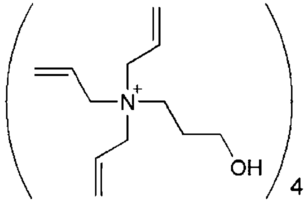

[SiW₁₁O₃₉(CH₃-Si)₂O]⁴⁻

The target compound was produced in the same manner as in Synthesis Example 1 except that an aqueous solution of (3-hydroxypropyl)triallylammonium iodide was used as a raw material compound instead of the aqueous solution of (2-trifluoromethylbenzyl)triallylammonium bromide.

¹H-NMR (400 MHz, DMSO-d6): δ (ppm) = 0.04 (s, 6H), 1.86-1.90 (m, 8H), 3.19-3.23 (m, 8H), 3.46 (t, 8H), 3.91 (d, 24H), 4.77 (br, 4H), 5.63-5.71 (m, 24H), 6.04-6.11 (m, 12H). ²⁹Si-NMR (119.22 MHz, DMSO-d6): δ (ppm) = -51.15 (s, 2Si), -85.08 (s, 1Si).

ESI-MS: POSITIVE m/z 196.2 ([C₁₂H₂₂NO]⁺)
NEGATIVE m/z 694.1 (median) ([C₂H₆O₄₀Si₃W₁₁]⁴⁻)

### <Synthesis Example 48: tetrakis(bis(2-(N-methylacrylamido)ethyl)dimethylammonium)(1,3-dimethyldisiloxane-1,1,3,3-tetrayl)undecatungstosilicate>

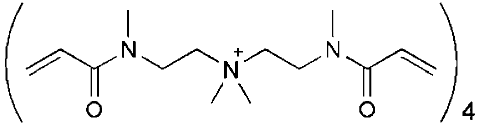

[SiW₁₁O₃₉(CH₃-Si)₂O]⁴⁻

The target compound was produced in the same manner as in Synthesis Example 1 except that an aqueous solution of bis(2-(N-methylacrylamido)ethyl)dimethylammonium iodide was used as a raw material compound instead of the aqueous solution of (2-trifluoromethylbenzyl)triallylammonium bromide.

¹H-NMR (400 MHz, DMSO-d6): δ (ppm) = 0.04 (s, 6H), 2.94-3.20 (m, 48H), 3.49 (t, 16H), 3.79 (t, 16H), 5.75 (dd, 8H), 6.17 (dd, 8H), 6.78 (dd, 8H).

²⁹Si-NMR (119.22 MHz, DMSO-d6): δ (ppm) = -51.15 (s, 2Si), -85.08 (s, 1Si).

ESI-MS: POSITIVE m/z 268.2 ([C₁₄H₂₆N₃O₂]⁺)
NEGATIVE m/z 694.1 (median) ([C₂H₆O₄₀Si₃W₁₁]⁴⁻)

### <Synthesis Example 49: tetrarubidium(1,3-di(4-methoxymethoxyphenyl)disiloxane-1,1,3,3-tetrayl)undecatungstosilicate>

(Rb⁺)₄ [SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of rubidium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 3.37 (s, 6H), 5.22 (s, 4H), 7.05 (d, 4H), 7.66 (d, 4H).

²⁹Si-NMR (119.22 MHz, DMSO-d6): δ (ppm) = -64.72 (s, 2Si), -85.02 (s, 1Si).

ESI-MS: POSITIVE m/z 84.9 ([Rb]⁺)
NEGATIVE m/z 755.1 (median) ([C₁₆H₁₈O₄₄Si₃W₁₁]⁴⁻)

### <Synthesis Example 50: tetracesium(1,3-di(4-methoxymethoxyphenyl)disiloxane-1,1,3,3-tetrayl)undecatungstosilicate>

(Cs⁺)₄ [SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of cesium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 3.37 (s, 6H), 5.22 (s, 4H), 7.05 (d, 4H), 7.66 (d, 4H).

²⁹Si-NMR (119.22 MHz, DMSO-d6): δ (ppm) = -64.72 (s, 2Si), -85.02 (s, 1Si).

ESI-MS: POSITIVE m/z 132.9 ([Cs]⁺)
NEGATIVE m/z 755.1 (median) ([C₁₆H₁₈O₄₄Si₃W₁₁]⁴⁻)

### <Synthesis Example 51: tetrakis(tetraallylammonium)(1,3-divinyldisiloxane-1,1,3,3-tetrayl)undecatungstosilicate>

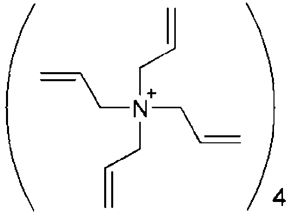

[SiW₁₁O₃₉(CH₂=CH-Si)₂O]⁴⁻

The target compound was produced in the same manner as in Synthesis Example 1 except that, as raw material compounds, an aqueous solution of tetraallylammonium iodide was used instead of the aqueous solution of (2-trifluoromethylbenzyl)triallylammonium bromide, and vinyltrimethoxysilane was used instead of methyltrimethoxysilane.

¹H-NMR (400 MHz, DMSO-d6): δ (ppm) = 3.90 (d, 32H), 5.66-5.72 (m, 32H), 5.94 (dd, 2H), 6.08 (dd, 2H), 6.05-6.14 (dd, 18H).

²⁹Si-NMR (119.22 MHz, DMSO-d6): δ (ppm) = -66.56 (s, 2Si), -85.29 (s, 1Si).

¹⁸³W-NMR (20.84 MHz, DMSO-d6): δ (ppm) = -105.52 (s, 2W), -107.54 (s, 2W), -112.04 (s, 1W), -126.08 (s, 2W), -170.95 (s, 2W), -247.66 (s, 2W).

ESI-MS: POSITIVE m/z 178.2 ([C₁₂H₂₀N]⁺)
NEGATIVE m/z 700.3 (median) ([C₄H₆O₄₀Si₃W₁₁]⁴⁻)

### <Synthesis Example 52: tetrakis((4-vinylbenzyl)triallylammonium)(1,3-divinyldisiloxane-1,1,3,3-tetrayl)undecatungstosilicate>

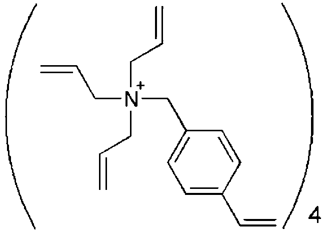

[SiW₁₁O₃₉(CH₂=CH-Si)₂O]⁴⁻

The target compound was produced in the same manner as in Synthesis Example 1 except that, as raw material compounds, an aqueous solution of (4-vinylbenzyl)triallylammonium chloride was used instead of the aqueous solution of (2-trifluoromethylbenzyl)triallylammonium bromide, and vinyltrimethoxysilane was used instead of methyltrimethoxysilane.

¹H-NMR (400 MHz, DMSO-d6): δ (ppm) = 3.93 (d, 24H), 4.58 (s, 8H), 5.38 (d, 4H), 5.64-5.73 (m, 24H), 5.94-5.96 (m, 6H), 6.08 (dd, 2H), 6.12-6.22 (m, 14H), 6.80 (dd, 4H), 7.62 (s, 16H).

²⁹Si-NMR (119.22 MHz, DMSO-d6): δ (ppm) = -66.56 (s, 2Si), -85.29 (s, 1Si).

¹⁸³W-NMR (20.84 MHz, DMSO-d6): δ (ppm) = -105.52 (s, 2W), -107.54 (s, 2W), -112.04 (s, 1W), -126.08 (s, 2W), -170.95 (s, 2W), -247.66 (s, 2W).

ESI-MS: POSITIVE m/z 254.2 ([C₁₈H₂₄N]⁺)
NEGATIVE m/z 700.3 (median) ([C₄H₆O₄₀Si₃W₁₁]⁴⁻)

### <Synthesis Example 53: tetrakis((3-hydroxypropyl)triallylammonium)(1,3-divinyldisiloxane-1,1,3,3-tetrayl)undecatungstosilicate>

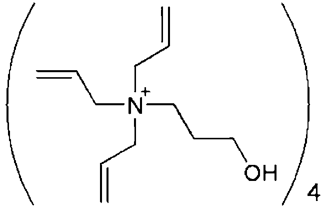

[SiW₁₁O₃₉(CH₂=CH-Si)₂O]⁴⁻

The target compound was produced in the same manner as in Synthesis Example 1 except that, as raw material compounds, an aqueous solution of (3-hydroxypropyl)triallylammonium iodide was used instead of the aqueous solution of (2-trifluoromethylbenzyl)triallylammonium bromide, and vinyltrimethoxysilane was used instead of methyltrimethoxysilane.

¹H-NMR (400 MHz, DMSO-d6): δ (ppm) = 1.86-1.90 (m, 8H), 3.19-3.23 (m, 8H), 3.46 (t, 8H), 3.91 (d, 24H), 4.77 (br, 4H), 5.63-5.71 (m, 24H), 5.94 (dd, 2H), 6.04-6.13 (m, 16H). ²⁹Si-NMR (119.22 MHz, DMSO-d6): δ (ppm) = -66.56 (s, 2Si), -85.29 (s, 1Si).

¹⁸³W-NMR (20.84 MHz, DMSO-d6): δ (ppm) = -105.52 (s, 2W), -107.54 (s, 2W), -112.04 (s, 1W), -126.08 (s, 2W), -170.95 (s, 2W), -247.66 (s, 2W).

ESI-MS: POSITIVE m/z 196.2 ([C₁₂H₂₂NO]⁺)
NEGATIVE m/z 700.3 (median) ([C₄H₆O₄₀Si₃W₁₁]⁴⁻)

### <Synthesis Example 54: tetrakis(bis(2-(N-methylacrylamido)ethyl)dimethylammonium)(1,3-divinyldisiloxane-1,1,3,3-tetrayl)undecatungstosilicate>

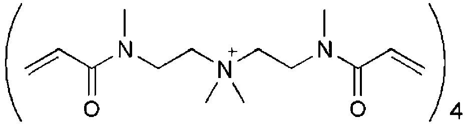

[SiW₁₁O₃₉(CH₂=CH-Si)₂O]⁴⁻

The target compound was produced in the same manner as in Synthesis Example 1 except that, as raw material compounds, an aqueous solution of bis(2-(N-methylacrylamido)ethyl)dimethylammonium iodide was used instead of the aqueous solution of (2-trifluoromethylbenzyl)triallylammonium bromide, and vinyltrimethoxysilane was used instead of methyltrimethoxysilane.

¹H-NMR (400 MHz, DMSO-d6): δ (ppm) = 2.94-3.20 (m, 48H), 3.49 (t, 16H), 3.79 (t, 16H), 5.75 (dd, 8H), 5.94 (dd, 2H), 6.08 (dd, 2H), 6.13 (dd, 2H), 6, 17 (dd, 8H), 6.78 (dd, 8H). ²⁹Si-NMR (119.22 MHz, DMSO-d6): δ (ppm) = -66.56 (s, 2Si), -85.29 (s, 1Si).

¹⁸³W-NMR (20.84 MHz, DMSO-d6): δ (ppm) = -105.52 (s, 2W), -107.54 (s, 2W), -112.04 (s, 1W), -126.08 (s, 2W), -170.95 (s, 2W), -247.66 (s, 2W).

ESI-MS: POSITIVE m/z 268.2 ([C₁₄H₂₆N₃O₂]⁺)
NEGATIVE m/z 700.3 (median) ([C₄H₆O₄₀Si₃W₁₁]⁴⁻)

### <Synthesis Example 55: tetrakis(diallyldimethylammonium)(1,3-divinyldisiloxane-1,1,3,3-tetrayl)undecatungstosilicate>

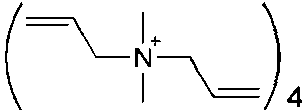

[SiW₁₁O₃₉(CH₂=CH-Si)₂O]⁴⁻

The target compound was produced in the same manner as in Synthesis Example 1 except that, as raw material compounds, an aqueous solution of diallyldimethylammonium chloride was used instead of the aqueous solution of (2-trifluoromethylbenzyl)triallylammonium bromide, and vinyltrimethoxysilane was used instead of methyltrimethoxysilane.

¹H-NMR (400 MHz, DMSO-d6): δ (ppm) = 3.01 (s, 24H), 4.02 (d, 16H), 5.62-5.68 (m, 16H), 5.94 (dd, 2H), 6.04-6.14 (m, 12H).

²⁹Si-NMR (119.22 MHz, DMSO-d6): δ (ppm) = -66.56 (s, 2Si), -85.29 (s, 1Si).

¹⁸³W-NMR (20.84 MHz, DMSO-d6): δ (ppm) = -105.52 (s, 2W), -107.54 (s, 2W), -112.04 (s, 1W), -126.08 (s, 2W), -170.95 (s, 2W), -247.66 (s, 2W).

ESI-MS: POSITIVE m/z 126.1 ([C₈H₁₆N]⁺)
NEGATIVE m/z 700.3 (median) ([C₄H₆O₄₀Si₃W₁₁]⁴⁻)

### <Synthesis Example 56: tetrakis(vinyltrimethylammonium)(1,3-divinyldisiloxane-1,1,3,3-tetrayl)undecatungstosilicate>

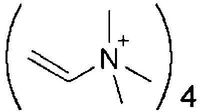

[SiW₁₁O₃₉(CH₂=CH-Si)₂O]⁴⁻

The target compound was produced in the same manner as in Synthesis Example 1 except that, as raw material compounds, an aqueous solution of vinyltrimethylammonium bromide was used instead of the aqueous solution of (2-trifluoromethylbenzyl)triallylammonium bromide, and vinyltrimethoxysilane was used instead of methyltrimethoxysilane.

¹H-NMR (400 MHz, DMSO-d6): δ (ppm) = 3.33 (s, 36H), 5.54-5.58 (m, 4H), 5.80 (d, 4H), 5.94 (dd, 2H), 6.08 (dd, 2H), 6.13 (dd, 2H), 6.67-6.73 (m, 4H).

²⁹Si-NMR (119.22 MHz, DMSO-d6): δ (ppm) = -66.56 (s, 2Si), -85.29 (s, 1Si).

¹⁸³W-NMR (20.84 MHz, DMSO-d6): δ (ppm) = -105.52 (s, 2W), -107.54 (s, 2W), -112.04 (s, 1W), -126.08 (s, 2W), -170.95 (s, 2W), -247.66 (s, 2W).

ESI-MS: POSITIVE m/z 86.1 ([C₅H₁₂N]⁺)
NEGATIVE m/z 700.3 (median) ([C₄H₆O₄₀Si₃W₁₁]⁴⁻)

### <Synthesis Example 57: tetrakis(allyltriphenylphosphonium)(1,3-divinyldisiloxane-1,1,3,3-tetrayl)undecatungstosilicate>

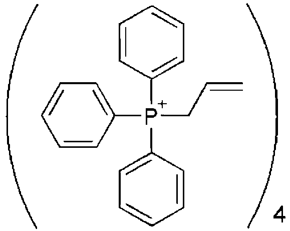

[SiW₁₁O₃₉(CH₂=CH-Si)₂O]⁴⁻

The target compound was produced in the same manner as in Synthesis Example 1 except that, as raw material compounds, an aqueous solution of allyltriphenylphosphonium chloride was used instead of the aqueous solution of (2-trifluoromethylbenzyl)triallylammonium bromide, and vinyltrimethoxysilane was used instead of methyltrimethoxysilane.

¹H-NMR (400 MHz, DMSO-d6): δ (ppm) = 4.89 (dd, 8H), 5.38-5.42 (m, 4H), 5.64-5.74 (m, 8H), 5.94 (dd, 2H), 6.08 (dd, 2H), 6.13 (dd, 2H), 7.69-7.88 (m, 60H).

²⁹Si-NMR (119.22 MHz, DMSO-d6): δ (ppm) = -66.56 (s, 2Si), -85.29 (s, 1Si).

¹⁸³W-NMR (20.84 MHz, DMSO-d6): δ (ppm) = -105.52 (s, 2W), -107.54 (s, 2W), -112.04 (s, 1W), -126.08 (s, 2W), -170.95 (s, 2W), -247.66 (s, 2W).

ESI-MS: POSITIVE m/z 301.1 ([C₂₁H₂₀P]⁺)
NEGATIVE m/z 700.3 (median) ([C₄H₆O₄₀Si₃W₁₁]⁴⁻)

### <Synthesis Example 58: tetrakis(tetraallylammonium)(1,3-di(p-styryl)disiloxane-1,1,3,3-tetrayl)undecatungstosilicate>

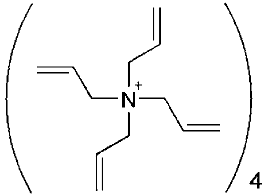

[SiW₁₁O₃₉(p-CH₂=CH-(C₆H₄)-Si)₂O]⁴⁻

The target compound was produced in the same manner as in Synthesis Example 1 except that, as raw material compounds, an aqueous solution of tetraallylammonium iodide was used instead of the aqueous solution of (2-trifluoromethylbenzyl)triallylammonium bromide, and p-styryltrimethoxysilane was used instead of methyltrimethoxysilane. ¹H-NMR (400 MHz, DMSO-d6): δ (ppm) = 3.90 (d, 32H), 5.29 (dd, 2H), 5.66-5.72 (m, 32H), 5.90 (dd, 2H), 6.05-6.14 (m, 16H), 6.75 (dd, 2H), 7.51 (d, 4H), 7.72 (d, 4H). ²⁹Si-NMR (119.22 MHz, DMSO-d6): δ (ppm) = -65.44 (s, 2Si), -84.98 (s, 1Si).

ESI-MS: POSITIVE m/z 178.2 ([C₁₂H₂₀N]⁺)
NEGATIVE m/z 738.1 (median) ([C₁₆H₁₄O₄₀Si₃W₁₁]⁴⁻)

### <Synthesis Example 59: tetrakis((4-vinylbenzyl)triallylammonium)(1,3-di(p-styryl)disiloxane-1,1,3,3-tetrayl)undecatungstosilicate>

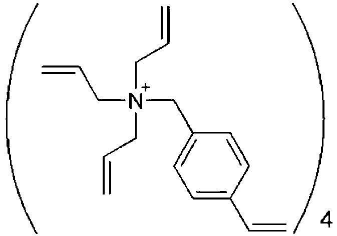

[SiW₁₁O₃₉(p-CH₂=CH-(C₆H₄)-Si)₂O]⁴⁻

The target compound was produced in the same manner as in Synthesis Example 1 except that, as raw material compounds, an aqueous solution of (4-vinylbenzyl)triallylammonium chloride was used instead of the aqueous solution of (2-trifluoromethylbenzyl)triallylammonium bromide, and p-styryltrimethoxysilane was used instead of methyltrimethoxysilane.

¹H-NMR (400 MHz, DMSO-d6): δ (ppm) = 3.93 (d, 24H), 4.58 (s, 8H), 5.29 (dd, 2H), 5.38 (d, 4H), 5.64-5.73 (m, 24H), 5.90 (dd, 2H), 5.96 (d, 4H), 6.12-6.22 (m, 12H), 6.75 (dd, 2H), 6.80 (dd, 4H), 7.51 (d, 4H), 7.62 (s, 16H), 7.72 (d, 4H).

²⁹Si-NMR (119.22 MHz, DMSO-d6): δ (ppm) = -65.44 (s, 2Si), -84.98 (s, 1Si).

ESI-MS: POSITIVE m/z 254.2 ([C₁₈H₂₄N]⁺)
NEGATIVE m/z 738.1 (median) ([C₁₆H₁₄O₄₀Si₃W₁₁]⁴⁻)

### <Synthesis Example 60: tetrakis((3-hydroxypropyl)triallylammonium)(1,3-di(p-styryl)disiloxane-1,1,3,3-tetrayl)undecatungstosilicate>

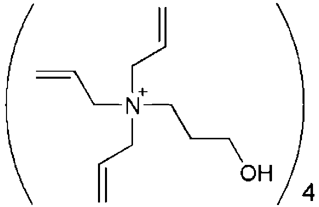

### [SiW₁₁O₃₉(p-CH₂=CH-(C₆H₄)-Si)₂O]⁴⁻

The target compound was produced in the same manner as in Synthesis Example 1 except that, as raw material compounds, an aqueous solution of (3-hydroxypropyl)triallylammonium iodide was used as a raw material compound instead of the aqueous solution of (2-trifluoromethylbenzyl)triallylammonium bromide, and p-styryltrimethoxysilane was used instead of methyltrimethoxysilane.

¹H-NMR (400 MHz, DMSO-d6): δ (ppm) = 1.86-1.90 (m, 8H), 3.19-3.23 (m, 8H), 3.46 (t, 8H), 3.91 (d, 24H), 4.77 (br, 4H), 5.29 (dd, 2H), 5.63-5.71 (m, 24H), 5.90 (dd, 2H), 6.04-6.11 (m, 12H), 6.75 (dd, 2H), 7.51 (d, 4H), 7.72 (d, 4H).

²⁹Si-NMR (119.22 MHz, DMSO-d6): δ (ppm) = -65.44 (s, 2Si), -84.98 (s, 1Si).

ESI-MS: POSITIVE m/z 196.2 ([C₁₂H₂₂NO]⁺)
NEGATIVE m/z 738.1 (median) ([C₁₆H₁₄O₄₀Si₃W₁₁]⁴⁻)

### <Synthesis Example 61: tetrakis((4-vinylbenzyl)trimethylammonium)(1,3-di(p-styryl)disiloxane-1,1,3,3-tetrayl)undecatungstosilicate>

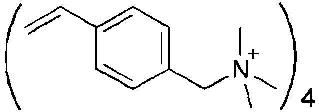

[SiW₁₁O₃₉(p-CH₂=CH-(C₆H₄)-Si)₂O]⁴⁻

The target compound was produced in the same manner as in Synthesis Example 1 except that, as raw material compounds, an aqueous solution of (4-vinylbenzyl)trimethylammonium chloride was used instead of the aqueous solution of (2-trifluoromethylbenzyl)triallylammonium bromide, and p-styryltrimethoxysilane was used instead of methyltrimethoxysilane.

¹H-NMR (400 MHz, DMSO-d6): δ (ppm) = 3.13 (s, 36H), 4.74 (s, 8H), 5.29 (dd, 2H), 5.38 (dd, 4H), 5.90-5.96 (m, 6H), 6.75-6.81 (m, 6H), 7.42-7.80 (m, 24H).

²⁹Si-NMR (119.22 MHz, DMSO-d6): δ (ppm) = -65.44 (s, 2Si), -84.98 (s, 1Si).

ESI-MS: POSITIVE m/z 176.1 ([C₁₂H₁₈N]⁺)
NEGATIVE m/z 738.1 (median) ([C₁₆H₁₄O₄₀Si₃W₁₁]⁴⁻)

### <Synthesis Example 62: tetrakis(bis(2-(N-methylacrylamido)ethyl)dimethylammonium)(1,3-di(3-acryloyloxypropyl)disiloxane-1,1,3,3-tetrayl)undecatungstosilicate>

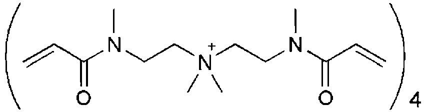

[SiW₁₁O₃₉(CH₂=CH-C(=O)-O-(CH₂)₃-Si)₂O]⁴⁻

The target compound was produced in the same manner as in Synthesis Example 1 except that, as raw material compounds, an aqueous solution of bis(2-(N-methylacrylamido)ethyl)dimethylammonium iodide was used instead of the aqueous solution of (2-trifluoromethylbenzyl)triallylammonium bromide, and 3-acryloyloxypropyloxytrimethoxysilane was used instead of methyltrimethoxysilane. ¹H-NMR (400 MHz, DMSO-d6): δ (ppm) = 0.57-0.61 (m, 4H), 1.77-1.85 (m, 4H), 2.94-3.20 (m, 48H), 3.49 (t, 16H), 3.79 (t, 16H), 4.10 (t, 4H), 5.75 (dd, 8H), 5.89 (dd, 2H), 6.16-6.19 (m, 10H), 6.31 (dd, 2H), 6.78 (dd, 8H).

²⁹Si-NMR (119.22 MHz, DMSO-d6): δ (ppm) = -53.11 (s, 2Si), -85.07 (s, 1Si).

ESI-MS: POSITIVE m/z 268.2 ([C₁₄H₂₆N₃O₂]⁺)
NEGATIVE m/z 743.1 (median) ([C₁₂H₁₈O₄₄Si₃W₁₁]⁴⁻)

### <Synthesis Example 63: tetrakis(3-acrylamidopropyltrimethylammonium)(1,3-di(3-acryloyloxypropyl)disiloxane-1,1,3,3-tetrayl)undecatungstosilicate>

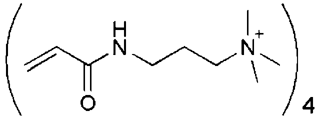

[SiW₁₁O₃₉(CH₂=CH-C(=O)-O-(CH₂)₃-Si)₂O]⁴⁻

The target compound was produced in the same manner as in Synthesis Example 1 except that, as raw material compounds, an aqueous solution of acrylamidopropyltrimethylammonium chloride was used instead of the aqueous solution of (2-trifluoromethylbenzyl)triallylammonium bromide, and 3-acryloyloxypropyltrimethoxysilane was used instead of methyltrimethoxysilane.

¹H-NMR (400 MHz, DMSO-d6): δ (ppm) = 0.57-0.61 (m, 4H), 1.77-1.93 (m, 12H), 3.09 (s, 36H), 3.20 (q, 8H), 3.38-3.42 (m, 8H), 4.10 (t, 4H), 5.60 (dd, 4H), 5.89 (dd, 2H), 6.10 (dd, 4H), 6.17 (dd, 2H), 6.31-6.32 (m, 6H), 8.68-8.71 (m, 4H).

²⁹Si-NMR (119.22 MHz, DMSO-d6): δ (ppm) = -53.11 (s, 2Si), -85.07 (s, 1Si).

ESI-MS: POSITIVE m/z 171.1 ([C₉H₁₉N₂O]⁺)
NEGATIVE m/z 743.1 (median) ([C₁₂H₁₈O₄₄Si₃W₁₁]⁴⁻)

### <Synthesis Example 64: tetrakis(methacryloylcholine)(1,3-di(3-acryloyloxypropyl)disiloxane-1,1,3,3-tetrayl)undecatungstosilicate>

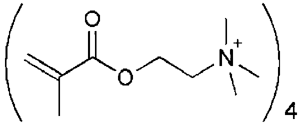

[SiW₁₁O₃₉(CH₂=CH-C(=O)-O-(CH₂)₃-Si)₂O]⁴⁻

The target compound was produced in the same manner as in Synthesis Example 1 except that, as raw material compounds, an aqueous solution of methacryloylcholine chloride was used instead of the aqueous solution of (2-trifluoromethylbenzyl)triallylammonium bromide, and 3-acryloyloxypropyltrimethoxysilane was used instead of methyltrimethoxysilane.

¹H-NMR (400 MHz, DMSO-d6): δ (ppm) = 0.57-0.61 (m, 4H), 1.77-1.85 (m, 4H), 1.90 (t, 12H), 3.20 (s, 36H), 3.79-3.81 (m, 8H), 4.10 (t, 4H), 4.54 (t, 8H), 5.75 (t, 4H), 5.89 (dd, 2H), 6.09 (t, 4H), 6.17 (dd, 2H), 6.31 (dd, 2H).

²⁹Si-NMR (119.22 MHz, DMSO-d6): δ (ppm) = -53.11 (s, 2Si), -85.07 (s, 1Si).

ESI-MS: POSITIVE m/z 172.1 ([C₉H₁₈NO₂]⁺)
NEGATIVE m/z 743.1 (median) ([C₁₂H₁₈O₄₄Si₃W₁₁]⁴⁻)

### <Synthesis Example 65: tetrakis((2-acryloyloxyethyl)benzyldimethylammonium)(1,3-di(3-acryloyloxypropyl)disiloxane-1,1,3,3-tetrayl)undecatungstosilicate>

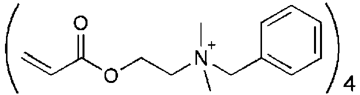

[SiW₁₁O₃₉(CH₂=CH-C(=O)-O-(CH₂)₃-Si)₂OSi)₂O]⁴⁻

The target compound was produced in the same manner as in Synthesis Example 1 except that, as raw material compounds, an aqueous solution of (2-acryloyloxyethyl)benzyldimethylammonium chloride was used instead of the aqueous solution of (2-trifluoromethylbenzyl)triallylammonium bromide, and 3-acryloyloxypropyltrimethoxysilane was used instead of methyltrimethoxysilane.

¹H-NMR (400 MHz, DMSO-d6): δ (ppm) = 0.57-0.61 (m, 4H), 1.77-1.85 (m, 4H), 3.01 (s, 24H), 3.78-3.81 (m, 8H), 4.10 (t, 4H), 4.65 (br, 8H), 4.75 (s, 8H), 5.89 (dd, 2H), 6.04 (dd, 4H), 6.17 (dd, 2H), 6.24 (dd, 4H), 6.31 (dd, 2H), 6.41 (dd, 4H), 7.52-7.55 (m, 12H), 7.63 (d, 8H).

²⁹Si-NMR (119.22 MHz, DMSO-d6): δ (ppm) = -53.11 (s, 2Si), -85.07 (s, 1Si).

ESI-MS: POSITIVE m/z 234.1 ([C₁₄H₂₀NO₂]⁺)
NEGATIVE m/z 743.1 (median) ([C₁₂H₁₈O₄₄Si₃W₁₁]⁴⁻)

### <Synthesis Example 66: tetrakis(propargyltriphenylphosphonium)(1,3-di(3-mercaptopropyl)disiloxane-1,1,3,3-tetrayl)undecatungstosilicate>

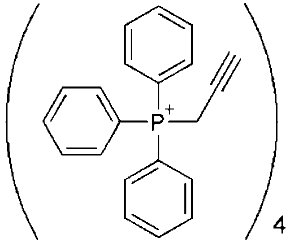

[SiW₁₁O₃₉(HS-(CH₂)₃-Si)₂O]⁴⁻

The target compound was produced in the same manner as in Synthesis Example 1 except that, as raw material compounds, an aqueous solution of propargyltriphenylphosphonium chloride was used instead of the aqueous solution of (2-trifluoromethylbenzyl)triallylammonium bromide, and 3-mercaptopropyltrimethoxysilane was used instead of methyltrimethoxysilane.

¹H-NMR (400 MHz, DMSO-d6): δ (ppm) = 0.67 (t, 4H), 1.70-1.83 (m, 4H), 2.14 (t, 2H), 2.53-2.61 (m, 4H), 3.48 (d, 4H), 5.21 (d, 8H), 7.76-7.99 (m, 60H).

²⁹Si-NMR (119.22 MHz, DMSO-d6): δ (ppm) = -53.06 (s, 2Si), -85.20 (s, 1Si).

ESI-MS: POSITIVE m/z 301.1 ([C₂₁H₁₈P]⁺)
NEGATIVE m/z 724.1 (median) ([C₆H₁₄O₄₀S₂Si₃W₁₁]⁴⁻)

### <Synthesis Example 67: tetrakis(allyltriphenylphosphonium)(1,3-di(3-mercaptopropyl)disiloxane-1,1,3,3-tetrayl)undecatungstosilicate>

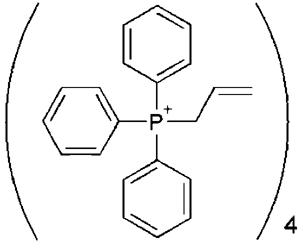

[SiW₁₁O₃₉(HS-(CH₂)₃-Si)₂O]⁴⁻

The target compound was produced in the same manner as in Synthesis Example 1 except that, as raw material compounds, an aqueous solution of allyltriphenylphosphonium chloride was used instead of the aqueous solution of (2-trifluoromethylbenzyl)triallylammonium bromide, and 3-mercaptopropyltrimethoxysilane was used instead of methyltrimethoxysilane.

¹H-NMR (400 MHz, DMSO-d6): δ (ppm) = 0.67 (t, 4H), 1.70-1.83 (m, 4H), 2.14 (t, 2H), 2.53-2.61 (m, 4H), 4.89 (dd, 8H), 5.38-5.42 (m, 4H), 5.64-5.74 (m, 8H), 7.69-7.88 (m, 60H). ²⁹Si-NMR (119.22 MHz, DMSO-d6): δ (ppm) = -53.06 (s, 2Si), -85.20 (s, 1Si).

ESI-MS: POSITIVE m/z 303.1 ([C₂₁H₂₀P]⁺)
NEGATIVE m/z 724.1 (median) ([C₆H₁₄O₄₀S₂Si₃W₁₁]⁴⁻)

### <Synthesis Example 68: tetrakis(tetraallylammonium)(1,3-di(3-mercaptopropyl)disiloxane-1,1,3,3-tetrayl)undecatungstosilicate>

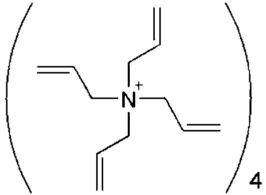

[SiW₁₁O₃₉(HS-(CH₂)₃-Si)₂O]⁴⁻

The target compound was produced in the same manner as in Synthesis Example 1 except that, as raw material compounds, an aqueous solution of tetraallylammonium iodide was used instead of the aqueous solution of (2-trifluoromethylbenzyl)triallylammonium bromide, and 3-mercaptopropyltrimethoxysilane was used instead of methyltrimethoxysilane.

¹H-NMR (400 MHz, DMSO-d6): δ (ppm) = 0.67 (t, 4H), 1.70-1.83 (m, 4H), 2.14 (t, 2H), 2.53-2.61 (m, 4H), 3.90 (d, 32H), 5.66-5.72 (m, 32H), 6.05-6.14 (m, 16H).

²⁹Si-NMR (119.22 MHz, DMSO-d6): δ (ppm) = -53.06 (s, 2Si), -85.20 (s, 1Si).

ESI-MS: POSITIVE m/z 178.2 ([C₁₂H₂₀N]⁺)
NEGATIVE m/z 724.1 (median) ([C₆H₁₄O₄₀S₂Si₃W₁₁]⁴⁻)

### <Synthesis Example 69: tetrakis((4-vinylbenzyl)triallylammonium)(1,3-di(3-mercaptopropyl)disiloxane-1,1,3,3-tetrayl)undecatungstosilicate>

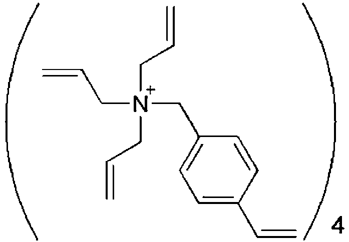

[SiW₁₁O₃₉(HS-(CH₂)₃-Si)₂O]⁴⁻

The target compound was produced in the same manner as in Synthesis Example 1 except that, as raw material compounds, an aqueous solution of (4-vinylbenzyl)triallylammonium chloride was used instead of the aqueous solution of (2-trifluoromethylbenzyl)triallylammonium bromide, and 3-mercaptopropyltrimethoxysilane was used instead of methyltrimethoxysilane.

¹H-NMR (400 MHz, DMSO-d6): δ (ppm) = 0.67 (t, 4H), 1.70-1.83 (m, 4H), 2.14 (t, 2H), 2.53-2.61 (m, 4H), 3.93 (d, 24H), 4.58 (s, 8H), 5.38 (d, 4H), 5.64-5.73 (m, 24H), 5.96 (d, 4H), 6.12-6.22 (m, 12H), 6.80 (dd, 4H), 7.62 (s, 16H).

²⁹Si-NMR (119.22 MHz, DMSO-d6): δ (ppm) = -53.06 (s, 2Si), -85.20 (s, 1Si).

ESI-MS: POSITIVE m/z 254.2 ([C₁₈H₂₄N]⁺)
NEGATIVE m/z 724.1 (median) ([C₆H₁₄O₄₀S₂Si₃W₁₁]⁴⁻)

### <Synthesis Example 70: tetrakis((3-hydroxypropyl)triallylammonium)(1,3-di(3-mercaptopropyl)disiloxane-1,1,3,3-tetrayl)undecatungstosilicate>

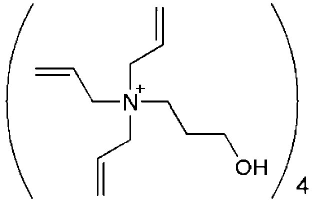

[SiW₁₁O₃₉(HS-(CH₂)₃-Si)₂O]⁴⁻

The target compound was produced in the same manner as in Synthesis Example 1 except that, as raw material compounds, an aqueous solution of (3-hydroxypropyl)triallylammonium iodide was used instead of the aqueous solution of (2-trifluoromethylbenzyl)triallylammonium bromide, and 3-mercaptopropyltrimethoxysilane was used instead of methyltrimethoxysilane.

¹H-NMR (400 MHz, DMSO-d6): δ (ppm) = 0.67 (t, 4H), 1.70-1.90 (m, 12H), 2.14 (t, 2H), 2.53-2.61 (m, 4H), 3.19-3.23 (m, 8H), 3.46 (t, 8H), 3.91 (d, 24H), 4.77 (br, 4H), 5.63-5.71 (m, 24H), 6.04-6.11 (m, 12H).

²⁹Si-NMR (119.22 MHz, DMSO-d6): δ (ppm) = -53.06 (s, 2Si), -85.20 (s, 1Si).

ESI-MS: POSITIVE m/z 196.2 ([C₁₂H₂₂NO]⁺)
NEGATIVE m/z 724.1 (median) ([C₆H₁₄O₄₀S₂Si₃W₁₁]⁴⁻)

### <Synthesis Example 71: tetrakis(bis(2-(N-methylacrylamido)ethyl)dimethylammonium)(1,3-di(3-mercaptopropyl)disiloxane-1,1,3,3-tetrayl)undecatungstosilicate>

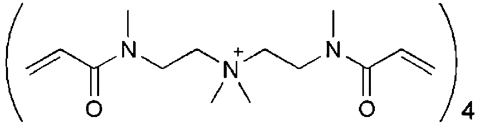

[SiW₁₁O₃₉(HS-(CH₂)₃-Si)₂O]⁴⁻

The target compound was produced in the same manner as in Synthesis Example 1 except that, as raw material compounds, an aqueous solution of bis(2-(N-methylacrylamido)ethyl)dimethylammonium iodide was used instead of the aqueous solution of (2-trifluoromethylbenzyl)triallylammonium bromide, and 3-mercaptopropyltrimethoxysilane was used instead of methyltrimethoxysilane.

¹H-NMR (400 MHz, DMSO-d6): δ (ppm) = 0.67 (t, 4H), 1.70-1.83 (m, 4H), 2.14 (t, 2H), 2.53-2.61 (m, 4H), 2.94-3.20 (m, 48H), 3.49 (t, 16H), 3.79 (t, 16H), 5.75 (dd, 8H), 6.17 (dd, 8H), 6.78 (dd, 8H).

²⁹Si-NMR (119.22 MHz, DMSO-d6): δ (ppm) = -53.06 (s, 2Si), -85.20 (s, 1Si).

ESI-MS: POSITIVE m/z 268.2 ([C₁₄H₂₆N₃O₂]⁺)
NEGATIVE m/z 724.1 (median) ([C₆H₁₄O₄₀S₂Si₃W₁₁]⁴⁻)

### <Synthesis Example 72: dicalcium(1,3-di(4-methoxymethoxyphenyl)disiloxane-1,1,3,3-tetrayl)undecatungstosilicate>

(Ca²⁺)₂[SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of calcium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 3.37 (s, 6H), 5.22 (s, 4H), 7.05 (d, 4H), 7.66 (d, 4H).

²⁹Si-NMR (119.22 MHz, DMSO-d6): δ (ppm) = -64.72 (s, 2Si), -85.02 (s, 1Si).

ESI-MS: POSITIVE m/z 20.0 ([Ca]²⁺)
NEGATIVE m/z 755.1 (median) ([C₁₆H₁₈O₄₄Si₃W₁₁]⁴⁻)

### <Synthesis Example 73: tetrasodium(1,3-di(4-methoxymethoxyphenyl)disiloxane-1,1,3,3-tetrayl)undecatungstosilicate>

(Na⁺)₄ [SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of sodium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 3.37 (s, 6H), 5.22 (s, 4H), 7.05 (d, 4H), 7.66 (d, 4H).

²⁹Si-NMR (119.22 MHz, DMSO-d6): δ (ppm) = -64.72 (s, 2Si), -85.02 (s, 1Si).

ESI-MS: POSITIVE m/z 23.0 ([Na]⁺)
NEGATIVE m/z 755.1 (median) ([C₁₆H₁₈O₄₄Si₃W₁₁]⁴⁻)

### <Synthesis Example 74: tetrapotassium(1,3-di(4-methoxymethoxyphenyl)disiloxane-1,1,3,3-tetrayl)undecatungstosilicate>

(K⁺)₄ [SiW₁₁O₃₉(p-CH₃-O-CH₂-O-(C₆H₄)-Si)₂O]⁴⁻

The target compound was produced in the same manner as in Synthesis Example 1 except that, as raw material compounds, an aqueous solution of potassium chloride was used instead of the aqueous solution of (2-trifluoromethylbenzyl)triallylammonium bromide, and 4-methoxymethoxyphenyltrimethoxysilane was used instead of methyltrimethoxysilane. ¹H-NMR (400 MHz, DMSO-d6): δ (ppm) = 3.37 (s, 6H), 5.22 (s, 4H), 7.05 (d, 4H), 7.66 (d, 4H).

²⁹Si-NMR (119.22 MHz, DMSO-d6): δ (ppm) = -64.72 (s, 2Si), -85.02 (s, 1Si).

ESI-MS: POSITIVE m/z 39.0 ([K]⁺)
NEGATIVE m/z 755.1 (median) ([C₁₆H₁₈O₄₄Si₃W₁₁]⁴⁻)

### <Synthesis Example 75: tetrasodium(1,3-di(2-((1-methylcyclopentyl)oxycarbonyl)ethyl)disiloxane-1,1,3,3-tetrayl)undecatungstosilicate>

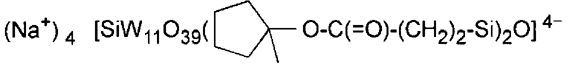

The target compound was produced in the same manner as in Synthesis Example 1 except that, as raw material compounds, an aqueous solution of sodium chloride was used instead of the aqueous solution of (2-trifluoromethylbenzyl)triallylammonium bromide, and 2-((1-methylcyclopentyl)oxycarbonyl)ethyltrimethoxysilane was used instead of methyltrimethoxysilane.

¹H-NMR (400 MHz, DMSO-d6): δ (ppm) = 0.77-0.81 (m, 4H), 1.49-1.84 (m, 22H), 2.37-2.42 (m, 4H).

²⁹Si-NMR (119.22 MHz, DMSO-d6): δ (ppm) = -53.12 (s, 2Si), -85.01 (s, 1Si).

ESI-MS: POSITIVE m/z 23.0 ([Na]⁺)
NEGATIVE m/z 764.4 (median) ([C₁₈H₃₀O₄₄Si₃W₁₁]⁴⁻)

### <Synthesis Example 76: tetrasodium(1,3-di(4-(t-butoxycarbonyloxy)phenyl)disiloxane-1,1,3,3-tetrayl)undecatungstosilicate>

(Na⁺)₄ [SiW₁₁O₃₉(p-t-Bu-O-C(=O)-O-(C₆H₄)-Si)₂O]⁴⁻

The target compound was produced in the same manner as in Synthesis Example 1 except that, as raw material compounds, an aqueous solution of sodium chloride was used instead of the aqueous solution of (2-trifluoromethylbenzyl)triallylammonium bromide, and 4-(t-butoxycarbonyloxy)phenyltrimethoxysilane was used instead of methyltrimethoxysilane.

¹H-NMR (400 MHz, DMSO-d6): δ (ppm) = 1.55 (s, 18H), 7.01 (d, 4H), 7.76 (d, 4H). ²⁹Si-NMR (119.22 MHz, DMSO-d6): δ (ppm) = -65.64 (s, 2Si), -85.03 (s, 1Si).

ESI-MS: POSITIVE m/z 23.0 ([Na]⁺)
NEGATIVE m/z 783.1 (median) ([C₂₂H₂₆O₄₆Si₃W₁₁]⁴⁻)

### <Synthesis Example 77: tetrakis(benzyltrimethylammonium)(1,3-di(2-((1-methylcyclopentyl)oxycarbonyl)ethyl)disiloxane-1,1,3,3-tetrayl)undecatungstosilicate>

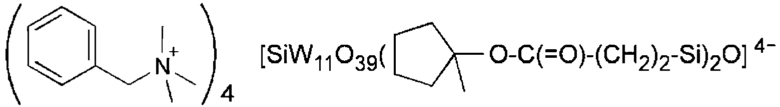

The target compound was produced in the same manner as in Synthesis Example 1 except that, as raw material compounds, an aqueous solution of benzyltrimethylammonium chloride was used instead of the aqueous solution of (2-trifluoromethylbenzyl)triallylammonium bromide, and 2-((1-methylcyclopentyl)oxycarbonyl)ethyltrimethoxysilane was used instead of methyltrimethoxysilane.

¹H-NMR (400 MHz, DMSO-d6): δ (ppm) = 0.77-0.81 (m, 4H), 1.49-1.84 (m, 22H), 2.37-2.42 (m, 4H), 3.04 (s, 36H), 4.53 (s, 8H), 7.50-7.57 (m, 20H).

²⁹Si-NMR (119.22 MHz, DMSO-d6): δ (ppm) = -53.12 (s, 2Si), -85.01 (s, 1Si).

ESI-MS: POSITIVE m/z 150.1 ([C₁₀H₁₆N]⁺)
NEGATIVE m/z 764.4 (median) ([C₁₈H₃₀0₄₄Si₃W₁₁]⁴⁻)

### <Synthesis Example 78: tetrakis(benzyltrimethylammonium)(1,3-di(4-(t-butoxycarbonyloxy)phenyl)disiloxane-1,1,3,3-tetrayl)undecatungstosilicate>

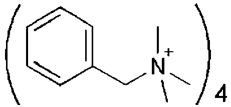

[SiW₁₁O₃₉(p-t-Bu-O-C(=O)-O-(C₆H₄)-Si)₂O]⁴⁻

The target compound was produced in the same manner as in Synthesis Example 1 except that, as raw material compounds, an aqueous solution of benzyltrimethylammonium chloride was used instead of the aqueous solution of (2-trifluoromethylbenzyl)triallylammonium bromide, and 4-(t-butoxycarbonyloxy)phenyltrimethoxysilane was used instead of methyltrimethoxysilane.

¹H-NMR (400 MHz, DMSO-d6): δ (ppm) = 1.55 (s, 18H), 3.04 (s, 36H), 4.53 (s, 8H), 7.01 (d, 4H), 7.50-7.57 (m, 20H), 7.76 (d, 4H).

²⁹Si-NMR (119.22 MHz, DMSO-d6): δ (ppm) = -65.64 (s, 2Si), -85.03 (s, 1Si).

ESI-MS: POSITIVE m/z 150.1 ([C₁₀H₁₆N]⁺)
NEGATIVE m/z 783.1 (median) ([C₂₂H₂₆O₄₆Si₃W₁₁]⁴⁻)

### <Synthesis Example 79: tetrakis(benzyltriethylammonium)(1,3-di(3-(t-butoxycarbonylmethylthio)propyl)disiloxane-1,1,3,3-tetrayl)undecatungstosilicate>

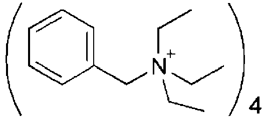

[SiW₁₁O₃₉(p-t-Bu-O-C(=O)-CH₂-S-(CH₂)₃-Si)₂O]⁴⁻

The target compound was produced in the same manner as in Synthesis Example 1 except that, as raw material compounds, an aqueous solution of benzyltriethylammonium chloride was used instead of the aqueous solution of (2-trifluoromethylbenzyl)triallylammonium bromide, and 3-(t-butoxycarbonylmethylthio)propyltrimethoxysilane was used instead of methyltrimethoxysilane.

¹H-NMR (400 MHz, DMSO-d6): δ (ppm) = 0.67 (t, 4H), 1.14 (s,18H), 1.34 (t, 36H), 1.72-1.84 (m, 4H), 2.68 (t, 4H), 3.14 (s, 4H), 3.20 (q, 24H), 4.49 (s, 8H), 7.52 (m, 20H). ²⁹Si-NMR (119.22 MHz, DMSO-d6): δ (ppm) = -53.05 (s, 2Si), -85.05 (s, 1Si).

ESI-MS: POSITIVE m/z 192.2 ([C₁₃H₂₂N]⁺)
NEGATIVE m/z 781.1 (median) ([C₁₈H₃₄O₄₄S₂Si₃W₁₁]⁴⁻)

### <Production Example 5: 3-((1-methylcyclopentyl)oxycarbonylmethylthio)propyltrimethoxysilane>

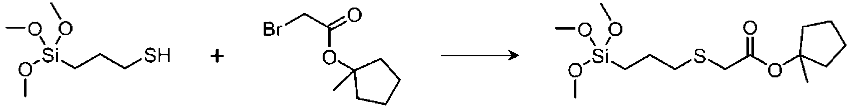

9.8 g of 3-mercaptopropyltrimethoxysilane and 6.1 g of triethylamine were dissolved in 125 mL of dehydrated tetrahydrofuran, and 13.3 g of 1-methylcyclopentyl 2-bromoacetate was added dropwise thereto at room temperature. The reaction liquid was then stirred at room temperature for 20 hours and was then filtered, and the filtrate was concentrated using a rotary evaporator. Finally, distillation under reduced pressure was performed to produce 16.0 g of the target compound as a colorless liquid.

¹H-NMR (400 MHz, DMSO-d6): δ (ppm) = 0.56 (t, 2H), 1.39 (s, 3H), 1.56-1.81 (m, 10H), 2.42 (t, 2H), 3.34 (s, 2H), 3.55 (s, 9H).

### <Synthesis Example 80: tetrakis(benzyltriethylammonium)(1,3-di(3-((1-methylcyclopentyl)oxycarbonylmethylthio)propyl)disiloxane-1,1,3,3-tetrayl)undecatungstosilicate>

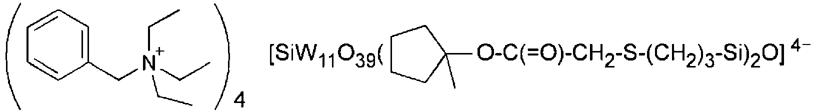

The target compound was produced in the same manner as in Synthesis Example 1 except that, as raw material compounds, an aqueous solution of benzyltriethylammonium chloride was used instead of the aqueous solution of (2-trifluoromethylbenzyl)triallylammonium bromide, and 3-((1-methylcyclopentyl)oxycarbonylmethylthio)propyltrimethoxysilane was used instead of methyltrimethoxysilane.

¹H-NMR (400 MHz, DMSO-d6): δ (ppm) = 0.67 (t, 4H), 1.34 (t, 36H), 1.49 (s, 6H), 1.54-1.84 (m, 16H), 1.99-2.08 (m, 4H), 2.68 (t, 4H), 3.14 (s, 4H), 3.20 (q, 24H), 4.49 (s, 8H), 7.52 (m, 20H).

²⁹Si-NMR (119.22 MHz, DMSO-d6): δ (ppm) = -53.04 (s, 2Si), -85.02 (s, 1Si).

ESI-MS: POSITIVE m/z 192.2 ([C₁₃H₂₂N]⁺)
NEGATIVE m/z 794.1 (median) ([C₂₂H₃₈O₄₄S₂Si₃W₁₁]⁴⁻)

### <Production Example 6: 3-(2-((1-methylcyclopentyl)oxycarbonyl)ethylthio)propyltrimethoxysilane>

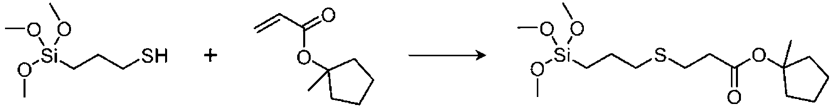

20 mL of methyl ethyl ketone was added to 7.7 g of 1-methylcyclopentyl acrylate and 9.8 g of 3-mercaptopropyltrimethoxysilane, and 0.3 g of 1, 1'-azobis(cyclohexane-1-carbonitrile) was added thereto, followed by uniform stirring. Nitrogen was then blown thereinto for 30 minutes for nitrogen bubbling. The reaction liquid was heated from room temperature to 90°C over 30 minutes in nitrogen. The reaction liquid was kept at 90°C for 7 hours. Methyl ethyl ketone was distilled off from the reaction liquid using a rotary evaporator. The reaction liquid was sufficiently dried under vacuum to produce 16.6 g of the target compound as a colorless liquid.

¹H-NMR (400 MHz, DMSO-d6): δ (ppm) = 0.56 (t, 2H), 1.39 (s, 3H), 1.56-1.81 (m, 10H), 2.42 (t, 2H), 2.58 (t, 2H), 2.83 (t, 2H), 3.55 (s, 9H).

### <Synthesis Example 81: tetrakis(benzyltriethylammonium)(1,3-di(3-(2-((1-methylcyclopentyl)oxycarbonyl)ethylthio)propyl)disiloxane-1,1,3,3-tetrayl)undecatungstosilicate>

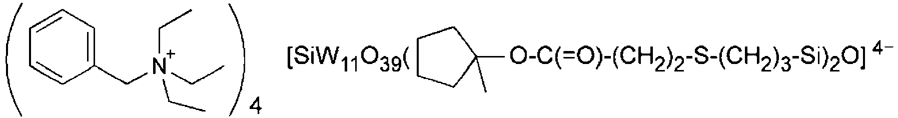

The target compound was produced in the same manner as in Synthesis Example 1 except that, as raw material compounds, an aqueous solution of benzyltriethylammonium chloride was used instead of the aqueous solution of (2-trifluoromethylbenzyl)triallylammonium bromide, and 3-(2-((1-methylcyclopentyl)oxycarbonyl)ethylthio)propyltrimethoxysilane was used instead of methyltrimethoxysilane.

¹H-NMR (400 MHz, DMSO-d6): δ (ppm) = 0.67 (t, 4H), 1.34 (t, 36H), 1.49 (s, 6H), 1.54-1.71 (m, 16H), 1.99-2.08 (m, 4H), 2.49 (t, 4H), 2.58 (t, 4H), 2.67 (t, 4H), 3.20 (q, 24H), 4.49 (s, 8H), 7.52 (m, 20H).

²⁹Si-NMR (119.22 MHz, DMSO-d6): δ (ppm) = -53.02 (s, 2Si), -85.04 (s, 1Si).

ESI-MS: POSITIVE m/z 192.2 ([C₁₃H₂₂N]⁺)
NEGATIVE m/z 801.1 (median) ([C₂₄H₄₂O₄₄S₂Si₃W₁₁]⁴⁻)

### <Synthesis Example 82: bis(1,4-diallyl-1,4-diazabicyclo[2.2.2]octane-1,4-diium)(1,3-di(3-(2-((1-methylcyclopentyl)oxycarbonyl)ethylthio)propyl)disiloxane-1,1,3,3-tetrayl)undecatungstosilicate>

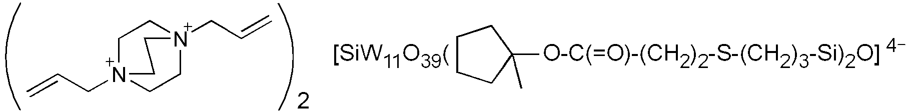

The target compound was produced in the same manner as in Synthesis Example 1 except that, as raw material compounds, an aqueous solution of 1,4-diallyl-1,4-diazabicyclo[2.2.2]octane-1,4-diium dibromide was used instead of the aqueous solution of (2-trifluoromethylbenzyl)triallylammonium bromide, and 3-(2-((1-methylcyclopentyl)oxycarbonyl)ethylthio)propyltrimethoxysilane was used instead of methyltrimethoxysilane.

¹H-NMR (400 MHz, DMSO-d6): δ (ppm) = 0.67 (t, 4H), 1.49 (s, 6H), 1.54-1.71 (m, 16H), 1.99-2.08 (m, 4H), 2.49 (t, 4H), 2.58 (t, 4H), 2.67 (t, 4H), 3.99 (s, 48H), 4.21 (d, 16H), 5.77-5.83 (m, 16H), 5.96-6.11 (m, 8H).

²⁹Si-NMR (119.22 MHz, DMSO-d6): δ (ppm) = -53.02 (s, 2Si), -85.04 (s, 1Si).

ESI-MS: POSITIVE m/z 97.1 ([C₁₂H₂₂N₂]²⁺)
NEGATIVE m/z 801.1 (median) ([C₂₄H₄₂O₄₄S₂Si₃W₁₁]⁴⁻)

### [2. Preparation of Film-Forming Material for Testing]

As film-forming materials for testing, test film-forming materials (R-1) to (R-6) were prepared by blending polyacid salts (A-1) to (A-6) shown below among the polyacid salts synthesized above and organic solvents (γ-butyrolactone and diacetone alcohol) so as to have the contents (unit: parts by mass) shown in Table 1 below.

As the polyacid salts (A-1) to (A-6) in Table 1, the following polyacid salts were used.
* Polyacid salt (A-1)

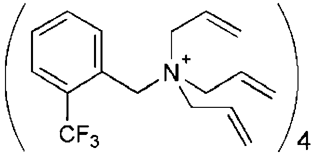

   [SiW₁₁O₃₉(CH₂=C(CH₃)-C(=O)-O-(CH₂)₃-Si)₂O]⁴⁻
* Polyacid salt (A-2)

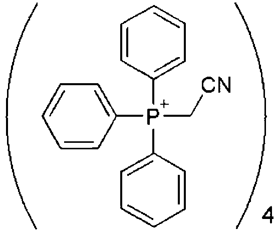

   [SiW₁₁O₃₉(p-CH₃-O-CH₂-O-(C₆H₄)-Si)₂O]⁴⁻
* Polyacid salt (A-3)

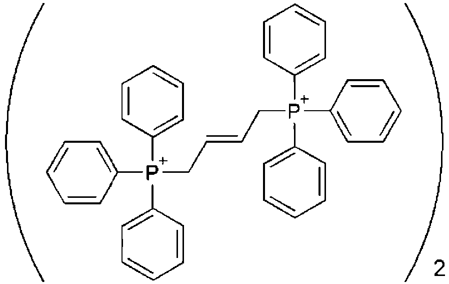

   [SiW₁₁O₃₉(p-CH₃-O-CH₂-O-(C₆H₄)-Si)₂O]⁴⁻
* Polyacid salt (A-4)

   (K⁺)₄ [SiW₁₁O₃₉(p-CH₃-O-CH₂-O-(C₆H₄)-Si)₂O]⁴⁻
* Polyacid salt (A-5) 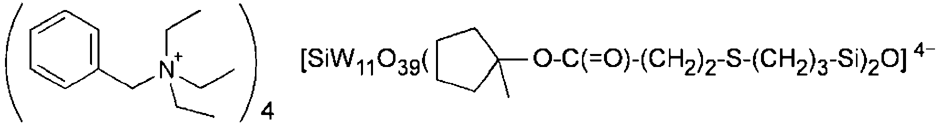
* Polyacid salt (A-6)

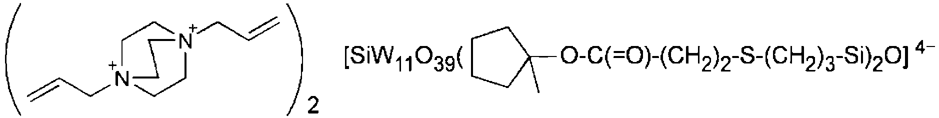

**[Table 1]**

| Component | R-1 | R-2 | R-3 | R-4 | R-5 | R-6 |
|---|---|---|---|---|---|---|
| Polyacid salt (A-1) | 2 | | | | | |
| Polyacid salt (A-2) | | 2 | | | | |
| Polyacid salt (A-3) | | | 2 | | | |
| Polyacid salt (A-4) | | | | 2 | | |
| Polyacid salt (A-5) | | | | | 2 | |
| Polyacid salt (A-6) | | | | | | 2 |
| γ-butyrolactone | 15 | 15 | 15 | 15 | 15 | 15 |
| Diacetone alcohol | 85 | 85 | 85 | 85 | 85 | 85 |

### [3. Film Formability Test and Evaluation]

Each of the test film-forming materials (R-1) to (R-6) was applied to an 8-inch silicon substrate using a spinner, was prebaked (PAB) on a hot plate at a temperature of 120°C for 60 seconds, and was dried to form test films (M-1) to (M-6), and the film formability of the test films (M-1) to (M-6) was evaluated according to the following criteria. Table 2 shows the results.
A: a film was formed without defects
B: a film was formed although there were some defects
C: a film was not formed due to defects on the front surface

**[Table 2]**

| | M-1 | M-2 | M-3 | M-4 | M-5 | M-6 |
|---|---|---|---|---|---|---|
| Evaluation of film formability | A | A | A | B | A | A |

The results of Table 2 show that the test films (M-1) to (M-3), (M-5), and (M-6) were formed without defects, wherein these test films were formed using the test film-forming materials containing the polyacid salts (A-1) and (A-5) in which the lacunary site of the heteropolyacid anion having the lacunary site was modified and an ammonium cation was introduced as a counter cation of the heteropolyacid anion, the polyacid salt (A-6) in which an ammonium dication was introduced, the polyacid salt (A-2) in which a phosphonium cation was introduced, and the polyacid salt (A-3) in which a phosphonium dication was introduced.

Furthermore, it is found that, by modifying the lacunary site of the heteropolyacid anion having the lacunary site, a film can be formed even when K⁺ is used as a counter cation of the heteropolyacid anion, although some defects are present.

These results show that, by modifying the lacunary site of the heteropolyacid anion having the lacunary site to form an organic/inorganic hybrid structure, as in the polyacid salts (A-1) to (A-6), good solubility or dispersibility in an organic solvent can be achieved, a uniform film with few defects can be formed, and the polyacid salts are useful as resist materials or the like.

## Claims

1. A heteropolyacid salt having a modified lacunary site represented by the general formula (I) or a mixture thereof.
(A^{m+})ₐ(Bⁿ⁺)_{b}(C^{(am+bn)-}) (I)
[wherein
A^{m+} each independently H⁺ or metal ion,
Bⁿ⁺ each independently denotes an ammonium cation, an ammonium dication, a phosphonium cation, or a phosphonium dication,
C^{(am+bn)-} denotes a heteropolyacid anion having a lacunary site, wherein the lacunary site is modified, and
m denotes an integer in the range of 1 to 5, n denotes an integer of 1 or 2, and a and b denote real numbers]

2. The heteropolyacid salt having the modified lacunary site or a mixture thereof according to claim 1, wherein the heteropolyacid anion includes Mo, W, V, Nb, or Ta as a polyatom and P, Si, B, S, or Ge as a heteroatom.

3. The heteropolyacid salt having the modified lacunary site or a mixture thereof according to claim 1, wherein the heteropolyacid anion having the lacunary site is a lacunary Keggin-type heteropolyacid anion or a lacunary Dawson-type heteropolyacid anion.

4. The heteropolyacid salt having the modified lacunary site or a mixture thereof according to claim 3, wherein the lacunary Keggin-type heteropolyacid anion is represented by the general formula (II-1), (II-2), or (II-3).
[XM₁₁O₃₉]^{c11-} (II-1)
[XM₁₀O₃₆]^{c12-} (II-2)
[XM₉O₃₄]^{c13-} (II-3)
(wherein
X denotes a heteroatom of P, Si, B, S, or Ge,
M denotes a polyatom of Mo, W, V, Nb, or Ta, and
c11- to c13- denote the number of negative charges, and c11 to c13 denote natural numbers)

5. The heteropolyacid salt having the modified lacunary site or a mixture thereof according to claim 3, wherein the lacunary Dawson-type heteropolyoxometalate anion is represented by the general formula (III-1), (III-2), or (III-3).
[X₂M₁₇O₆₁]^{c21-} (III-1)
[X₂M₁₆O₅₈]^{c22-} (III-2)
[X₂M₁₅O₅₆]^{c23-} (III-3)
(wherein
X denotes a heteroatom of P, Si, B, S, or Ge,
M denotes a polyatom of Mo, W, V, Nb, or Ta, and
c21- to c23- denote the number of negative charges, and c21 to c23 denote natural numbers)

6. The heteropolyacid salt having the modified lacunary site or a mixture thereof according to claim 1, wherein the modification is performed by bonding a group having one or more heteroatoms P, Si, Ge, or Sn, to which one or more hydro groups or organic groups are bonded, to the heteropolyacid anion having the lacunary site via a part or all of the heteroatoms.

7. The heteropolyacid salt having the modified lacunary site or a mixture thereof according to claim 6, wherein the organic group has one or more halogen atoms, haloalkyl groups, hydroxy groups, thiol groups, nitro groups, cyano groups, carboxy groups, amino groups, sulfo groups, epoxy groups, glycidyl groups, or amide groups.

8. The heteropolyacid salt having the modified lacunary site or a mixture thereof according to claim 6, wherein the organic group has one or more ethylenically unsaturated double bonds.

9. The heteropolyacid salt having the modified lacunary site or a mixture thereof according to claim 8, wherein the ethylenically unsaturated double bond is a vinyl group, a (meth)acryloyl group, a (meth)acryloyloxy group, a (meth)acrylamide group, or a styryl group.

10. The heteropolyacid salt having the modified lacunary site or a mixture thereof according to claim 6, wherein the organic group has one or more hydroxy groups or carboxy groups, each having a protective group.

11. The heteropolyacid salt having the modified lacunary site or a mixture thereof according to claim 1, wherein the ammonium cation is an organic quaternary ammonium cation represented by the general formula (VIII). 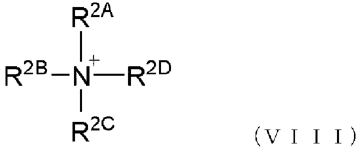 [In the formula (VIII),
R^{2A} to R^{2D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{2A} to R^{2D} except terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{2A} to R^{2D} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (1) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (s), and a divalent carbon atom at any position of these substituents except terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
any two of R^{2A} to R^{2D} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with a nitrogen atom in the formula (VIII)]

12. The heteropolyacid salt having the modified lacunary site or a mixture thereof according to claim 11, wherein at least one hydrogen atom in R^{2A} to R^{2D} of the organic quaternary ammonium cation represented by the general formula (VIII) is substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group.

13. The heteropolyacid salt having the modified lacunary site or a mixture thereof according to claim 1, wherein the ammonium dication is an organic quaternary ammonium dication represented by the general formula (IX). 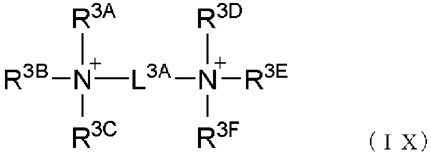 [In the formula (IX),
R^{3A} to R^{3F} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{3A} to R^{3F} except terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
L^{3A} denotes an optionally substituted C₁₋₁₈ hydrocarbylene group,
a divalent carbon atom at any position of L^{3A} may be substituted with -O-, -C(=O)-, -C(=O)O-, -OCO-, -CONH-, -NHCO-, -S-, or SO₂- (provided that adjacent divalent carbon atoms are not substituted at the same time),
a hydrogen atom in R^{3A} to R^{3F} and L^{3A} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (s), and a divalent carbon atom at any position of these substituents except terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time), and
any two of R^{3A} to R^{3F} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with a nitrogen atom in the formula (IX)]

14. The heteropolyacid salt having the modified lacunary site or a mixture thereof according to claim 1, wherein the phosphonium cation is an organic quaternary phosphonium cation represented by the general formula (X). 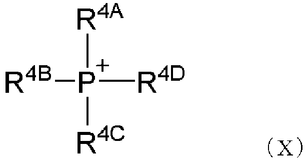 [In the formula (X),
R^{4A} to R^{4D} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{4A} to R^{4D} except terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
a hydrogen atom in R^{4A} to R^{4D} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (1) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group, and
any two of R^{4A} to R^{4D} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with the phosphorus atom in the formula (X)].

15. The heteropolyacid salt having the modified lacunary site or a mixture thereof according to claim 14, wherein at least one hydrogen atom in R^{4A} to R^{4D} of the organic phosphonium cation represented by the general formula (X) is substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, or (s) a hydroxy group or a carboxy group each having a protective group.

16. The heteropolyacid salt having the modified lacunary site or a mixture thereof according to claim 1, wherein the phosphonium dication is an organic quaternary phosphonium dication represented by the general formula (XI). 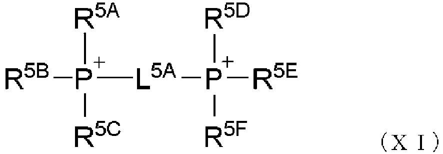 [In the formula (XI),
R^{5A} to R^{5F} each independently denote a C₁₋₁₈ hydrocarbyl group, a 3- to 18-membered non-aromatic heterocyclic group, or a 5- to 18-membered aromatic heterocyclic group, the hydrocarbyl group and the heterocyclic groups each optionally having a substituent,
a divalent carbon atom at any position of R^{5A} to R^{5F} except terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
L^{5A} denotes an optionally substituted C₁₋₁₈ hydrocarbylene group,
a divalent carbon atom at any position of L^{5A} may be substituted with -O-, -C(=O)-, -C(=O)O-, -OCO-, -CONH-, -NHCO-, -S-, or SO₂- (provided that adjacent divalent carbon atoms are not substituted at the same time),
a hydrogen atom in R^{5A} to R^{5F} and L^{5A} may be substituted with (a) a halogen atom, (b) a haloalkyl group, (c) a hydroxy group, (d) a thiol group, (e) a nitro group, (f) a cyano group, (g) a carboxy group, (h) an amino group, (i) a sulfo group, (j) a vinyl group, (k) an allyl group, (l) a (meth)acryloyl group, (m) a (meth)acryloyloxy group, (n) a (meth)acrylamide group, (o) a styryl group, (p) an epoxy group, (q) a glycidyl group, (r) an amide group, (s) a hydroxy group or a carboxy group each having a protective group, or (t) a C₁₋₁₈ hydrocarbyl group, a C₁₋₁₈ hydrocarbyloxy group, a C₁₋₁₈ hydrocarbylcarbonyl group, a C₁₋₁₈ hydrocarbylcarbonyloxy group, a C₁₋₁₈ hydrocarbyloxycarbonyl group, a C₁₋₁₈ hydrocarbyloxycarbonyloxy group, a C₁₋₁₈ hydrocarbylamino group, a di-C₁₋₁₈ hydrocarbylamino group, a C₁₋₁₈ hydrocarbylaminocarbonyl group, a di-C₁₋₁₈ hydrocarbylaminocarbonyl group, a C₁₋₁₈ hydrocarbylcarbonylamino group, a C₁₋₁₈ hydrocarbylaminocarbonyloxy group, a di-C₁₋₁₈ hydrocarbylaminocarbonyloxy group, a C₁₋₁₈ hydrocarbylaminocarbonylamino group, a di-C₁₋₁₈ hydrocarbylaminocarbonylamino group, a C₁₋₁₈ hydrocarbyloxycarbonylamino group, or a C₁₋₁₈ hydrocarbylthio group, in which at least part of hydrogen atoms may be substituted with (a) to (s), and a divalent carbon atom at any position of these substituents except terminal positions may be substituted with -O-, -C(=O)-, -C(=O)O-, -OCO-, -CONH-, -NHCO-, -NH(C=O)O-, -S-, or -SO₂- (provided that adjacent divalent carbon atoms are not substituted at the same time),
any two of R^{5A} to R^{5F} may be directly bonded to each other by a single bond or may be bonded to each other via a divalent linking group -O-, -S-, -C(=O)-, -S(=O)-, -S(=O)₂-, - C(=O)O-, or a C₁₋₃ alkylene group to form a ring together with a phosphorus atom in the formula (XI)]

17. The heteropolyacid salt having the modified lacunary site or a mixture thereof according to claim 1, wherein the metal ion is Na⁺, K⁺, Rb⁺, Cs⁺, Ca²⁺, Al³⁺, or Co³⁺.
